# EUROPEAN PATENT APPLICATION

(11) **EP 1 724 350 A1**
(43) Date of publication of application: **22.11.2006**
(21) Application number: 06009530.4
(22) Date of filing: 27.11.1996
(51) Int. Cl.: C12N 15/12, A61K 48/00

(54) **Method and compositions for the diagnosis and treatment of cancer**

(30) Priority: 30.11.1995 US 7810 P
(62) Divisional of application: 96942100.7
(71) Applicant: THE BOARD OF REGENTS, THE UNIVERSITY OF TEXAS SYSTEM, Austin, Texas 78701 (US)
(72) Inventor: Clayman, Gary L., Dr., Houston, TX 77005 (US)
(74) Representative: Bösl, Raphael Konrad

(57) **Abstract**

Methods for the treatment of squamous cell carcinoma using a p53-expressing viral vector are disclosed. In particular embodiments, the vector is a replication-deficient adenovirus. In addition, there are provided methods for examining the development and treatment of microscopic residual disease in the context of post-surgical environments and in body cavities.

## Description

### BACKGROUND OF THE INVENTION

The present application is a continuation-in-part of U.S. Provisional Patent Application Serial No. 60/007,810 filed November 30, 1995. The entire text of the above-referenced disclosure is specifically incorporated by reference herein without disclaimer

### A. Field of the Invention

The present invention is related generally to the field of cancer biology. In particular, the invention relates to compositions and methods of treatment for squamous cell carcinoma. Also provided is an animal model for the examination of microscopic residual tumors and tumor seeding into body cavities, as well as methods for treatment thereof.

### B. Related Art

Balancing rates of cell proliferation and cell death is important in maintaining normal tissue homeostasis. Disruption of this balance may be a major factor in the multistep process of tumorigenesis, and inhibition of apoptosis, or programmed cell death, is one cause of this disruption. The effects of such defects are catastrophic, causing over half a million deaths annually in the United States alone.

There is considerable evidence implicating mutations of the *p53* gene, a tumor suppressor, in the etiology of many human cancers. Reports have demonstrated that growth of several different human cancer cell lines, including representatives of colon cancer, glioblastoma, breast cancer, osteosarcoma and lung cancer can be functionally suppressed by viral-mediated transfer of a wild-type *p53* gene. Induction of exogenous *p53* expression of wild-type *p53* has been shown to induce apoptosis in colon cancer cell lines and in human lung cancer spheroids, suggesting a role for *p53* in programmed cell death.

Patients with squamous cell carcinoma of the head and neck (SCCHN) are afflicted with a disease that often has profound effects on speech, swallowing and cosmesis. Moreover, the overall rate of survival among these patients, approximately 50%, has remained unchanged for the nearly 30 years since contemporary surgery and radiation therapy were instituted. Recurrences are predominantly local and regional as opposed to systemic, indicating that microscopic residual carcinoma at the primary tumor site are the main cause of mortality. Given these facts, the ability to effectively attack microscopic residual disease in SCCHN is an endeavor that could improve the therapeutic efficacy of cancer treatments.

### SUMMARY OF THE INVENTION

Therefore, it is an object of the present invention to provide improved methods for the *in vivo* treatment of squamous cell carcinoma. It is another object of the present invention to provide a method for assessing the development and treatment of microscopic residual carcinoma and microscopic tumor seeding of body cavities.

In fulfilling these objects, there is provided a method for treating a subject with squamous cell carcinoma comprising the steps of (a) providing an expression construct comprising a promoter functional in eukaryotic cells and a polynucleotide encoding a *p53,* wherein the polynucleotide is positioned sense to and under the control of the promoter; and (b) contacting the expression construct with the squamous cell carcinoma *in vivo.*

The squamous cell carcinoma may be a head and neck carcinoma. The endogenous *p53* of the squamous cell carcinoma may or may not be mutated. The expression construct preferably is a viral vector, such as a retroviral vector, an adenoviral vector and an adeno-associated viral vector, with a replication-deficient adenoviral vector being most preferred. In a particular embodiment, the *p53* gene is tagged so that expression of *p53* from the expression vector can be detected. A preferred tag is an immunologic one, such as a continuous antibody epitope.

The method may further comprise surgical resection of the tumor, with additional contacting of the tumor bed, or "artificial body cavity," with the expression construct after resection. The volume used to contact the tumor bed is about 3 ml. to about 10 ml. Where an adenovirus vector is employed, the amount of adenovirus administered in each contacting is about 10⁷, 10⁸, 10⁹, 10¹⁰, 10¹¹ or 10¹² pfu.

Continuous perfusion of the expression construct also is contemplated. The amount of construct delivered in continuous perfusion will be determined from the amount delivered via injections so as to approximate the same total dosage over a given time period, although somewhat greater total dosages may be achieved using continuous perfusion.

In another embodiment, the expression construct is injected into an natural body cavity such as the mouth, pharynx, esophagus, larynx, trachea, pleural cavity, peritoneal cavity, or hollow organ cavities including the bladder, colon or other visceral organs.

Also provided is a method for determining the effectiveness of a therapy on microscopic residual cancer comprising (a) providing a rodent with an incision into subcutaneous tissue; (b) seeding the incision with tumor cells; (c) treating the rodent with a therapeutic regimen; and(d) assessing the impact of the regimen on the development of tumors. The incision may be sealed following step (b) and prior to step (c). Further, the regimen may comprise introduction of a therapeutic composition into the incision, the incision being reopened after sealing and resealed after introduction of said therapeutic composition.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein:

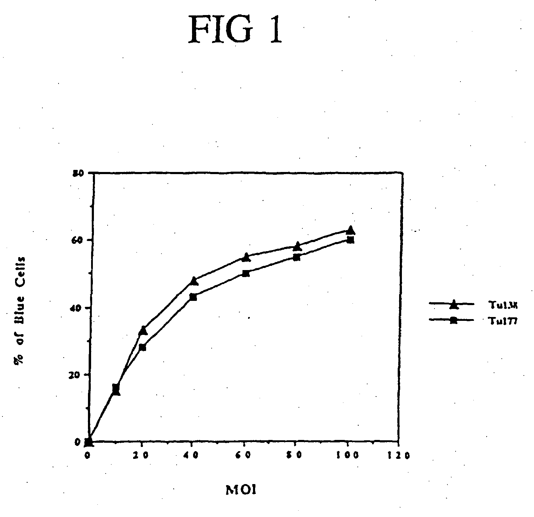

**FIG. 1.** Transduction efficiency of SCCHN cell lines Tu-138 (closed triangles) and Tu177 (closed squares). A recombinant β-gal adenovirus was used to infect the cells at different MOI ranging from 10 to 100. The percentages of β-gal-positive cells were obtained from scoring 500 cells each on replicate dishes.

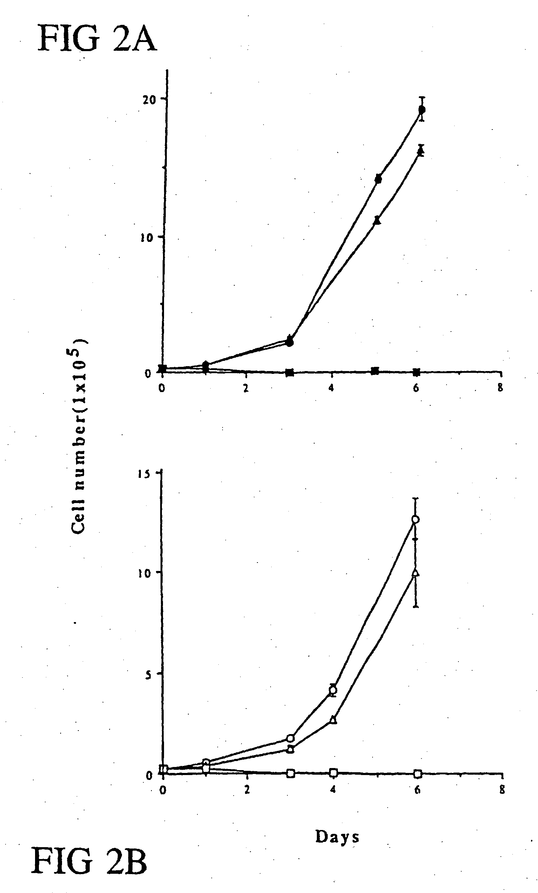

**FIG. 2A and FIG. 2B.** Inhibition of SCCHN cell growth *in vitro.* **(FIG. 2A)** Growth curve of mock-infected Tu-138 cells (closed circles), d1312-infected cells (closed triangles), and Ad5CMV-*p53*-infected cells (closed squares). (**FIG. 2B)** Growth curve of mock-infected Tu-177 cells (open circles), d1312-infected cells (open triangles), and AdSCMV-*p53*-infected cells (open squares). At each indicated time point, three dishes of cells were trypsinized and counted. The mean ± SEM of cell counts per triplicate wells following infection were plotted against the number of days since infection.

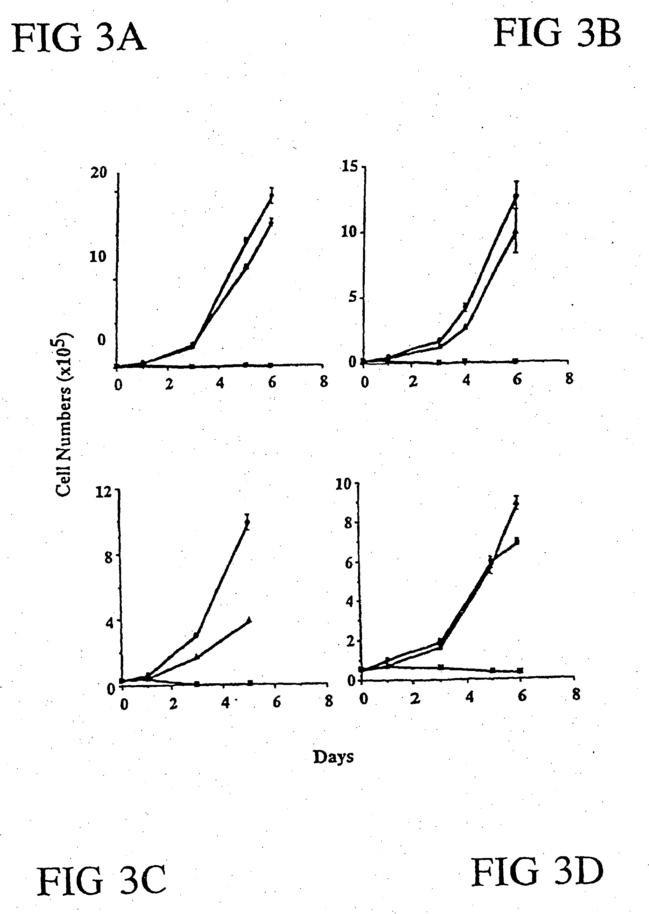

**FIG. 3A, FIG. 3B, FIG. 3C and FIG. 3D.** Composite Growth Curve of four SCCHN cell lines. **(FIG. 3A)** Tu-138. **(FIG. 3B)** Tu-177. **(FIG. 3C)** MDA 686-LN. **(FIG. 3D)** MDA 886. Mock infected cells (closed circles), d1312-infected cells (closed triangle), and Ad5CMV-*p53* infected cells (closed square). The mean of cell counts per triplicate wells following infection were plotted against the number of days since infection; bars, SEM.

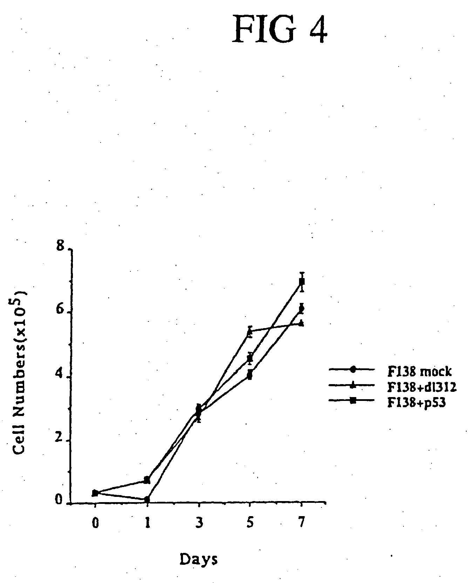

**FIG. 4.** Growth curve of normal fibroblast cell line. Mock-infected cells (closed circle), d1312-infected cells (closed triangle), and Ad5CMV-*p53*-infected cells (closed square).

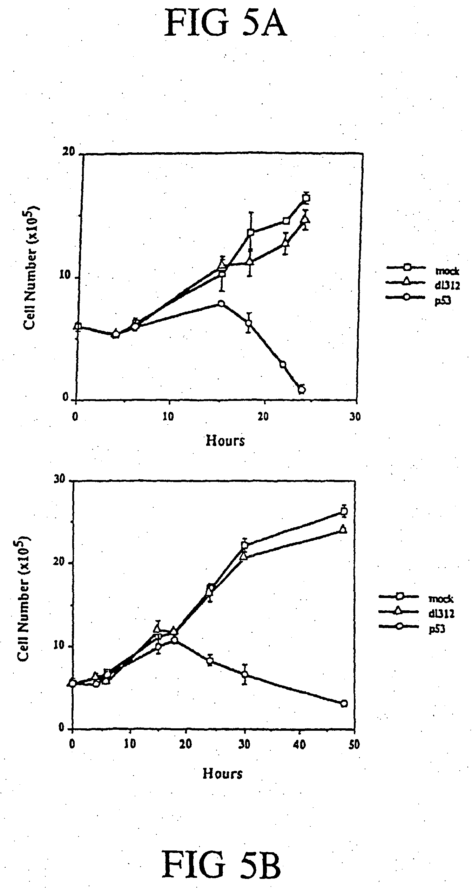

**FIG. 5A and FIG. 5B.** Composite growth curve of SCCHN cell lines. **FIG. 5A:** Tu-138; FIG. 5B: MDA 686LN; Mock infected cells (open square), dl312 infected cells (open triangle), and Ad5CMV-*p53* infected cells (open circle). At each indicated time point, three dishes of cells were trypsinized and counted. The mean of cell counts per triplicate dishes were plotted against the number of hours post-infection; *bars,* SEM.

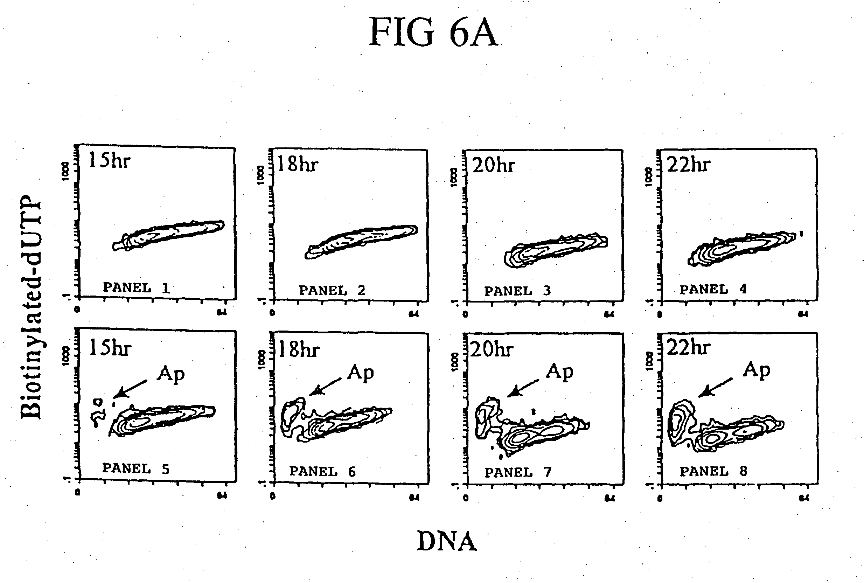

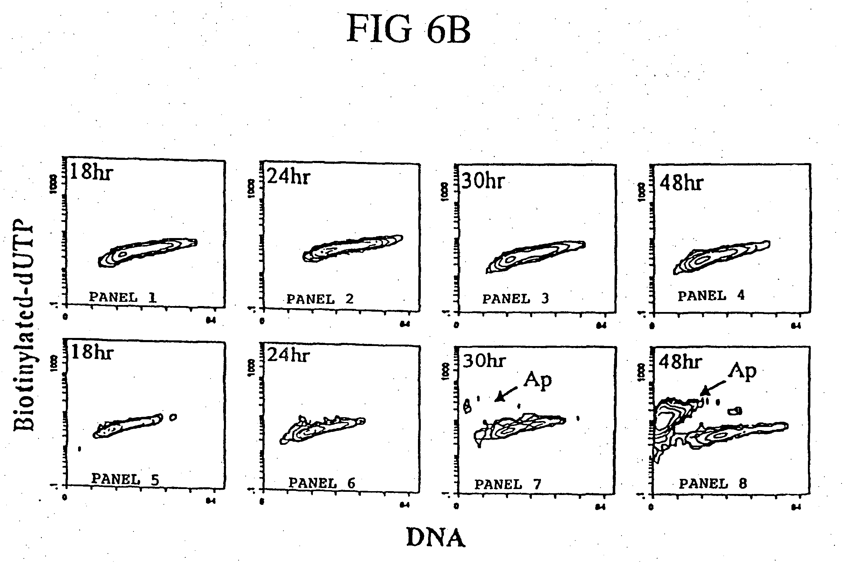

**FIG. 6A and FIG. 6B.** Labeling of DNA breaks in apoptotic cells with biotinylafed-dUTP by TUNEL method. Following infection, flow cytometric analysis for apoptosis was performed in a time course study. **FIG. 6A.** Tu-138 cells which are infected with d1312, a replication-defective adenovirus (panel 1-panel 4), Tu-138 cells infected with the wild-type *p53* adenovirus (panel 5- panel 8). **FIG. 6B.** MDA 686LN cells which are infected with d1312, a replication-defective adenovirus (A-D), MDA 686LN cells infected with the wild-type *p53* adenovirus (E-H). Ap stands for apoptosis.

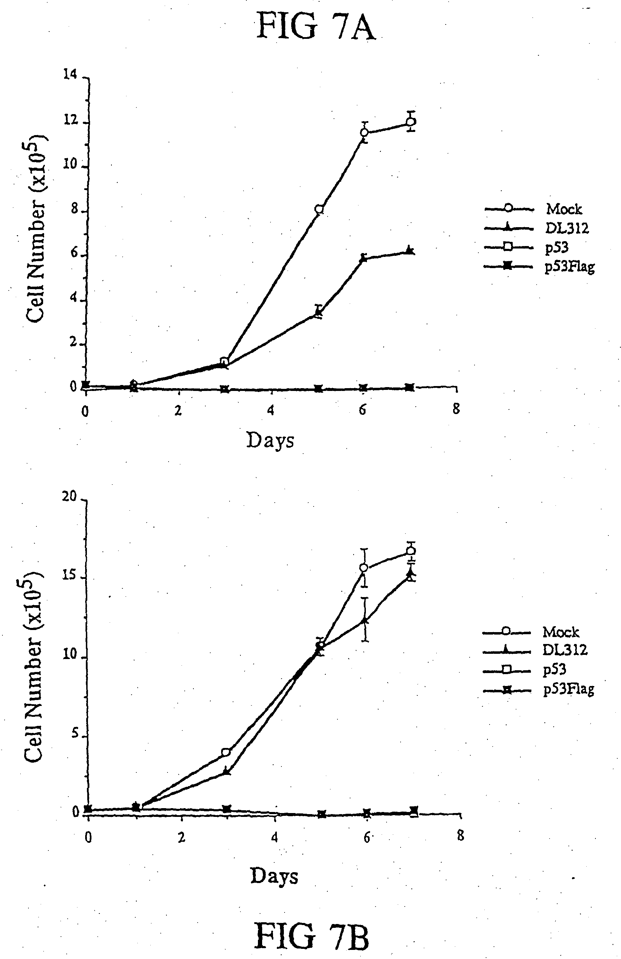

**FIG. 7A and FIG. 7B.** Composite growth curve of SCCHN cell line. **FIG. 7A:** Tu-138; **FIG. 7B:** MD686LN; Mock infected cells (open circles), d1312 infected cells (closed triangles), Ad5CMV-*p53* infected cells (open squares) and AdSCMV-*p53*-FLAG infected cells (closed squares). At each indicated time point, three dishes of cells were trypsinized and counted. The mean of cell counts per triplicate dishes were plotted against the number of hours post-infection; *bars,* SEM.

### DETAILED DESCRIPTION OF THE PREFERRED EMBODIMENTS

Information available to date suggests that one of the primary shortcomings of treatments for SCCHN is the inability to completely eradicate disease at the primary tumor site, or in the immediate local or regional tissues. Therefore, the present invention is designed to provide gene therapeutic methodologies that permit a more complete and effective treatment of SCCHN, specifically, by attacking microscopic residual carcinoma. This methodology may be used alone or as an adjunct to more conventional treatments such as chemo- or radiotherapy or surgical intervention. Moreover, using an animal model specifically designed to address microscopic residual carcinoma, as well as microscopic tumor seeding of body cavities, the present inventor has demonstrated efficacy of these methods. The details of the invention are described more completely below.

More generally, it now has been observed that p53 gene therapy of cancers may be effective regardless of the p53 status of the tumor cell. Surprisingly, therapeutic effects have been observed when a viral vector carrying the wild-type p53 gene is used to treat a tumor, the cells of which express a functional p53 molecule. This result would not have been predicted based on the current understanding of how tumor suppressors function. It also is surprising given that normal cells, which also express a functional p53 molecule, are apparently unaffected by expression of high levels of p53 from a viral construct. This raises the possibility that p53 gene therapy may be more broadly applicable to the treatment of cancers than was initially suspected.

### A. p53 Proteins and Polynucleotides

Throughout this application, the term *"p53"* is intended to refer to the exemplified *p53* molecules as well as all *p53* homologues from other species. "Wild-type" and "mutant" *p53* refer, respectively, to a *p53* gene expressing normal tumor suppressor activity and to a *p53* gene lacking or having reduced suppressor activity and/or having transforming activity. Thus "mutant" *p53's* are not merely sequence variants but rather, are those variants showing altered functional profiles.

*p53* is currently recognized as a tumor suppressor gene (Montenarh, 1992). High levels have been found in many cells transformed by chemical carcinogenesis, ultraviolet radiation, and several viruses, including SV40. The *p53* gene is a frequent target of mutational inactivation in a wide variety of human tumors and is already documented to be the most frequently-mutated gene in common human cancers (Mercer, 1992). It is mutated in over 50% of human NSCLC (Hollestein *et al.,* 1991) and in a wide spectrum of other tumors.

While tumors containing a mutated *p53* gene are a preferred target according to the present invention, the utility of the claimed *p53* expression vectors extends to the treatment of tumors having wild-type or functional *p53*. Though the mechanism is not completely understood, the present inventor has determined that *p53* expression can limit the growth of tumors expressing a functional *p53* product, and even induce apoptosis in such cells. Thus, the *p53* status of a tumor, though potentially useful in a diagnostic context, is not essential for practice of the present invention. This phenomenon is not limited to SCCHN tumors, but may be applied to a wide variety of malignancies including gliomas, sarcomas, carcinomas, leukemias, lymphomas and melanoma, including tumors of the skin, liver, testes, bone, brain, pancreas, head and neck, stomach, liver, lung, ovary, breast, colon, prostate and bladder.

### n53 Polypeptides

The *p53* gene encodes a 375-amino-acid phosphoprotein that can form complexes with viral proteins such as large-T antigen and E1B. The protein is found in normal tissues and cells, but at concentrations which are minute by comparison with many transformed cells or tumor tissue. Interestingly, wild-type *p53* appears to be important in regulating cell growth and division. Overexpression of wild-type *p53* has been shown in some cases to be anti-proliferative in human tumor cell lines. Thus *p53* can act as a negative regulator of cell growth (Weinberg, 1991) and may directly suppress uncontrolled cell growth or indirectly activate genes that suppress this growth. Thus, absence or inactivation of wild-type *p53* may contribute to transformation. However, some studies indicate that the presence of mutant *p53* may be necessary for full expression of the transforming potential of the gene.

Although wild-type *p53* is recognized as a centrally important growth regulator in many cell types, its genetic and biochemical traits appear to have a role as well. Mis-sense mutations are common for the *p53* gene and are essential for the transforming ability of the oncogene. A single genetic change prompted by a point mutation can create carcinogenic *p53*. Unlike other oncogenes, however, *p53* point mutations are known to occur in at least 30 distinct codons, often creating dominant alleles that produce shifts in cell phenotype without a reduction to homozygosity. Additionally, many of these dominant negative alleles appear to be tolerated in the organism and passed on in the germ line. Various mutant alleles appear to range from minimally dysfunctional to strongly penetrant, dominant negative alleles (Weinberg, 1991).

Casey and colleagues have reported that transfection of DNA encoding wild-type *p53* into two human breast cancer cell lines restores growth suppression control in such cells (Casey *et al.,* 1991). A similar effect has also been demonstrated on transfection of wild-type, but not mutant, *p53* into human lung cancer cell lines (Takahasi *et al.,* 1992). The *p53* wild-type appears dominant over the mutant gene and will select against proliferation when transfected into cells with the mutant gene. Expression of the transfected *p53* does not affect the growth of normal cells with endogenous *p53.* Thus, such constructs might be taken up by normal cells without adverse effects.

It is thus possible that the treatment of *p53*-associated cancers with wild-type *p53* may reduce the number of malignant cells. However, studies such as those described above are far from achieving such a goal, not least because DNA transfection cannot be employed to introduce DNA into cancer cells within a patients' body.

### p53-Encoding Polynucleotides

The polynucleotides according to the present invention may encode an entire *p53* gene, a functional *p53* protein domain, or any *p53* polypeptide. "Complementary" polynucleotides are those which are capable of base-pairing according to the standard Watson-Crick complementarity rules. That is, the larger purines will base pair with the smaller pyrimidines to form combinations of guanine paired with cytosine (G:C) and adenine paired with either thymine (A:T) in the case of DNA, or adenine paired with uracil (A:U) in the case of RNA. Inclusion of less common bases such as inosine, 5-methylcytosine, 6-methyladenine, hypoxanthine and others in hybridizing sequences does not interfere with pairing.

As used herein, the term "complementary sequences" means polynucleotide sequences that are substantially complementary over their entire length and have very few base mismatches. For example, sequences of fifteen bases in length may be termed complementary when they have a complementary nucleotide at thirteen or fourteen positions. Naturally, sequences which are "completely complementary" will be sequences which are entirely complementary throughout their entire length and have no base mismatches.

Other sequences with lower degrees of homology also are contemplated. For example, an antisense construct which has limited regions of high homology, but also contains a non-homologous region (*e.g.,* a ribozyme) could be designed. These molecules, though having less than 50% homology, would bind to target sequences under appropriate conditions.

The polynucleotides may be derived from genomic DNA, *i. e.,* cloned directly from the genome of a particular organism. In other embodiments, however, the polynucleotides may be complementary DNA (cDNA). cDNA is DNA prepared using messenger RNA (mRNA) as template. Thus, a cDNA does not contain any interrupted coding sequences and usually contains almost exclusively the coding region(s) for the corresponding protein. In other embodiments, the polynucleotide may be produced synthetically.

It may be advantageous to combine portions of the genomic DNA with cDNA or synthetic sequences to generate specific constructs. For example, where an intron is desired in the ultimate construct, a genomic clone will need to be used. Introns may be derived from other genes in addition to *p53.* The cDNA or a synthesized polynucleotide may provide more convenient restriction sites for the remaining portion of the construct and, therefore, would be used for the rest of the sequence.

The human and mouse DNA sequence for *p53* is provided in SEQ ID NO:1 and SEQ ID NO:3 respectively, with the corresponding amino acids being provided in SEQ ID NO:2 and SEQ ID NO:4, respectively.

It is contemplated that natural variants of *p53* exist that have different sequences than those disclosed herein. Thus, the present invention is not limited to use of the provided polynucleotide sequence for *p53* but, rather, includes use of any naturally-occurring variants. The present invention also encompasses chemically synthesized mutants of these sequences.

Another kind of sequence variant results from codon variation. Because there are several codons for most of the 20 normal amino acids, many different DNA's can encode the *p53*. Reference to the following table will allow such variants to be identified.

**TABLE 1**

| Amino Acids | Codons | | | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

Allowing for the degeneracy of the genetic code, sequences that have between about 50% and about 75 %, or between about 76% and about 99% of nucleotides that are identical to the nucleotides disclosed herein will be preferred. Sequences that are within the scope of "a *p53*-encoding polynucleotide" are those that are capable of base-pairing with a polynucleotide segment set forth above under intracellular conditions.

As stated above, although the *p53* encoding sequences may be full length genomic or cDNA copies, or large fragments thereof. The present invention also may employ shorter oligonucleotides of p53. Sequences of 17 bases long should occur only once in the human genome and, therefore, suffice to specify a unique target sequence. Although shorter oligomers are easier to make and increase *in vivo* accessibility, numerous other factors are involved in determining the specificity of base-pairing. Both binding affinity and sequence specificity of an oligonucleotide to its complementary target increases with increasing length. It is contemplated that oligonucleotides of 8, 9, 10, 11, 12, 13, 14, 15, 16, 17, 18, 19 or 20 base pairs will be used, for example, in the preparation of p53 mutants and in PCR reactions.

Any sequence of 17 bases long should occur only once in the human genome and, therefore, suffice to specify a unique target sequence. Although shorter oligomers are easier to make and increase *in vivo* accessibility, numerous other factors are involved in determining the specificity of hybridization. Both binding affinity and sequence specificity of an oligonucleotide to its complementary target increases with increasing length.

In certain embodiments, one may wish to employ constructs which include other elements, for example, those which include C-5 propyne pyrimidines. Oligonucleotides which contain C-5 propyne analogues of uridine and cytidine have been shown to bind RNA with high affinity (Wagner *et al.,* 1993).

It also is well understood by the skilled artisan that, inherent in the definition of a biologically functional equivalent protein or peptide, is the concept that there is a limit to the number of changes that may be made within a defined portion of the molecule and still result in a molecule with an acceptable level of equivalent biological activity. Biologically functional equivalent peptides are thus defined herein as those peptides in which certain, not most or all, of the amino acids may be substituted. In particular, where the N-terminus of the p16 protein is concerned, it is contemplated that only about 16 or more preferably, about 5 amino acids may be changed within a given peptide. Of course, a plurality of distinct proteins/peptides with different substitutions may easily be made and used in accordance with the invention.

Amino acid substitutions are generally based on the relative similarity of the amino acid side-chain substituents, for example, their hydrophobicity, hydrophilicity, charge, size, and the like. An analysis of the size, shape and type of the amino acid side-chain substituents reveals that arginine, lysine and histidine are all positively charged residues; that alanine, glycine and serine are all a similar size; and that phenylalanine, tryptophan and tyrosine all have a generally similar shape. Therefore, based upon these considerations, arginine, lysine and histidine; alanine, glycine and serine; and phenylalanine, tryptophan and tyrosine; are defined herein as biologically functional equivalents.

In making changes, the hydropathic index of amino acids may be considered. Each amino acid has been assigned a hydropathic index on the basis of their hydrophobicity and charge characteristics, these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is generally understood in the art (Kyte & Doolittle, 1982, incorporated herein by reference). It is known that ceratin amino acids may be substituted for other amino acids having a similar hydropathic index or score and still retain a similar biological activity. In making changes based upon the hydropathic index, the substitution of amino acids whose hydropathic indices are within ±2 is preferred, those which are within ± 1 are particularly preferred, and those within ±0.5 are even more particularly preferred.

It is understood that an amino acid can be substituted for another having a similar hydrophilicity value and still obtain a biologically equivalent protein. As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

In making changes based upon similar hydrophilicity values, the substitution of amino acids whose hydrophilicity values are within ±2 is preferred, those which are within ± 1 are particularly preferred, and those within ±0.5 are even more particularly preferred.

### B. Expression Vectors

Throughout this application, the term "expression construct" is meant to include any type of genetic construct containing a nucleic acid coding for a gene product in which part or all of the nucleic acid encoding sequence is capable of being transcribed. The transcript may be translated into a protein, but it need not be. Thus, in certain embodiments, expression includes both transcription of a *p53* gene and translation of a *p53* mRNA into a *p53* protein product. In other embodiments, expression only includes transcription of the nucleic acid encoding a *p53* or its complement.

In order for the construct to effect expression of at least a *p53* transcript, the polynucleotide encoding the *p53* polynucleotide will be under the transcriptional control of a promoter. A "promoter" refers to a DNA sequence recognized by the synthetic machinery of the host cell, or introduced synthetic machinery, that is required to initiate the specific transcription of a gene. The phrase "under transcriptional control" means that the promoter is in the correct location in relation to the polynucleotide to control RNA polymerase initiation and expression of the polynucleotide.

The term promoter will be used here to refer to a group of transcriptional control modules that are clustered around the initiation site for RNA polymerase II. Much of the thinking about how promoters are organized derives from analyses of several viral promoters, including those for the HSV thymidine kinase (tk) and SV40 early transcription units. These studies, augmented by more recent work, have shown that promoters are composed of discrete functional modules, each consisting of approximately 7-20 bp of DNA, and containing one or more recognition sites for transcriptional activator or repressor proteins.

At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either co-operatively or independently to activate transcription.

The particular promoter that is employed to control the expression of a *p53* polynucleotide is not believed to be critical, so long as it is capable of expressing the polynucleotide in the targeted cell at sufficient levels. Thus, where a human cell is targeted, it is preferable to position the polynucleotide coding region adjacent to and under the control of a promoter that is capable of being expressed in a human cell. Generally speaking, such a promoter might include either a human or viral promoter.

In various embodiments, the human cytomegalovirus (CMV) immediate early gene promoter, the SV40 early promoter and the Rous sarcoma virus long terminal repeat can be used to obtain high-level expression of the *p53* polynucleotide. The use of other viral or mammalian cellular or bacterial phage promoters which are well-known in the art to achieve expression of polynucleotides is contemplated as well, provided that the levels of expression are sufficient to produce a growth inhibitory effect.

By employing a promoter with well-known properties, the level and pattern of expression of a polynucleotide following transfection can be optimized. For example, selection of a promoter which is active in specific cells, such as tyrosinase (melanoma), alpha-fetoprotein and albumin (liver tumors), CC10 (lung tumor) and prostate-specific antigen (prostate tumor) will permit tissue-specific expression of *p53* polynucleotides. Table 2 lists several elements/promoters which may be employed, in the context of the present invention, to regulate the expression of *p53* constructs. This list is not intended to be exhaustive of all the possible elements involved in the promotion of *p53* expression but, merely, to be exemplary thereof.

Enhancers were originally detected as genetic elements that increased transcription from a promoter located at a distant position on the same molecule of DNA. This ability to act over a large distance had little precedent in classic studies of prokaryotic transcriptional regulation. Subsequent work showed that regions of DNA with enhancer activity are organized much like promoters. That is, they are composed of many individual elements, each of which binds to one or more transcriptional proteins.

The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are often overlapping and contiguous, often seeming to have a very similar modular organization.

Additionally any promoter/enhancer combination (as per the Eukaryotic Promoter Data Base EPDB) could also be used to drive expression of a *p53* construct. Use of a T3, T7 or SP6 cytoplasmic expression system is another possible embodiment. Eukaryotic cells can support cytoplasmic transcription from certain bacteriophage promoters if the appropriate bacteriophage polymerase is provided, either as part of the delivery complex or as an additional genetic expression vector.

**TABLE 2**

| **ENHANCER** |
|---|
| Immunoglobulin Heavy Chain |
| Immunoglobulin Light Chain |
| T-Cell Receptor |
| HLA DQ α and DQ β |
| β-Interferon |
| Interleukin-2 |
| Interleukin-2 Receptor |
| MHC Class II 5 |
| MHC Class II HLA-DRα |
| β-Actin |
| Muscle Creatine Kinase |
| Prealbumin (Transthyretin) |
| Elastase *I* |
| Metallothionein |
| Collagenase |
| Albumin Gene |
| α-Fetoprotein |
| τ-Globin |
| β-Globin |
| c-fos |
| c-HA-ras |
| Insulin |
| Neural Cell Adhesion Molecule (NCAM) |
| α₁-Antitrypsin |
| H2B (TH2B) Histone |
| Mouse or Type I Collagen |
| Glucose-Regulated Proteins (GRP94 and GRP78) |
| Rat Growth Hormone |
| Human Serum Amyloid A (SAA) |
| Troponin I (TN I) |
| Platelet-Derived Growth Factor |
| Duchenne Muscular Dystrophy |
| SV40 |
| Polyoma |
| Retroviruses |
| Papilloma Virus |
| Hepatitis B Virus |
| Human Immunodeficiency Virus |
| Cytomegalovirus |
| Gibbon Ape Leukemia Virus |

Further, selection of a promoter that is regulated in response to specific physiologic signals can permit inducible expression of the *p53* construct. For example, with the polynucleotide under the control of the human PAI-1 promoter, expression is inducible by tumor necrosis factor. Table 3 illustrates several promoter/inducer combinations:

**TABLE 3**

| **Element** | **Inducer** |
|---|---|
| MT II | Phorbol Ester (TFA) Heavy metals |
| MMTV (mouse mammary tumor virus) | Glucocorticoids |
| β-Interferon | poly(rI)X |
| | poly(rc) |
| Adenovirus 5 E2 | Ela |
| c-jun | Phorbol Ester (TPA), H₂O₂ |
| Collagenase | Phorbol Ester (TPA) |
| Stromelysin | Phorbol Ester (TPA), IL-1 |
| SV40 | Phorbol Ester (TPA) |
| Murine MX Gene | Interferon, Newcastle Disease Virus |
| GRP78 Gene | A23187 |
| α-2-Macroglobulin | IL-6 |
| Vimentin | Serum |
| MHC Class I Gene H-2kB | Interferon |
| HSP70 | Ela, SV40 Large T Antigen |
| Proliferin | Phorbol Ester-TPA |
| Tumor Necrosis Factor | FMA |
| Thyroid Stimulating Hormone α Gene | Thyroid Hormone |

In certain embodiments of the invention, the delivery of an expression vector in a cell may be identified *in vitro* or *in vivo* by including a marker in the expression vector. The marker would result in an identifiable change to the transfected cell permitting easy identification of expression. Usually the inclusion of a drug selection marker aids in cloning and in the selection of transformants. Alternatively, enzymes such as herpes simplex virus thymidine kinase (tk) (eukaryotic) or chloramphenicol acetyltransferase (CAT) (prokaryotic) may be employed. Immunologic markers also can be employed. The selectable marker employed is not believed to be important, so long as it is capable of being expressed along with the polynucleotide encoding *p53.* Further examples of selectable markers are well known to one of skill in the art.

One typically will include a polyadenylation signal to effect proper polyadenylation of the transcript. The nature of the polyadenylation signal is not believed to be crucial to the successful practice of the invention, and any such sequence may be employed. The inventor has employed the SV40 polyadenylation signal in that it was convenient and known to function well in the target cells employed. Also contemplated as an element of the expression construct is a terminator. These elements can serve to enhance message levels and to minimize read through from the construct into other sequences.

In preferred embodiments of the present invention, the expression construct comprises a virus or engineered construct derived from a viral genome. The ability of certain viruses to enter cells via receptor-mediated endocytosis and, in some cases, integrate into the host cell chromosomes, have made them attractive candidates for gene transfer in to mammalian cells. However, because it has been demonstrated that direct uptake of naked DNA, as well as receptor-mediated uptake of DNA complexes (discussed below), expression vectors need not be viral but, instead, may be any plasmid, cosmid or phage construct that is capable of supporting expression of encoded genes in mammalian cells, such as pUC or Bluescript™ plasmid series.

### Retroviruses

The retroviruses are a group of single-stranded RNA viruses characterized by an ability to convert their RNA to double-stranded DNA in infected cells by a process of reverse-transcription (Coffm, 1990). The resulting DNA then stably integrates into cellular chromosomes as a provirus and directs synthesis of viral proteins. The integration results in the retention of the viral gene sequences in the recipient cell and its descendants. The retroviral genome contains three genes - *gag, pol,* and env - that code for capsid proteins, polymerase enzyme, and envelope components, respectively. A sequence found upstream from the *gag* gene, termed Ψ, functions as a signal for packaging of the genome into virions. Two long terminal repeat (LTR) sequences are present at the 5' and 3' ends of the viral genome. 'These contain strong promoter and enhancer sequences and are also required for integration in the host cell genome (Coffin, 1990).

In order to construct a retroviral vector, a nucleic acid encoding a *p53* is inserted into the viral genome in the place of certain viral sequences to produce a virus that is replication-defective. In order to produce virions, a packaging cell line containing the *gag, pol* and *env* genes but without the LTR and ψ components is constructed (Mann *et al.,* 1983). When a recombinant plasmid containing a human cDNA, together with the retroviral LTR andψ sequences is introduced into this cell line (by calcium phosphate precipitation for example), theψ sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al. ,* 1975).

A novel approach designed to allow specific targeting of retrovirus vectors was recently developed based on the chemical modification of a retrovirus by the chemical addition of lactose residues to the viral envelope. This modification could permit the specific infection of hepatocytes via sialoglycoproteinreceptors.

A different approach to targeting of recombinant retroviruses was designed in which biotinylated antibodies against a retroviral envelope protein and against a specific cell receptor were used. The antibodies were coupled via the biotin components by using streptavidin (Roux *et al. ,* 1989). Using antibodies against major histocompatibility complex class I and class II antigens, they demonstrated the infection of a variety of human cells that bore those surface antigens with an ecotropic virus *in vitro* (Roux *et al.,* 1989).

### Adenoviruses

Human adenoviruses are double-stranded DNA tumor viruses with genome sizes of approximate 36 kb (Tooze, 1981). As a model system for eukaryotic gene expression, adenoviruses have been widely studied and well characterized, which makes them an attractive system for development of adenovirus as a gene transfer system. This group of viruses is easy to grow and manipulate, and they exhibit a broad host range *in vitro* and *in vivo.* In lytically infected cells, adenoviruses are capable of shutting off host protein synthesis, directing cellular machineries to synthesize large quantities of viral proteins, and producing copious amounts of virus.

The E1 region of the genome includes E1A and E1B which encode proteins responsible for transcription regulation of the viral genome, as well as a few cellular genes. E2 expression, including E2A and E2B, allows synthesis of viral replicative functions, e.g. DNA-binding protein, DNA polymerase, and a terminal protein that primes replication. E3 gene products prevent cytolysis by cytotoxic T cells and tumor necrosis factor and appear to be important for viral propagation. Functions associated with the E4 proteins include DNA replication, late gene expression, and host cell shutoff. The late gene products include most of the virion capsid proteins, and these are expressed only after most of the processing of a single primary transcript from the major late promoter has occurred. The major late promoter (MLP) exhibits high efficiency during the late phase of the infection (Stratford-Perricaudet and Perricaudet, 1991a).

As only a small portion of the viral genome appears to be required *in cis* (Tooze, 1981), adenovirus-derived vectors offer excellent potential for the substitution of large DNA fragments when used in connection with cell lines such as 293 cells. Ad5-transformed human embryonic kidney cell lines (Graham, *et al.,* 1977) have been developed to provide the essential viral proteins *in trans.* The inventor thus reasoned that the characteristics of adenoviruses rendered them good candidates for use in targeting cancer cells *in vivo* (Grunhaus & Horwitz, 1992).

Particular advantages of an adenovirus system for delivering foreign proteins to a cell include (i) the ability to substitute relatively large pieces of viral DNA by foreign DNA; (ii) the structural stability of recombinant adenoviruses; (iii) the safety of adenoviral administration to humans; and (iv) lack of any known association of adenoviral infection with cancer or malignancies; (v) the ability to obtain high titers of the recombinant virus; and (vi) the high infectivity of Adenovirus.

Further advantages of adenovirus vectors over retroviruses include the higher levels of gene expression. Additionally, adenovirus replication is independent of host gene replication, unlike retroviral sequences. Because adenovirus transforming genes in the E1 region can be readily deleted and still provide efficient expression vectors, oncogenic risk from adenovirus vectors is thought to be negligible (Grunhaus & Horwitz, 1992).

In general, adenovirus gene transfer systems are based upon recombinant, engineered adenovirus which is rendered replicatiorrincompetent by deletion of a portion of its genome, such as E1, and yet still retains its competency for infection. Sequences encoding relatively large foreign proteins can be expressed when additional deletions are made in the adenovirus genome. For example, adenoviruses deleted in both E1 and E3 regions are capable of carrying up to 10 Kb of foreign DNA and can be grown to high titers in 293 cells (Stratford-Perricaudet and Perricaudet, 1991a). Surprisingly persistent expression of transgenes following adenoviral infection has also been reported.

Adenovirus-mediated gene transfer has recently been investigated as a means of mediating gene transfer into eukaryotic cells and into whole animals. For example, in treating mice with the rare recessive genetic disorder ornithine transcarbamylase (OTC) deficiency, it was found that adenoviral constructs could be employed to supply the normal OTC enzyme. Unfortunately, the expression of normal levels of OTC was only achieved in 4 out of 17 instances (Stratford-Perricaudet *et al.,* 1991b). Therefore, the defect was only partially corrected in most of the mice and led to no physiological or phenotypic change. These type of results therefore offer little encouragement for the use of adenoviral vectors in cancer therapy.

Attempts to use adenovirus to transfer the gene for cystic fibrosis transmembrane conductance regulator (CFTR) into the pulmonary epithelium of cotton rats have also been partially successful, although it has not been possible to assess the biological activity of the transferred gene in the epithelium of the animals (Rosenfeld *et al.,* 1992). Again, these studies demonstrated gene transfer and expression of the CFTR protein in lung airway cells but showed no physiologic effect. In the 1991 *Science* article, Rosenfeld *et al.* showed lung expression of al-antitrypsin protein but again showed no physiologic effect. In fact, they estimated that the levels of expression that they observed were only about 2 % of the level required for protection of the lung in humans, i.e., far below that necessary for a physiologic effect.

The gene for human α1-antitrypsin has been introduced into the liver of normal rats by intraportal injection, where it was expressed and resulted in the secretion of the introduced human protein into the plasma of these rats (Jaffe *et al. ,* 1992). However, and disappointingly, the levels that were obtained were not high enough to be of therapeutic value.

These type of results do not demonstrate that adenovirus is able to direct the expression of sufficient protein in recombinant cells to achieve a physiologically relevant effect, and they do not, therefore, suggest a usefulness of the adenovirus system for use in connection with cancer therapy. Furthermore, prior to the present invention, it was thought that *p53* could not be incorporated into a packaging cell, such as those used to prepare adenovirus, as it would be toxic. As E1B of adenovirus binds to *p53,* this was thought to be a further reason why adenovirus and *p53* technology could not be combined.

### Other Viral Vectors as Expression Constructs

Other viral vectors may be employed as expression constructs in the present invention. Vectors derived from viruses such as vaccinia virus (Ridgeway, 1988; Baichwal and Sugden, 1986; Coupar *et al.,* 1988) adeno-associated virus (AAV) (Ridgeway, 1988; Baichwal and Sugden, 1986; Hermonat and Muzycska, 1984) and herpesviruses may be employed. They offer several attractive features for various mammalian cells (Friedmann, 1989; Ridgeway, 1988; Baichwal and Sugden, 1986; Coupar *et al.,* 1988; Horwich *et al.,* 1990).

With the recent recognition of defective hepatitis B viruses, new insight was gained into the structure-function relationship of different viral sequences. *in vitro* studies showed that the virus could retain the ability for helper-dependent packaging and reverse transcription despite the deletion of up to 80 % of its genome (Horwich *et al.,* 1990). This suggested that large portions of the genome could be replaced with foreign genetic material. The hepatotropism and persistence (integration) were particularly attractive properties for liver-directed gene transfer. Chang *et al.* recently introduced the chloramphenicol acetyltransferase (CAT) gene into duck hepatitis B virus genome in the place of the polymerase, surface, and pre-surface coding sequences. It was cotransfected with wild-type virus into an avian hepatoma cell line. Culture media containing high titers of the recombinant virus were used to infect primary duckling hepatocytes. Stable CAT gene expression was detected for at least 24 days after transfection (Chang *et al.,* 1991).

### C. Alternative Methods for Gene Delivery

In order to effect expression of *p53* constructs, the expression vector must be delivered into a cell. As described above, the preferred mechanism for delivery is via viral infection where the expression vector is encapsidated in an infectious adenovirus particle.

Several non-viral methods for the transfer of expression vectors into cultured mammalian cells also are contemplated by the present invention. These include calcium phosphate precipitation (Graham and Van Der Eb, 1973; Chen and Okayama, 1987; Rippe *et al.,* 1990) DEAE-dextran (Gopal, 1985), electroporation (Tur-Kaspa *et al.,* 1986; Potter *et al.,* 1984), direct microinjection (Harland and Weintraub, 1985), DNA-loaded liposomes (Nicolau and Sene, 1982; Fraley *et al.,* 1979) and Iipofectamine-DNA complexes, cell sonication (Fechheimer et al., 1987), gene bombardment using high velocity microprojectiles (Yang *et al.,* 1990), polycations (Boussif *et al.,* 1995) and receptor-mediated transfection (Wu and Wu, 1987; Wu and Wu, 1988). Some of these techniques may be successfully adapted for *in vivo* or *ex vivo* use.

In one embodiment of the invention, the adenoviral expression vector may simply consist of naked recombinant vector. Transfer of the construct may, be performed by any of the methods mentioned above which physically or chemically permeabilize the cell membrane. For example, Dubensky *et al.* (1984) successfully injected polyomavirus DNA in the form of CaPO₄ precipitates into liver and spleen of adult and newborn mice demonstrating active viral replication and acute infection. Benvenisty and Neshif (1986) also demonstrated that direct intraperitoneal injection of CaPO₄ precipitated plasmids results in expression of the transfected genes. It is envisioned that DNA encoding an *p53* construct may also be transferred in a similar manner *in vivo.*

Another embodiment of the invention for transferring a naked DNA expression vector into cells may involve particle bombardment. This method depends on the ability to accelerate DNA coated microprojectiles to a high velocity allowing them to pierce cell membranes and enter cells without killing them (Klein *et al.,* 1987). Several devices for accelerating small particles have been developed. One such device relies on a high voltage discharge to generate an electrical current, which in turn provides the motive force (Yang *et al.,* 1990). The microprojectiles used have consisted of biologically inert substances such as tungsten or gold beads.

Selected organs including the liver, skin, and muscle tissue of rats and mice have been bombarded *in* vivo (Yang *et al.,* 1990; Zelenin *et al.,* 1991). This may require surgical exposure of the tissue or cells, to eliminate any intervening tissue between the gun and the target organ. DNA encoding a p53 construct may be delivered via this method.

In a further embodiment of the invention, the expression vector may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated are lipofectamine-DNA complexes.

Liposome-mediated polynucleotide delivery and expression of foreign DNA *in vitro* has been very successful. Wong *et al.* (1980) demonstrated the feasibility of liposome-mediated delivery and expression of foreign DNA in cultured chick embryo, HeLa and hepatoma cells. Nicolau *et al.* (1987) accomplished successful liposome-mediated gene transfer in rats after intravenous injection.

In certain embodiments of the invention, the liposome may be complexed with a hemagglutinating virus (HVJ). This has been shown to facilitate fusion with the cell membrane and promote cell entry of liposome-encapsulated DNA (Kaneda *et al.,* 1989). In other embodiments, the liposome may be complexed or employed in conjunction with nuclear non-histone chromosomal proteins (HMG-1) (Kato *et al.,* 1991). In yet further embodiments, the liposome may be complexed or employed in conjunction with both HVJ and HMG-1. In that such expression vectors have been successfully employed in transfer and expression of a polynucleotide *in vitro* and *in vivo,* then they are applicable for the present invention. Where a bacteriophage promoter is employed in the DNA construct, it also will be desirable to include within the liposome an appropriate bacteriophage polymerase.

Another mechanism for transferring expression vectors into cells is receptor-mediated delivery. This approach takes advantage of the selective uptake of macromolecules by receptor-mediated endocytosis in almost all eukaryotic cells. Because of the cell type-specific distribution of various receptors, the delivery can be highly specific (Wu and Wu, 1993). Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (ASOR) (Wu and Wu, 1987) and transferrin (Wagner *et al.,* 1993). Recently, a synthetic neoglycoprotein, which recognizes the same receptor as ASOR, has been used as a gene delivery vehicle (Ferkol *et al.,* 1993; Perales *et al.,* 1994) and epidermal growth factor (EGF) has also been used to deliver genes to squamous carcinoma cells (Myers, EPO 0273085).

In other embodiments, the delivery vehicle may comprise a ligand and a liposome. For example, Nicolau *et al.* (1987) employed lactosyl-ceramide, a galactose-terminal asialganglioside, incorporated into liposomes and observed an increase in the uptake of the insulin gene by hepatocytes. Thus, it is feasible that an adenoviral expression vector also may be specifically delivered into a cell type such as lung, epithelial or tumor cells, by any number of receptor-ligand systems, with or without liposomes. For example, epidermal growth factor (EGF) may be used as the receptor for mediated delivery of *p53* construct in many tumor cells that exhibit upregulation of EGF receptor. Mannose can be used to target the mannose receptor on liver cells. Also, antibodies to CD5 (CLL), CD22 (lymphoma), CD25 (T-ceil leukemia) and MAA (melanoma) can similarly be used as targeting moieties.

In certain embodiments, gene transfer may more easily be performed under ex *vivo* conditions. *Ex vivo* gene therapy refers to the isolation of cells from an animal, the delivery of a polynucleotide into the cells, *in vitro,* and then the return of the modified cells back into an animal. This may involve the surgical removal of dssue/organs from an animal or the primary culture of cells and tissues. Anderson et al., U.S. Patent 5,399,346, and incorporated herein in its entirety, disclose ex *vivo* therapeutic methods. During ex vivo culture, the expression vector can express the p53 construct. Finally, the cells may be reintroduced into the original animal, or administered into a distinct animal, in a pharmaceutically acceptable form by any of the means described below.

### D. Pharmaceutical Compositions and Routes of Administration

Where clinical application of an adenoviral expression vector according to the present invention is contemplated, it will be necessary to prepare the complex as a pharmaceutical composition appropriate for the intended application. Generally this will entail preparing a pharmaceutical composition that is essentially free of pyrogens, as well as any other impurities that could be harmful to humans or animals. One also will generally desire to employ appropriate salts and buffers to render the complex stable and allow for complex uptake by target cells.

Aqueous compositions of the present invention comprise an effective amount of the expression vector, dissolved or dispersed in a pharmaceutically acceptable carrier or aqueous medium. Such compositions also are referred to as inocula. The phrases "pharmaceutically or pharmacologically acceptable" refer to molecular entities and compositions that do not produce an adverse, allergic or other untoward reaction when administered to an animal, or a human, as appropriate. As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients also can be incorporated into the compositions.

Solutions of the active compounds as free base or pharmacologically acceptable salts can be prepared in water suitably mixed with a surfactant, such as hydroxypropylcellulose. Dispersions also can be prepared in glycerol, liquid polyethylene glycols, mixtures thereof and in oils. Under ordinary conditions of storage and use, these preparations contain a preservative to prevent the growth of microorganisms.

The expression vectors and delivery vehicles of the present invention may include classic pharmaceutical preparations. Administration of therapeutic compositions according to the present invention will be via any common route so long as the target tissue is available via that route. This includes oral, nasal, buccal, rectal, vaginal or topical. Alternatively, administration will be by orthotopic, intradermal, intraocular, subcutaneous, intramuscular, intraperitoneal or intravenous injection. Such compositions would normally be administered as pharmaceutically acceptable compositions that include physiologically acceptable carriers, buffers or other excipients.

The therapeutic compositions of the present invention are advantageously administered in the form of injectable compositions either as liquid solutions or suspensions; solid forms suitable for solution in, or suspension in, liquid prior to injection may also be prepared. These preparations also may be emulsified. A typical composition for such purpose comprises a pharmaceutically acceptable carrier. For instance, the composition may contain 10 mg, 25 mg, 50 mg or up to about 100 mg of human serum albumin per milliliter of phosphate buffered saline. Other pharmaceutically acceptable carriers include aqueous solutions, non-toxic excipients, including salts, preservatives, buffers and the like. Examples of non-aqueous solvents are propylene glycol, polyethylene glycol, vegetable oil and injectable organic esters such as ethyloleate. Aqueous carriers include water, alcoholic/aqueous solutions, saline solutions, parenteral vehicles such as sodium chloride, Ringer's dextrose, etc. Intravenous vehicles include fluid and nutrient replenishers. Preservatives include antimicrobial agents, anti-oxidants, chelating agents and inert gases. The pH and exact concentration of the various components the pharmaceutical composition are adjusted according to well known parameters.

Additional formulations are suitable for oral administration. Oral formulations include such typical excipients as, for example, pharmaceutical grades of mannitol, lactose, starch, magnesium stearate, sodium saccharine, cellulose, magnesium carbonate and the like. The compositions take the form of solutions, suspensions, tablets, pills, capsules, sustained release formulations or powders. When the route is topical, the form may be a cream, ointment, salve, liquid or spray.

An effective amount of the therapeutic agent is determined based on the intended goal, for example (i) inhibition of tumor cell proliferation or (ii) elimination of tumor cells. The term "unit dose" refers to physically discrete units suitable for use in a subject, each unit containing a predetermined-quantity of the therapeutic composition calculated to produce the desired responses, discussed above, in association with its administration, i.e., the appropriate route and treatment regimen. The quantity to be administered, both according to number of treatments and unit dose, depends on the subject to be treated, the state of the subject and the protection desired. Precise amounts of the therapeutic composition also depend on the judgment of the practitioner and are peculiar to each individual.

In certain embodiments, it may be desirable to provide a continuous supply of therapeutic compositions to the patient. For intravenous or intraarterial routes, this is accomplished by drip system. For topical applications, repeated application would be employed. For various approaches, delayed release formulations could be used that provided limited but constant amounts of the therapeutic agent over and extended period of time. For internal application, continuous perfusion of the region of interest may be preferred. This could be accomplished by catheterization, post-operatively in some cases, followed by continuous administration of the therapeutic agent. The time period for perfusion would be selected by the clinician for the particular patient and situation, but times could range from about 1-2 hours, to 2-6 hours, to about 6-10 hours, to about 10-24 hours, to about 1-2 days, to about 1-2 weeks or longer. Generally, the dose of the therapeutic composition via continuous perfusion will be equivalent to that given by single or multiple injections, adjusted for the period of time over which the injections are administered. It is believed that higher doses may be achieved via perfusion, however.

### Clinical Protocol for SCCHN

A clinical protocol has been developed to facilitate the treatment of SCCHN disease using the adenoviral constructs discussed below in the examples. In accordance with this protocol, patients having histologic proof of squamous cell carcinoma of the head and neck will be selected. Patients may, but need not have received previous chemo-, radio- or gene therapies. Optimally, patients will have adequate bone marrow function (defined as peripheral absolute granulocyte count of > 2,000/mm³ and platelet count of 100,000/mm³), adequate liver function (bilirubin ≤ 1.5 mg/dl) and adequate renal function (creatinine < 1.5 mg/dl).

The protocol calls for single dose administration, via intratumoral injection, of a pharmaceutical composition containing between 10⁶ and 10⁹ infectious particles of a *p53* adenovirus expression construct. For tumors of ≥ 4 cm, the volume administered will be 4-10 ml (preferably 10 ml), while for tumors < 4 cm, a volume of 1-3 ml will be used (preferably 3 ml). Multiple injections will be delivered for a single dose, in 0.1-0.5 ml volumes, with spacing of approximately 1 cm or more.

The treatment course consists of about six doses, delivered over two weeks. Upon election by the clinician, the regimen may be continued, six doses each two weeks, or on a less frequent (monthly, bimonthly, quarterly, etc.) basis.

Where patients are eligible for surgical resection, the tumor will be treated as described above for at least two consecutive two-week treatment courses. Within one week of completion of the second (or more, e.g., third, fourth, fifth, sixth, seventh, eighth, etc.) course, the patient will receive surgical resection. Prior to close of the incision, 10 ml of a pharmaceutical composition containing the *p53* adenovirus expression construct (10⁶-10⁹ infectious particles) will be delivered to the surgical site (operative bed) and allowed to remain in contact for at least 60 minutes. The wound is closed and a drain or catheter placed therein. On the third post-operative day, additional 10 ml of the pharmaceutical composition is administered via the drain and allowed to remain in contact with the operative bed for at least two hours. Removal by suction is then performed, and the drain removed at a clinically appropriate time.

### Treatment of Artificial and Natural Body Cavities

One of the prime sources of recurrent SCCHN is the residual, microscopic disease that remains at the primary tumor site, as well as locally and regionally, following tumor excision. In addition, there are analogous situations where natural body cavities are seeded by microscopic tumor cells. The effective treatment of such microscopic disease would present a significant advance in therapeutic regimens.

Thus, in certain embodiments, a cancer may be removed by surgical excision, creating a "cavity." Both at the time of surgery, and thereafter (periodically or continuously), the therapeutic composition of the present invention is administered to the body cavity. This is, in essence, a "topical" treatment of the surface of the cavity. The volume of the composition should be sufficient to ensure that the entire surface of the cavity is contacted by the expression construct.

In one embodiment, administration simply will entail injection of the therapeutic composition into the cavity formed by the tumor excision. In another embodiment, mechanical application via a sponge, swab or other device may be desired. Either of these approaches can be used subsequent to the tumor removal as well as during the initial surgery. In still another embodiment, a catheter is inserted into the cavity prior to closure of the surgical entry site. The cavity may then be continuously perfused for a desired period of time.

In another form of this treatment, the "topical" application of the therapeutic composition is targeted at a natural body cavity such as the mouth, pharynx, esophagus, larynx, trachea, pleural cavity, peritoneal cavity, or hollow organ cavities including the bladder, colon or other visceral organs. In this situation, there may or may not be a significant, primary tumor in the cavity. The treatment targets microscopic disease in the cavity, but incidentally may also affect a primary tumor mass if it has not been previously removed or a pre-neoplastic lesion which may be present within this cavity. Again, a variety of methods may be employed to affect the "topical" application into these visceral organs or cavity surfaces. For example, the oral cavity in the pharynx may be affected by simply oral swishing and gargling with solutions. However, topical treatment within the larynx and trachea may require endoscopic visualization and topical delivery of the therapeutic composition. Visceral organs such as the bladder or colonic mucosa may require indwelling catheters with infusion or again direct visualization with a cystoscope or other endoscopic instrument. Cavities such as the pleural and peritoneal cavities may be accessed by indwelling catheters or surgical approaches which provide access to those areas.

### Monitorine p53 Expression Following Administration

Another aspect of the present invention involves the monitoring of *p53* expression following administration of the therapeutic composition. Because destruction of microscopic tumor cells cannot be observed, it is important to determine whether the target site has been effectively contacted with the expression construct. This may be accomplished by identifying cells in which the expression construct is actively producing the *p53* product. It is important, however, to be able to distinguish between the exogenous *p53* and that present in tumor and non-tumor cells in the treatment area. Tagging of the exogenous *p53* with a tracer element would provide definitive evidence for expression of that molecule and not an endogenous version thereof.

One such tracer is provided by the FLAG biosystem (Hopp *et al.,* 1988). The FLAG polypeptide is an octapeptide (AspTyrLysAspAspAspAspLys) and its small size does not disrupt the expression of the delivered gene therapy protein. The coexpression of FLAG and the protein of interest is traced through the use of antibodies raised against FLAG protein.

Other immunologic marker systems, such as the 6XHis system (Qiagen) also may be employed. For that matter, any linear epitope could be used to generate a fusion protein with *p53* so long as (i) the immunologic integrity of the epitope is not compromised by the fusion and (ii) the functional integrity *of p53* is not compromised by the fusion.

### E. Combined Therapy Protocols

Tumor cell resistance to DNA damaging agents represents a major problem in clinical oncology. One goal of current cancer research is to find ways to improve the efficacy of chemo- and radiotherapy by combining it with gene therapy. For example, the herpes simplex-thymidine kinase (HS-tK) gene, when delivered to brain tumors by a retroviral vector system, successfully induced susceptibility to the antiviral agent ganciclovir (Culver, *et al.,* 1992). In the context of the present invention, it is contemplated that *p53* therapy could be used similarly in conjunction with chemo- or radiotherapeutic intervention.

To kill cells, such as malignant or metastatic cells, using the methods and compositions of the present invention, one would generally contact a "target" cell with an expression vector and at least one DNA damaging agent. These compositions would be provided in a combined amount effective to kill or inhibit proliferation of the cell. This process may involve contacting the cells with the expression vector and the DNA damaging agent(s) or factor(s) at the same time. This may be achieved by contacting the cell with a single composition or pharmacological formulation that includes both agents, or by contacting the cell with two distinct compositions or formulations, at the same time, wherein one composition includes the *p53* expression vector and the other includes the DNA damaging agent.

Alternatively, the *p53* treatment may precede or follow the DNA damaging agent treatment by intervals ranging from minutes to weeks. In embodiments where the DNA damaging factor and *p53* expression vector are applied separately to the cell, one would generally ensure that a significant period of time did not expire between the time of each delivery, such that the DNA damaging agent and expression vector would still be able to exert an advantageously combined effect on the cell. In such instances, it is contemplated that one would contact the cell with both agents within about 6 hours to one week of each other and, more preferably, within about 24-72 hours of each other, with a delay time of only about 48 hours being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

It also is conceivable that more than one administration of either the *p53* construct or the DNA damaging agent will be desired. Various combinations may be employed, where *p53* is "A" and the DNA damaging agent is "B":

| | | | | | | |
|---|---|---|---|---|---|---|
| A/B/A | B/A/B | B/B/A | A/A/B | B/A/A | A/B/B | A/B/B/B |
| B/B/B/A | B/B/A/B | A/A/B/B | A/B/A/B | A/B/B/A | B/B/A/A | B/A/B/B |
| B/A/B/A | B/A/A/B | A/A/A/B | B/A/A/A | A/B/A/A | A/A/B/A | B/A/A/B |

To achieve cell killing, both agents are delivered to a cell in a combined amount effective to kill the cell.

DNA damaging agents or factors are defined herein as any chemical compound or treatment method that induces DNA damage when applied to a cell. Such agents and factors include radiation and waves that induce DNA damage such as, γ-irradiation, X-rays, UV-irradiation, microwaves, electronic emissions, and the like. A variety of chemical compounds, also described as "chemotherapeutic agents", function to induce DNA damage, all of which are intended to be of use in the combined treatment methods disclosed herein. Chemotherapeutic agents contemplated to be of use, include, e.g., adriamycin, 5-fluorouracil (5FU), etoposide (VP-16), camptothecin, actinomycin-D, mitomycin C, cisplatin (CDDP) and even hydrogen peroxide. The invention also encompasses the use of a combination of one or more DNA damaging agents, whether radiation-based or actual compounds, such as the use of X-rays with cisplatin or the use of cisplatin with etoposide. In certain embodiments, the use of cisplatin in combination with a p53 expression vector is particularly preferred.

In treating cancer according to the invention, one would contact the tumor cells with a DNA damaging agent in addition to the expression vector. This may be achieved by irradiating the localized tumor site with DNA damaging radiation such as X-rays, UV-light, g-rays or even microwaves. Alternatively, the tumor cells may be contacted with the DNA damaging agent by administering to the subject a therapeutically effective amount of a pharmaceutical composition comprising a DNA damaging compound such as, adriamycin, 5-fluorouracil, etoposide, camptothecin, actinomycin-D, mitomycin C, or more preferably, cisplatin. The DNA damaging agent may be prepared and used as a combined therapeutic composition, or kit, by combining it with a *p53* expression vector, as described above.

Agents that directly cross-link polynucleotides, specifically DNA, are envisaged and are shown herein, to eventuate DNA damage leading to a synergistic antineoplastic combination. Agents such as cisplatin, and other DNA alkylating may be used. Cisplatin has been widely used to treat cancer, with efficacious doses used in clinical applications of 20 mg/m² for 5 days every three weeks for a total of three courses. Cisplatin is not absorbed orally and must therefore be delivered via injection intravenously, subcutaneously, intratumorally or intraperitoneally.

Agents that damage DNA also include compounds that interfere with DNA replication, mitosis and chromosomal segregation. Such chemotherapeutic compounds include adriamycin, also known as doxorubicin, etoposide, verapamil, podophyllotoxin, and the like. Widely used in a clinical setting for the treatment of neoplasms, these compounds are administered through bolus injections intravenously at doses ranging from 25-75 mg/m² at 21 day intervals for adriamycin, to 35-50 mg/m2 for etoposide intravenously or double the intravenous dose orally.

Agents that disrupt the synthesis and fidelity of polynucleotide precursors and subunits also lead to DNA damage. As such a number of polynucleotide precursors have been developed. Particularly useful are agents that have undergone extensive testing and are readily available. As such, agents such as 5-fluorouracil (5-FU), are preferentially used by neoplastic tissue, making this agent particularly useful for targeting to neoplastic cells. Although quite toxic, 5-FU, is applicable in a wide range of carriers, including topical, however intravenous administration with doses ranging from 3 to 15 mg/kg/day being commonly used.

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of DNA damage, or the precursors of DNA, the replication and repair of DNA, and the assembly and maintenance of chromosomes. Dosage ranges for X-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells.

The skilled artisan is directed to "Remington's Pharmaceutical Sciences" 15th Edition, chapter 33, in particular pages 624-652. Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

The inventor proposes that the regional delivery of *p53* expression vectors to patients with p53-linked cancers will be a very efficient method for delivering a therapeutically effective gene to counteract the clinical disease. Similarly, the chemo-or radiotherapy may be directed to a particular, affected region of the subject's body. Alternatively, systemic delivery of the expression vector or the DNA damaging agent may be appropriate in certain circumstances, for example, where extensive metastasis has occurred.

Cytokine therapy also has proven to be an effective partner for combined therapeutic regimens. Various cytokines may be employed in such combined approaches. Examples of cytokines include IL-1α IL-1β IL-2, IL-3, IL-4, IL-5, IL-6, IL-7, IL-8, IL-9, IL-10, IL-11, IL-12, IL-13, TGF-ß, GM-CSF, M-CSF, G-CSF, TNFα, TNFβ, LAF, TCGF, BCGF, TRF, BAF, BDG, MP, LIF, OSM, TMF, PDGF, IFN-α, IFN-ß, IFN-γ. Cytokines are administered according to standard regimens, as described below, consistent with clinical indications such as the condition of the patient and relative toxicity of the cytokine.

In addition to combining *p53*-targeted therapies with chemo-, radio and cytokine therapies, it also is contemplated that combination with other gene therapies will be advantageous. For example, targeting of *K-ras* and *p53* mutations at the same time may produce an improved anti-cancer treatment. Any other tumor-related gene conceivably can be targeted in this manner, for example, p21, p16, p27, E₂F, Dp family of genes, Rb, APC, DCC, NF-1, NF-2, WT-1, MEN-I, MEN-II, BRCA1, VHL, FCC, MCC, other ras molecules, *myc, neu, raf, erb, src, fms, jun, trk, ret, gsp, hst, bcl,* and *abl.* It also may be desirable to combine *p53* therapy with an antibody-based gene therapy treatment involving the use of a single-chain antibody construct in which the antibody binds to any of the foregoing molecules, or in combination with genes encoding one or more of the cytokines listed above. It also may be advantageous to combine *p53* with other genes that have been implicated in apoptotic processes, *e.g.,* adenovirus E1A, Bax, Bcl-Xₛ, *etc.*

### F. Kits

All the essential materials and reagents required for inhibiting tumor cell proliferation may be assembled together in a kit. This generally will comprise selected adenoviral expression vectors. Also included may be various media for replication of the expression vectors and host cells for such replication. Such kits will comprise distinct containers for each individual reagent.

When the components of the kit are provided in one or more liquid solutions, the liquid solution preferably is an aqueous solution, with a sterile aqueous solution being particularly preferred. For *in vivo* use, the expression vector may be formulated into a pharmaceutically acceptable syringeable composition. In this case, the container means may itself be an inhalent, syringe, pipette, eye dropper, or other such like apparatus, from which the formulation may be applied to an infected area of the body, such as the lungs, injected into an animal, or even applied to and mixed with the other components of the kit.

The components of the kit may also be provided in dried or lyophilized forms. When reagents or components are provided as a dried form, reconstitution generally is by the addition of a suitable solvent. It is envisioned that the solvent also may be provided in another container means.

The kits of the present invention also will typically include a means for containing the vials in close confinement for commercial sale such as, e.g., injection or blow-molded plastic containers into which the desired vials are retained. Irrespective of the number or type of containers, the kits of the invention also may comprise, or be packaged with, an instrument for assisting with the injection/administration or placement of the ultimate complex composition within the body of an animal. Such an instrument may be an inhalent, syringe, pipette, forceps, measured spoon, eye dropper or any such medically approved delivery vehicle.

### G. Animal Model for Microscopic Tumor Seeding and Residual Disease

Another aspect of the present invention involves the development of an animal model for the analysis of microscopic residual carcinomas and microscopic seeding of body cavities. "Carcinoma," as used herein, may refer to a single cell or a multicellular tumor mass. In microscopic disease, the "tumor" will consist or one or a few carcinoma cells which cannot be observed with the naked eye.

The animal model described herein is particularly advantageous mimicking (i) the post surgical environment of head and neck cancer patients, particularly in advanced stages of disease and (ii) the body cavity of an affected subject wherein microscopic carcinoma has been established. The model, similar to other animal models for cancer, derives from inoculation of tumor cells into an animal. A distinction, however, lies in the creation, subcutaneously, of a pouch that is a physiologic equivalent of a natural body cavity or a post-surgical cavity created by the excision of a tumor mass.

The instant inventions exemplifies nude mice as the model organism. Virtually any animal may be employed, however, for use according to the present invention. Particularly preferred animals will be small mammals that are routinely used in laboratory protocols. Even more preferred animals will be those of the rodent group, such as mice, rats, guinea pigs and hamsters. Rabbits also are a preferred species. The criteria for choosing an animal will be largely dependent upon the particular preference of an investigator.

The first step is to create a tissue flap in the experimental animal. The term "tissue flap" means any incision in the flesh of the animal that exposes the target tissue. It is generally preferred that an incision be made in the dorsal flank of an animal, as this represents a readily accessible site. However, it will be understood that an incision could well be made at other points on the animal, and the choice of tissue sites may be dependent upon various factors such as the particular type of therapeutics that are being investigated.

Once a target tissue site is exposed, carcinoma cells, either individually or in microscopic tumors, are contacted with the tissue site. The most convenient manner for seeding the cancer cells into the tissue site is to apply a suspension of tissue culture media containing the cells to the exposed tissue. Cancer cell application may be achieved simply using a sterile pipette or any other convenient applicator. Naturally, this procedure will be conducted under sterile conditions.

In one embodiment, 2.5 x 10⁶ SCCHN cells are inoculated into the exposed tissue flap of a nude mouse. Those of skill in the art will be able to readily determine, for a given purpose, what the appropriate number of cells will be. The number of cells will be dependent upon various factors, such as the size of the animal, the site of incision, the replicative capacity of the tumor cells themselves, the time intended for tumor growth, the potential anti-tumor therapeutic to be tested, and the like. Although establishing an optimal model system for any particular type of tumor may require a certain adjustment in the number of cells administered, this in no way represents an undue amount of experimentation. Those skilled in the area of animal testing will appreciate that such optimization is required.

This can be accomplished, for example, by conducting preliminary studies in which differing numbers of cells are delivered to the animal and the cell growth is monitored following resealing of the tissue flap. Naturally, administering larger numbers of cells will result in a larger population of microscopic residual tumor cells.

In the present study the flaps were effectively sealed using mattress sutures. However, it is envisioned that persons skilled in the art may use any of a variety of methods routinely used to seal the incision such as the use of adhesives, clamps, stitches, sutures, *etc.,* depending on the particular use contemplated.

### H. Examples

The following examples are provided merely to illustrate certain specific embodiments and should not be considered as limiting the scope of the invention in any way.

### EXAMPLE 1

### Growth Suppression of Human Head and Neck Cancer Cells by the Introduction of a Wild-Type p53 Gene via a Recombinant Adenovirus

### Materials and Methods

**Cell Lines and Culture Conditions.** Human SCCHN cell lines Tu-138 and Tu-177 were both established at the Department of Head and Neck Surgery, M.D. Anderson Cancer Center. Tu-138 and Tu-177 were established from a gingivo-labial moderately differentiated squamous carcinoma and a poorly differentiated squamous carcinoma of the larynx, respectively. Both cell lines were developed via primary explant technique and are cytokeratin positive and tumorigenic in athymic nude and SCID mice. These cells were grown in DMEM/F12 medium supplemented with 10% heat-inactivated fetal bovine serum (FBS) with penicillin/streptomycin.

**Recombinant Adenovirus Preparation and Infection.** The recombinant p53 adenovirus (Ad5CMV-*p53*) (Zhang *et al.,* 1994) contains the cytomegalovirus (CMV) promoter, wild-type *p53* cDNA, and SV40 polyadenylation signal in a mini-gene cassette inserted into the E1-deleted region of modified adenovirus type 5 (Ad5). Viral stocks were propagated in 293 cells. Cells were harvested 36-40h after infection, pelleted, resuspended in phosphate-buffered saline, lysed, and cell debris was removed by subjecting to CsCl gradient purification. Concentrated virus was dialyzed, aliquoted and stored at -80°C. Infection was carried out by addition of the virus in DMEM/F12 medium and 2% FBS to cell monolayers, the cells were incubated at 37°C for 60 min with constant agitation, then complete medium (DMEM/F12/10% FBS) was added and the cells were incubated at 37°C for the desired length of time.

**Northern Blot Analysis.** Total RNA was isolated by the acid-guanidinium thiocyanate method of (Chomczynski and Sacchi, 1987). Northern analyses were performed on 20 µg of total RNA. The membrane was hybridizated with a *p53* cDNA probe labeled by the random primer method in 5 x SSC/5X Denhardt's solution/0.5% SDS/denatured salmon sperm DNA (20 µg/ml). The membrane was also stripped and reprobed with GAPDH cDNA for RNA loading control. The relative quantities of *p53* expressed were determined by densitometer (Molecular Dynamics Inc., Sunnyvale, CA).

**Western Blot Analysis.** Total cell lysates were prepared by sonicating the cells 24-h post-infection in RIPA buffer (150 mM NaCl, 1.0% NP-40, 0.5% DOC, 0.1 % SDS, 50 mM Tris, pH 8.0) for 5s. Fifty micrograms of protein from samples were subjected to 10% SDS-PAGE and transferred to Hybond-ECL membrane (Amersham). The membrane was blocked with Blotto/Tween (5% nonfat dry milk, 0.2% Tween 20, 0.02% sodium azide in phosphate-buffered saline) and probed with the primary antibodies, mouse anti-human *p53* monoclonal antibody PAb1801 and mouse anti-human β-actin monoclonal antibody (Amersham), and the secondary antibody, horseradish peroxidase-conjugated goat anti-mouse IgG (Boehringer Mannheim, Indianapolis, IN). The membrane was processed and developed as the manufacturer suggested.

**Immunohistochemical Analysis.** The infected cell monolayers were fixed with 3.8% formalin and treated with 3% H₂O₂ in methanol for 5 min. Immunohistochemical staining was performed by using the Vectastain Elite kit (Vector, Burlingame, CA). The primary antibody used was the anti-*p53* antibody PAb1801, and the secondary antibody was an avidin-labeled anti-mouse IgG (Vector). The biotinylated horseradish peroxidase ABC complex reagent was used to detect the antigen-antibody complex. Preadsorption controls were used in each immunostaining experiment. The cells were then counterstained with Harris hematoxylin (Sigma Chemical Co., St. Louis, MO).

**Cell Growth Assay**. Cells were plated at a density of 2x10⁴ cells/ml in 6-well plates in triplicate. Cells were infected with either wild-type (Ad5CMV-*p53*) or replication-deficient adenovirus as a control. Cells were harvested every 2 days, counted and their viability was determined by trypan blue exclusion.

**Inhibition of Tumor Growth *In Vivo.*** The effect of Ad5CMV-*p53* on established subcutaneous tumor nodules was determined in nude mice in a defined pathogen free environment. Experiments were reviewed and approved by institutional committees for both animal care and utilization and for recombinant DNA research. Briefly, following induction of acepromazine/ketamine anesthesia, three separate subcutaneous flaps were elevated on each animal and 5 x 10⁶ cells in 150 ml of complete media were injected subcutaneously into each flap using a blunt needle; the cells were kept in the pocket with a horizontal mattress suture. Four animals were used for each cell line. After 4 days, the animals were re-anesthetized and the flaps were re-elevated for the delivery of 100 ml of 1) Ad5CMV-*p*53 (50 MOI) in the right anterior flap; 2) replication-defective virus (50 MOI) in the right posterior flap; and 3) transport medium alone, in the left posterior flank. All injection sites had developed subcutaneous visual and palpable nodules before treatment was administered. Animals were observed daily and sacrificed on day 20. *in vivo* tumor volume was calculated by assuming a spherical shape with the average tumor diameter calculated as the square root of the product of cross-sectional diameters. Following sacrifice, excised tumors were measured 3-dimensionally by microcalipers to determine tumor volume. A non-parametric Friedman's 2-way ANOVA test was used to test the significance of the difference between means of samples; the SPSS/PC+ software package (SPSS Inc., Chicago, II) was used.

### Results

**Adenoviral Infection of SCCHN Cells.** The conditions for optimal adenoviral transduction of Tu-138 and Tu-177 cells were determined by infecting these cells with adenovirus expressing the E. coli β-gal gene. The transduction efficiency was assessed by counting the number of blue cells after X-gal staining. There appeared to be a linear relationship between the number of infected cells and the number of adenovirus particles used. Cells inoculated with a single dose of 100 MOI β-gal adenovirus exhibited 60% blue cells (FIG. 1), and this was improved to 100% by multiple infections. The transduction efficiency of this vector in SCCHN cells is quite different from that of other cell lines previously examined: HeLa, HepG2, LM2 and human non-small cell lung cancer cell lines showed 97% to 100% infection efficiencies after incubation with 30 to 50 MOI β-gal adenovirus (Zhang *et* al., 1994).

### Expression of Exogenous p53 mRNA in Adenovirus Infected SCCHN

**Cells.** Two human SCCHN cell lines were chosen for this study: both cell lines Tu-138 and Tu-177 possess a mutated *p53* gene. The recently created recombinant wild-type *p53* adenovirus, Ad5CMV-*p53*, was used to infect Tu-138 and Tu-177 cells. Twenty-four hours after infection, total RNA was isolated and northern blot analysis was performed. The transformed primary human embryonal kidney cell line 293 was used as a positive control because of its high level of expression of the *p53* gene product, whereas K562, a lymphoblastoma cell. line with a homozygous deletion of the *p53* gene, was the negative control. The levels of the 2.8-kb endogenous p53 mRNA detected in the samples isolated from mock-infected cells and from the cells infected with a replication-defective adenovirus, d1312 were similar. Up to 10-fold higher levels of exogenous 1.9-Kb *p53* mRNA were present in the cells infected with Ad5CMV-*p53*, indicating that the exogenous *p53* cDNA was successfully transduced into these cells and efficiently transcribed. Interestingly, the level of endogenous *p53* mRNA in these cells was 5-fold higher than in the experimental controls. Northern blots exhibited no evidence of Ad5CMV*-p53* (DNA) contamination of RNA.

**Expression of *p53* Protein in Adenovirus-Infected SCCHN Cells. Western** blot analysis was performed to compare the levels of *p53* mRNA to the amount *of p53* protein produced. A *p53* band, recognized by monospecific anti *p53* antibody, PAb1801, was observed in cellular extracts isolated from all samples except K562 cells. Cell line 293 showed high levels of *p53* protein. Samples isolated from mock-infected Tu-138 and Tu-177 cells exhibited low levels of *p53* protein. The level of *p53* expression remained similar in those cells infected with the d1312 adenovirus. The levels of *p53* antigen detected in Ad5CMV-*p53*-infected cells were significantly higher than the levels of the endogenous mutated proteins in both cell lines. This result demonstrates that the exogenous *p53* mRNA produced from cells infected with AdSCMV *p53* is efficiently translated into immunoreactive*p53* protein. Furthermore, immunohistochemical analysis of cells infected with Ad5CMV-*p53* revealed the characteristic nuclear staining *of p53* protein, while mock-infected cells failed to show similar staining despite the presence of the p53 protein in these cells. This inability to detect the protein may be attributable to the insensitivity of the assay.

**Effect of Exogenous p53 on SCCHN Cell Growth *In Vitro.*** Cells infected with control virus d1312 had growth rates similar to those of the mock-infected cells (FIG. 2A and 2B), whereas, growth of the AdSCMV-*p53*-infected Tu-138 (FIG. 2A) and Tu-177 (FIG. 2B) cells was greatly suppressed. Twenty-four hours after infection, an apparent morphologic change occurred, with portions of the cell population rounding up and their outer membranes forming blebs. These are part of a series of histologically predictable events that constitute programmed cell death. The effect was more prominent for Tu-138 than for Tu-177 cells. Cells infected with the replication-defective adenovirus, d1312, demonstrated normal growth characteristics with no histomorphologic abnormalities. Growth assays were reproducible in four repeated experiments.

**Inhibition of Tumor Growth *In Vivo.*** Four animals were tested for each cell lines. One animal in the Tu-177 group died following the second flap surgery and delivery of the therapeutic interventions, presumably due to profound anesthesia and subsequent mutilation by cage mates. Necropsy revealed no evidence of metastasis or systemic effects. Sizable tumors are apparent on both posterior flaps of the animals (i.e., the sites that did not receive Ad5CMV-*p53*). The lack of tumor progression is significant in the right anterior flaps of the animals, which received Ad5CMV-*p53* (*p* < .04) That Tu-177 cells have a slower growth rate has previously been established in these animals. Two animals in the Tu-138 group were killed early because they were experiencing rapid growth and ulceration of the control tumor sites. All surgical sites had developed lesions of at least 9 mm³ before intervention. The tumor volumes on necropsy are shown in Table 4. Differences in volume were not statistically significant in the Tu-177 group which may be a reflection of the limited sample size.

**TABLE 4Effect of AdSCMV-p53 on Tumor Growth in Nude Mice^{a}**

| Treatment | Mean volume | |
|---|---|---|
| | [mm³ ± SEM] | |
| | TU-138(4) | Tu-177(3) |
| Ad5CMV-*p53* | 36.0 ± 23.5 | 19 ± 14.9 |
| Ad5(d1312) | 1187.5 ± 419.0 | 226 ± 84.0 |
| Medium | 1909.3 ± 776.8 | 454 ± 276.8 |

| Significance | *p* value | *p* value |
|---|---|---|
| *p53*^{b} : d1312 | 0.04 | 0.09 |
| *p53* : Medium | 0.05 | 0.09 |

| | | |
|---|---|---|
| ^{a}The cells were injected subcutaneously at 5 × 10⁶ cells/flap. Tumor sizes were determined at day 20 after treatment. Numbers in parentheses represent the number of animals evaluated. ^{b}AdSCMV-*p53* is abbreviated as *p53;* d1312 as an abbreviation for Ad5(d1312). | | |

### EXAMPLE 2

### in vivo Molecular Therapy with p53 Adenovirus for Microscopic Residual Head and Neck Squamous Carcinoma

### Materials and Methods

**Cell Lines and Culture Conditions.** Human SCCHN cell lines Tu-138, Tu-177, MDA 686-LN, and MDA 886 were all established have been previously characterized (Clayman *et al.,* 1993; Sacks *et al.,* 1988). These cells were grown in Dulbecco's modified Eagle's medium (DMEM/F12) supplemented with 10% heat-inactivated fetal bovine serum (FBS) and penicillin/streptomycin.

**Recombinant Adenovirus Preparation and Infection; Cell Growth Assay; Western Blot Analysis**. All the procedures have been previously described in Example 1. Cell growth assays were all performed in triplicate.

***In Vivo* Transduction with β-Galactosidase Adenovirus.** X-gal staining of tissue specimens were performed on O.C.T. frozen tissue sections to determine transduction efficiency. Eight-micrometer thick specimens were washed in cold PBS and fixed in 0.5 % glutaraldehyde at room temperature for 5 minutes. Slides were then washed twice with 4°C PBS and incubated 4 h in X-gal solution (1.3 mM MgCl₂; 15 mM, NaCl; 44 mM Hepes Buffer pH7.4; 3 mM Potassium ferricyanide; 3 mM Potassium ferrocyanide; 2 % X-gal in DMF). Slides were counterstained with hematoxylin and eosin.

**Immunohistochemical Analysis.** Formalin-fixed paraffin-embedded *in vivo* animal experimental tissues were cut at 4-5 µm, dried at 60°C, deparaffinized, and hydrated with distilled water. Sections were then treated with 0.5% saponin in distilled water and rinsed in several changes of distilled water; endogenous peroxidase activity was blocked with 3% hydrogen peroxide in methanol, followed by rinsing in several changes of distilled water. Sections were microwave-irradiated in distilled water for 3 min using a Sharp Model R9H81 microwave oven operating at a frequency of 2450 MHz at 700 watts. After cooling, sections were washed in several changes of distilled water and placed in PBS; immunochemical studies were performed by using the avidin-biotin-peroxidase complex (ABC) method of Hsu et *al.*, (1981) in the following manner: sections were blocked with normal horse serum and incubated overnight at 4°C with rabbit anti-human *p53* polyclonal antibody, clone OM-1, 1:80 (Signet Laboratories, Denham, MA). An anti-rabbit IgG Elite kit (Vector Laboratories, Burlingame, CA) was then used to apply biotinylated anti-rabbit IgG and ABC complexes, which were incubated for 45 min each. The immunostaining reaction was visualized by using 0.5% DAB in PBS containing 0.01 % hydrogen peroxide (pH 7.6), counterstained with 0.01 % toluidine blue, dehydrated, cleared, and mounted in Permount. To verify the specificity of the immunostaining reaction, immunoperoxidase staining was performed, using the same method as on test samples, on a known positive cytospin of a tissue culture of a squamous carcinoma cell line as well as on a negative rabbit monoclonal antibody control.

**Inhibition of Tumor Growth *In Vivo.*** This procedure was performed as described in Example 1. All surgical sites were evaluated pathologically as well as by necropsy analysis for systemic toxicity.

### Results

**Effect of Exogenous p53 on SCCHN Cell Growth *In Vitro.*** Example 1 described the *in vitro* inhibition of cell growth by Ad5CMV-*p53* in SCCHN cell lines with endogenously mutated *p53*. This present example is to determine whether SCCHN cell lines with endogenous wild-type *p53* would be similarly affected. The effect of Ad5CMV-*p53* on nonmalignant fibroblasts is also investigated.

Four human SCCHN cell lines were chosen for this study: Tu-138 and Tu-177 possess a mutated *p53* gene, whereas MDA 686-LN and 886 both are homozygous for the wild-type *p53* gene. A fibroblast cell line derived from normal fibroblast outgrowth, which is karyotypically normal and nontumorigenic, was used as a nonmalignant control cell line. Cells infected with the control virus, d1312, had growth rates similar to those of the mock-infected cells, whereas the growth of tumor cells infected with Ad5CMV*-p53* was significantly suppressed (FIG. 3A, FIG. 3B, FIG. 3C AND FIG. 3D). Twenty-four hours to forty-eight hours after infection, an apparent morphologic change occurred in all tumor cells, with portions of the cell population rounding up and their outer membranes forming blebs. These are part of a series of histologically predictable events that constitute programmed cell death. The effect occurred earlier in cells with endogenous mutated *p53* than in those with wild-type *p53.* Cells infected with replication-defective adenovirus dl312 demonstrated normal growth characteristics with no histomorphologic abnormalities. Growth assays were reproducible in four repeated experiments.

### Expression of Exogenous p53 Protein in Adenovirus Infected Normal

**Fibroblasts and its Effect on Growth Rate**. Additionally, the effect of the AdSCMV-*p53* on karyotypically normal and non-tumorigenic fibroblast cell lines was also investigated. These cells were isolated during the establishment of primary tumor cell lines. Twenty-four hours after infection, Western blot analysis was performed to compare the levels of protein produced by the different infected cell types. A *p53* band, recognized by the monospecific anti-*p53* antibody, PAb1801, was observed in cellular extracts isolated from all samples infected with the AdSCMV-*p53*. As has been in Example 1, cell line Tu-138 infected with the *p53* adenovirus showed high levels of *p53* protein following transduction and served as a control. The level of *p53* expression remained similar in both mock-infected and d1312 infected cells. The AdSCMV-*p53* infected fibroblasts showed higher levels of *p53* protein than that of the control cells. This result indicates that the *p53* gene is efficiently translated into normal fibroblasts infected with Ad5CMV-*p53* as evidenced by production of immunoreactive *p53* protein. The protein expression and transduction efficiency of cytospins of Ad5CMV-*p53* infected fibroblasts were verified by immunohistochemical analysis. This fibroblast cell line exhibited normal growth rate and morphology, independent of the intervention (mock, replication-defective virus, or Ad5CMV-*p53*) (FIG. 4). These experiments were repeated twice and also verified in other normal human fibroblast cell lines.

**In Vivo Transduction Efficiency.** To measure the efficiency of gene transfer in vivo, the subcutaneous flap site was resected 72 hours following the molecular or control intervention. Dose-response experiments with the adenovirus β-galactosidase marker vector demonstrate dose-response transduction efficiency in this model. This was confirmed with immunohistochemical analysis 4 days following infection with Ad5CMV-*p53*. Both groups of experiments exhibited an *in vivo* dose response which had been previously described *in vitro* (Example 1). In no instances did doses of virus exceeding 10¹⁰ PFU effect expression of *p53* in other organ systems, including brain, liver, lung, heart, abdominal visceral organs, and skin. These experiments illustrated a dose-response relationship between viral titer and transduction efficiency as well as the possibility of achieving extensive transient expression of the transduced gene within the desired surgical model field.

**Suppression of Tumor Growth** ***In Vivo**.* Studies were designed to determine whether *in vivo* Ad5CMV*-p53* mediated gene transfer would affect the establishment or growth of SCCHN cells implanted into a subcutaneous flap. To achieve this aim a microscopic residual disease model was created. In this model, three subcutaneous flaps were elevated on athymic nude female mice and 2.5x10⁶ of tumor cells were seeded by pipetting. Instead of allowing the tumor cells to form nodules (generally occurring in 4 days), a single dose of molecular intervention at 48 hours following tumor cells seeding. In this manner, although no gross tumors were present, microscopic tumor cells were within the surgical site mimicking the clinical dilemma of surgical excision of all gross tumor. The development of tumors was directly related to the number of tumor cells, the time allotted for implantation, and the dose of Ad5CMV-*p53.* Of the mice which received microscopically implanted tumor cells (2.5 x 10⁶) and were treated with Ad5CMV-*p53* at 10⁸ plaque forming units (PFU) or greater, only two developed tumors, both implanted with the wild-type *p53* cell line (MDA 886-LN). All other cell lines exhibited absence of tumor development (Table 5). These experiments clearly demonstrate that the growth of microscopic tumor cells can be effectively suppressed *in vivo* if exposed to the Ad5CMV-*p53*. Tumor formation was evaluated at the end of a 12 week period (earlier animal sacrifice in circumstances of excessive tumor burden) by gross and histologic analysis of the surgical sites. The data of tumor establishment is summarized in Table 5.

**TABLE 5 Effect of Ad5CMV-p53 on Tumorigenicity in a Microscopic Residual Disease Model of SCCHN**

| **Cell Line** | **Treatment No. Mice Developing Tumors/Total Mice Vehicle** | | |
|---|---|---|---|
| | **PBS** | **dl312** | **AdSCMV-*p53*** |
| Tu-138 (homozygous mutation *p53)* | 8/8 | 8/8 | 0/8 |
| Tu-177 (homozygous mutation *p53*) | 8/8 | 8/8 | 0/8 |
| 686-LN (homozygous wild-type *p53)* | 5/8 | 5/8 | 0/8 |
| 886 (homozygous wild-type *p53)* | 6/6 | 6/6 | 2/6 |

Immunohistochemical analysis was performed on the tumor sections of experimental animals. This cell line possesses the wild-type endogenous *p53* gene. There was a lack of significant basal immunostaining with viable tumor of MDA 686-LN (mock infection). 10⁷ PFU AdSCMV-*p53* exhibits peripheral tumor necrosis with immunostaining in the more central portion of the tumor. 10⁸ PFU Ad5CMV-*p53* reveals total necrosis of the tumor with immunostaining found in the entire surgical pocket with multiple layers expressing protein including stroma and superficial muscular layers. 10⁹ PFU Ad5CMV-*p53*) shows similar results to that of 10⁸ PFU Ad5CMV-*p53*, however increased exogenous *p53* expression throughout the surgical site and edema are prominent.

Using animals, which served as their own internal controls, implants of 4.0 x 10⁶ or more cells significantly increased the establishment of subcutaneous implants as compared to the tumor implantation of 2.5 x 10⁶ cells (P < 0.01), even when treated at the surgical site with Ad5CMV-*p53* 48 h after inoculation. Allowing implanted cells to establish for 72 or 96 h prior to the Ad5CMV-*p53* intervention similarly increased tumor take. Dose-response experiments established that 10⁸ and 10⁹ PFU of the Ad5CMV-*p53* were equally effective in inhibiting tumor burdens of 2.5 x 10⁶ cells implanted for 48 h (FIG. 6). Endogenous *p53* status of implanted tumor cell lines (whether homozygous mutated or wild-type *p53)* had little impact on the effectiveness of the Ad5CMV-*p53* in the cessation of tumor development.

### EXAMPLE 3

### Apoptosis Induction Mediated by Wild-Type p53 Adenovirus Gene Transfer in Squamous Cell Carcinoma of Head and Neck

### Materials and Methods

**Cell Lines and Culture Conditions; Recombinant Adenovirus Preparation and Infection.** All procedures were performed and cell lines maintained as previously described in Examples 1 and 2.

**DNA Fragmentation Analysis.** Following incubation with wild-type *p53* adenovirus as well as replication defective adenovirus controls at various time intervals, cells were harvested, resuspended in 300 µl of PBS with the addition of 3 ml of extraction buffer (10 mM Tris, pH 8.0, 0.1M EDTA, 20 µg/ml RNAse, 0.5% SDS) and incubated at 37°C for 1-2 h. At the end of incubation, proteinase K was added to a final concentration of 100 µg/ml and the solution placed in a 50°C water bath for at least 3 h. DNA was extracted once with equal volume of 0.5 M Tris (pH 8.0) saturated phenol then the extraction repeated with phenollchloroform. Precipitated DNA was analyzed in a 1 % agarose gel.

**Cell Fixation.** For TUNEL method, the cells were fixed in 1 % formaldehyde in PBS (pH 7.4) for 30 min on ice. Cells were then washed with 3 ml of PBS, resuspended in 70 % ice-cold ethanol and stored at -20°C until used. For cell-cycle analysis, cells were fixed in 70% ice-cold ethanol only.

**Terminal Deoxynucleotidyl Transferase Assay.** The assay was performed according to Gorczyca *et al.,* procedure (Gorczyca *et al.,* 1993). Briefly, after fixation and washing, cells were resuspended in 50 µl of TdT buffer containing 0.2 M sodium cacodylate (pH 7.0), 2.5 mM Tris-HCl, 2.5 mM C₀Cl₂ (Sigma Chemical Company, St. Louis, MO), 0.1 mM DTT (Sigma Chemical Company), 0.25 mg/ml BSA (Sigma Chemical Company), 5 units of terminal transferase (Boehringer Mannheim Biochemicals, Indianapolis, IN), and 0.5 nmoles biotin-16-dUTP along with dATP,dGTP and dCTP at a concentration of 20 µM. Controls were prepared by incubating a separate aliquot of each test sample without d-UTP. The cells were incubated in the solution at 37°C for 30 min, rinsed in PBS, and resuspended in 100 µl of, FITC, the staining solution containing 4X SSC, 0.1 % Triton X-100 and 2.5 µg/ml fluoresceined avidin (Vector Labs. Inc., Burlingame, CA). Tubes were incubated for 30 min in the dark at room temperature. Cells were rinsed in PBS with 0.1% Triton X-100 and resuspended in 0.5 ml PBS containing propidium iodide (5 µg/ml) and 70 µl (1 mg/ml) RNAse. Tubes were incubated in the dark on ice for 30 min prior to flow cytometric analysis.

**Flow Cytometry Analysis.** All samples were analyzed using an EPICS Profile II flow cytometer (Coulter Corp., Hialeah, FL) with the standard optical configuration. At least 5,000 events were collected for each sample. Positivity for TdT end-labeling was determined by subtracting the control histogram from the test histogram using the immuno-4 program of the Elite workstation software (Coulter Corp., Hialeah, FL).

**Cell Growth Assay.** Cells were plated and growth was monitored as described in Example 1.

***In Vivo* Analysis for Apoptosis.** Gene therapy in a microscopic residual disease model of SCCHN has been described above in Example 2.

***In Situ* End-labeling.** The procedure was performed as previously described (Wijsman *et al.,* 1993). Briefly, paraffin sections were dewaxed in xylene for 5 min three times each, and progressively hydrated by immersing the slides for 3 min each in 100%, 90%, 70% and 30% ethanol solutions. Endogenous peroxidase was inactivated by immersing the slides for 20 min in 0.75 % H₂O₂ v/v in 100% methanol. After the slides were washed in PBS, sections were digested with 0.1 % pepsin (Fisher Scientific, Houston, TX) w/v in 0.1 N HCl for 5 min at 37°C and extensively washed in PBS. Sections were then incubated in a moist chamber at 37°C for 1 h with an end-labeling cocktail including the following: terminal deoxynucleotidyl transferase 0.5 unit/µl; biotinylated dUTP, 0.06 mM; 5X tdt buffer, 10 µl; double-distilled water up to 50 µl. The reaction was terminated by immersing the slides in a buffer containing 300 mM NaCl and 30 mM NaCitrate in double-distilled water. After the slides were washed in PBS, sections were incubated with horseradish peroxide-conjugated avidin for 1 h at 37°C in a moist chamber. Staining was developed using 3,3'-diaminobenzidine and sections were counterstained with methyl green.

### Results

**Growth suppression of SCCHN cell lines by *p53* adenovirus. The** Examples above demonstrate that the wild-type *p53* gene can be efficiently transduced into SCCHN cell lines by a recombinant adenoviral vector. Consequently, the insulted tumor cells lose their ability to proliferate *in vitro* as well as *in vivo.* The suppression effect is independent of the endogenous *p53* status of the cell lines. Previous growth rate analyses were carried out through a week period. The present example investigates the early effects of the wild-type *p53* on SCCHN cells growth (i.e. earlier time intervals, hours).

Two representative cell lines were used in this study. Cell line, Tu-138, harbors a mutated *p53* gene whereas cell line, MDA 686LN, possesses a wild-type *p53* gene. Cells infected with replication-defective virus, d1312, had growth rates similar to those of the mock-infected cells (FIG. 5A and FIG. 5B). On the other hand, growth of the Ad5CMV-*p53* infected Tu138 (FIG. 5A) and MDA 686LN (FIG. 5B) cells were significantly suppressed. It appeared that the exogenous *p53* protein had an earlier and more profound growth suppression of the Tu138 as compared to the MDA 686LN. An apparent morphologic change was observed, with portions of the cell populations rounding up and their outer membranes forming blebs, resembling apoptosis, which occurred concomitantly with the initiation of the growth suppression. Cells infected with the replication-defective adenovirus, d1312, demonstrated normal growth characteristics with no histomorphologic abnormalities. Importantly, these effects were not observed following the *p53* adenovirus infection of karyotypically normal fibroblasts, as detailed in Example 2 above, as well as human oral keratinocytes (immortalized but nontumorigenic).

**DNA fragmentation analysis.** One of the characteristic markers in apoptosis that distinguishes it from necrosis is the biochemically observable appearance of the ladder of DNA fragments. To confirm the notion that the cells had undergone apoptosis following the *pS3* adenovirus irlfection, performed DNA fragmentation analysis was performed. Chromosomal DNA extracted from the viable cells following the replicationdefective or the wild-type *p53* adenovirus infection were subjected to agarose gel electrophoresis. The appearance of DNA fragments equivalent to approximately 200 bp and their multiples was noticed in both cell lines. The fragmented DNA appeared at 22 h following the *p53* adenovirus infection in Tu-138 cell line whereas in the MDA 686LN cell line, fragmented DNA were visible at 30 h and more evident at 48 h following the wild-type *p53* adenovirus infection. No detectable fragmented DNA emerged from the mock-infected and d1312-infected cells.

***In Vitro* Terminal Deoxynucleotidyl Transferase Assay. Another** characteristic marker of apoptosis is the morphological change and destruction of the structural organization of the nucleus which results in chromatin condensation. Electron microscopy has been used extensively to detect such ultra-structural alteration. However, recently flow cytometry methods for identifying apoptotic cells have gained favor due to the ability to scan and analyze cellular populations as compared to electron microscopy (Gorczyca *et al.,* 1993). The inventor employed here the TUNEL (terminal deoxynucleotidyl transferase-mediated dUTRbiotin nick end-labeling) method (Gorczyca *et al.,* 1993) which is based upon detection of the extensive DNA breakage to identify the apoptotic cells. Fifteen h following the *p53* adenovirus infection, 4.4% of the viable Tu-138 cell population were in apoptotic stages as compared to none of the MDA 686LN (FIG. 6A and FIG. 6B). The number of apoptotic cells increased proportionally as the duration of observation was also increased following *p53* adenovirus incubation. Nearly 31 % of the Tu-138 cells had undergone apoptosis at 22 h. Although delayed in initial induction of apoptosis, approximately 60 % of the MDA 686LN cells were in apoptotic stages 48 h following the *p53* adenovirus infection. Noteworthy, the percentage of apoptotic cells as determined by Tunel method may be significantly underestimated since only viable cells were subjected to the analysis. These data correlated well with the growth rate and DNA fragmentation analyses. There were no detectable cell populations undergoing apoptosis in control experiments using mock infection as well as replication defective virus controls (100 M.O.I.). Therefore, the apoptosis was not the function of transduced adenoviral gene products themselves.

***In Vivo* Analysis for Apoptosis.** The examples above show that the *p53* adenovirus suppresses tumor formation *in vivo*. This example was designed to show whether the suppression of tumor growth *in vivo* was the consequence of apoptosis. *In situ* end-labeling analysis was performed to detect apoptotic cells in paraffin embedded sections obtained from example 2. Clearly, no staining was observed in the tissue sections isolated from MDA 686LN bearing animals which had received PBS treatment as a control. On the other hand, tissue sections isolated from MDA 686LN bearing mice treated with the wild-type *p53* adenovirus showed highly positive staining, demonstrating that apoptosis indeed was the event involved in suppression of tumor growth *in vivo.*

Further to these studies the inventor sought to determine whether the growth suppression is, in part, due to the cell cycle arrest by the induced p21 protein or primarily a result of apoptosis. Western blotting showed that the p21 protein was induced in the wild-type *p53* adenovirus infected SCCHN cells. However, cell cycle analyses indicated that despite the elevated level of p21 protein in the *p53* adenovirus infected cells, there was no significant accumulation of cells at the G₁ stage when compared to S phase.

### EXAMPLE 4

### Growth Suppression of Head and Neck Squamous Cells by p53-FLAG: an Effective Marker for Gene Therapy Trails

### Materials and Methods.

**Cell Lines and Culture Conditions. Recombinant Adenovirus Preparation and Infection; Northern Blot Analysis; Western Blot Analysis; Cell Growth Assay; Immunohistochemical Staining *in vitro* Cell Layers.** All procedures were performed and cell lines maintained as described in Example 1.

**Generation of the *p53*-FLAG Adenovirus.** The *p53* cDNA sequence was excised from the *pC53-*SN by digestion with *Bam*H1 and cloned into the *Bam*H1 site of pGEM7Z. A recombinant plasmid with the proper insert orientation was then digested with Acc1 and Kpn1 to remove 22 amino acids from the 3' end of *p53* cDNA. A linker with Acc1-Kpn1 compatible ends containing the sequence of the FLAG peptide including a stop codon was then ligated into the digested plasmid to create the *p53*-FLAG fusion gene. The resulting *p53*-FLAG fusion gene was then cloned into an expression vector with the human CMV promoter and SV polyadenylation signal. The final construct was subsequently inserted into a shuttle vector pXCJL.1 ((Zhang *et al.,* 1994) to generate a recombinant *p53*-FLAG adenovirus.

***In* Vivo Microscopic Residual Disease Experiments**. The studies were carried out in a defined pathogen free environment using the athymic nude model system described in Example 1. Two different sets of repeated experiments were performed. The first was a dose-response experiment using the AdCMV-*p53*-FLAG virus in three of the flaps at descending concentrations (10¹⁰ pfu, 10⁹ pfu, 10⁸ pfu). The fourth flap served as a control and was randomized to either PBS or the replication defective adenovirus (DL312). The second study was performed using 10¹⁰ pfu of AdCMV-*p53*-FLAG, AdCMV-*p53*, and replication defective adenovirus in three separate flaps. The fourth flap was inoculated with the same volume (100 µl) of sterile PBS. Forty eight hours after treatment, two of these animals were sacrificed and the flaps harvested for immunohistochemical analysis. The remaining animals were observed for 21 days and then sacrificed. Tumor volumes were measured for comparison using calipers.

### Results

**Expression of mRNA after Infection with AdCMV-*p53* and AdCMV*-p53-*FLAG Virus.** Both Tu-138 and MDA 686-LN were examined for expression of *p53* mRNA. Total RNA was isolated after adenovirus infection. Northern blot analysis was performed. Similar levels of exogenous AdCMV-*p53* mRNA were detected between AdCMV-*p53* and AdCMV-*p53*-FLAG infected cells. The level of *p53* mRNA expression following infection with AdCMV-*p53* and AdCMV-*p53*-FLAG were comparable for Tu-138 and for MDA 686-LN. Variation of intensity is felt to be related to loading dose. Endogenous expression of *p53* mRNA is seen in lanes 2 and 3 in the mutated *p53* cell line, Tu-138. There was no significant endogenous *p53* mRNA expression in the MDA 686-LN cell line, which is wild-type for the *p53* gene. These data suggest that the AdCMV-p53-FLAG virus, like the AdCMV-*p53* virus, is successfully transduced and efficiently transcribed. Northern analysis did not reveal evidence of AdCMV-*p53*, DNA contamination.

**Expression of Exogenous *p53* Protein in AdCMV-*p53* and AdCMVp53-FLAG Infected SCCHN Cell Lines.** Western blot analysis was performed to compare the amount of protein expressed by the AdCMV-*p53* infected and AdCMV-p53-FLAG infected cells. Protein bands were identified using the monospecific *p53* antibody (PAb1801) and the anti-FLAG M2 antibody (IB13025) on two simultaneously run gels. Using the *p53* antibody (pAB1801), a similar high level of *p53* protein expression in both cell lines that were infected with the AdCMV-*p53* and AdCMV-*p53*-FLAG. The Tu-138 and MDA 686-LN cells infected with the AdCMV-*p53*, were also tested. No change in *p53* protein expression was, noted in either the replication defective adenovirus infected cells or in the mock infection group. When a similarly executed gel that was probed with the mouse anti-FLAG M2 antibody. The level of *p53*-FLAG protein expression appeared to be similar to that expressed following *p53* antibody probing, but no detectable band was noted in those cells infected with the AdCMV-*p53* virus. The mock and DL312 infected cells exhibited no detectable level of the immunoreactive *p53* or FLAG protein in either cell line.

**Effect of AdCMV-*p53* and AdCMV-*p*53-FLAG on SCCHN Cell Growth *In Vitro.*** The cytotoxic effect of wild-type *p53* therapy in Tu-138 and MDA 686-LN cell lines has been detailed above. The Tu-138 cell line has an endogenously mutated *p53* gene and the MDA 686-LN cell line possesses the wild-type *p53* gene. This study sought to determine if any difference in efficacy would be seen following recombination of the AdCMV-*p53* virus by inserting the FLAG sequence.

Cells infected with the replication defective adenovirus had a similar growth rate to the mock infection cells. A mild cytotoxic effect may be seen with the replication defective adenovirus (FIG. 7A). In contrast, those cells infected with either the AdCMV-*p53* or AdCMV-*p53*-FLAG experienced virtual total tumor cell death by day three. Histologic examination revealed bleb formation by the plasma membrane which is the characteristic feature of apoptosis and has been characterized as the mechanism of cell death in AdCMV-*p53* infected SCCHN cell lines (Example 1). As noted above, the effect was more prominent for the Tu-138 cell line (mutated *p53*) than it was for the MDA 686-LN cell line (wild-type *p53*). Growth curve assays were reproducible in three repeated studies without a significant difference being noted between the effect of the AdCMV-*p53* and the AdCMV-*p53*-FLAG viruses, suggesting that the addition of the FLAG peptide did not affect the ability of *p53* in suppression of cell growth.

**Immunohistochemical Staining of SCCHN Cell Lines Infected with Adenovirus**. Infected cell monolayers were compared for expression of the *p53* and p53-FLAG protein using standard immunohistochemical techniques. Neither the *p53 .* nor FLAG protein could be clearly identified in the mock infection of DL312 infected cells in the MDA 686-LN cell line. However, in Tu-138, which has a mutated *p53* gene, endogenous staining for *p53* was positive. When cells were infected with the AdCMV-*p53* virus a strong staining was noted in both cell lines. Visual inspection of those cells infected with the AdCMV-*p53*-FLAG virus showed identical intensity of staining and number of positive cells with PAb1801 for antibody as compared to the cells infected with the AdCMV-*p53* virus. The cells infected with the AdCMV-*p53-*FLAG virus also showed strong immunohistochemical positivity with the M2 FLAG antibody. The quality of staining was different though, both within the nucleus and to a lesser degree in the cytoplasm.

***In Vivo* Suppression of Growth.** Dose response studies using 10⁸, 10⁹, and 10¹⁰ plaque forming units (pfu) of AdCMV-*p53*-FLAG virus compared to a control flap that was either PBS or DL312 were performed using the microscopic model method described in Example 1 on the Tu-138 cell line. The mean tumor size for the mock infection was 1205 +/- 205 mm³. Tumor size decreased in a linear fashion with increasing concentration of virus used in the molecular intervention. Mean tumor size was 637 +/-113 mm³, 392 +/- 109 mm³, and 193 +/- 74 mm³ for those flaps treated with 10⁸, 10⁹, and 10¹⁰ pfu of the AdCMV-*p53*-FLAG respectively. Each animal was compared against itself using a paired t test and a significant dose response effect was noted at p < 0.05 in all comparisons except between the flap treated with 10⁹ and 10¹⁰ pfu. Clearly, the greater the amount of virus, the greater the tumor growth inhibition visualized. In an additional study the effects of AdCMV*-p53* were compared to that of the AdCMV-*p53*-FLAG. No significant difference in activity was noted.

**Immunohistochemical Demonstration of Exogenous Tumor Suppression Effect in the Microscopic Residual Disease Animal Model. After proving** comparable *in vitro* and *in vivo* activity of the AdCMV*-p53* and the AdCMV*-p53-*FLAG, the inventor applied immunohistochemical techniques to demonstrate p53-FLAG fusion protein product *in vivo.* Using the Tu-138 and MDA 686-LN cell lines, microscopic residual disease flaps were harvested 48 hours after treatment, fixed in formalin, and paraffin-embedded. On neighboring sections of tumor cells treated with the AdCMV-*p53*-FLAG virus, staining for both the *p53* and FLAG protein were applied. Staining intensity and the number of cells staining positively was directly proportional to the amount of virus used in the infection. Controls were negative for staining with both *p53* and FLAG antibodies in the MDA 686-LN cells. Endogenous staining for *p53* was noted in the Tu-138 tumor cells. A histologic specimen stained with Hematoxylin and Eosin, the *p53* antibody, and the FLAG antibody. The characteristic cytoplasmic staining with the FLAG M2 antibody contrasted with the intranuclear staining of the *p53* antibody. This is the first time that the FLAG M2 antibody has been proven to be effective on paraffin-embedded fixed tissue. The staining demonstrates that the tumor suppressive effect is directed by the exogenous therapy, and that in an *in vivo* model one can identify the exogenous therapy using the applied FLAG system.

In conclusion, it is clear that the co-delivery of the FLAG protein along with the desired gene therapy offers potential utility as a marker of gene therapy. The invention clearly shows that it was simultaneously promoted along with the *p53* gene and that expression of the messenger RNA and protein were not decreased. More importantly, the biologic activity of the delivered tumor suppressor gene was not altered. For the first time, the FLAG antibody was proven effective when immunohistochemical analysis was performed on formalin-fixed paraffin-embedded tissue. These factors suggest the utility of this novel protein as a tracer in further gene therapy studies.

### EXAMPLE 5

### Treatment of Squamous Cell Carcinoma of the Head and Neck Using p53-Adenovirus

### Patient A

A 53 year-old male patient presents with an inoperable SCCHN tumor of the head. The tumor mass is approximately 6.5 cm diameter. Following examination of bone marrow function, platelet count and renal function, the patient receives a first treatment with 10⁸ infectious particles of an adenovirus-*p53* expression construct, diluted in sterile phosphate buffered saline, via eight distinct intratumoral injections (total volume 10 ml). Every three days, the patient receives an identical treatment, until a total of six treatments have been given.

Three days after the sixth treatment, the tumor is examined and found to be > 4.0 cm in diameter. Histologic examination shows considerable cell fragmentation at the tumor margin. A second course of six treatments in undertaken, following which the tumor is found to be > 2.0 cm in diameter and necrotic. The patient continues to receive one a week treatments for three months, at which time the tumor is no longer evident.

### Patient B

A 44 year-old female patient presents with an operable SCCHN tumor of the neck. The tumor mass is approximately 2.5 cm diameter. Following examination of bone marrow function, platelet count and renal function, the patient receives a first treatment with 5 X 10⁷ infectious particles of an adenovirus-*p53* expression construct, diluted in sterile phosphate buffered saline, via three distinct intratumoral injections (total volume 3 ml). Every three days, the patient receives an identical treatment, until a total of six treatments have been given.

Three days after the sixth treatment, the tumor is excised. The tumor bed is bathed in 6 ml of sterile phosphate buffered saline for 60 min. The inoculum is removed, the wound closed, and a drain left in the tumor bed. On days 4, 7, 10 and 14 following surgery, 5 X 10⁷ infectious particles of an adenovirus-*p53* expression construct, diluted in sterile phosphate buffered saline (total volume 3 ml) are infused via the drain. After contacting the tumor bed for two hours, the inoculum is removed by suction. Sixth months following termination of treatment no primary, local or regional tumors are observed.

### EXAMPLE 6

### Wild-Type P53 Gene Transfer Via An Adenoviral Vector In A Phase I Trial Of Patients With Advanced Recurrent Head And Neck Squamous Carcinoma

An adenoviral vector containing the normal "wild-type" p53 gene was delivered in logorithmically increasing doses in patients with biopsy-confirmed recurrent squamous carcinoma of the head and neck. Direct tumoral injections were performed three times weekly for two consecutive weeks. Patients were stratified into two groups: 1) resectable recurrent disease, 2) unresectable recurrent disease.

Those patients stratified into the resectable disease group underwent total gross surgical resection of their recurrent neoplasm 72 hours following the sixth gene transfer events over a 2-week period. The adenoviral vector also was delivered intraoperatively and 72 hours following the surgical procedure via retrograde catheter infusion. Unresectable patients underwent repeat gene transfer attempts via direct tumoral injections monthly over two week cycles until disease progression or deterioration in patient performance status was observed. The safety of this treatment was monitored by close hospital observation, biopsies to assess gene transfer efficiency, bodily fluid analysis for shed vectors and necropsy analysis.

### Methods

**Study Subjects.** Twenty-one patients with advanced recurrent squamous carcinomas of the upper aerodigestive tract with an Eastern Cooperative Oncology Group performance status 2 patients were entered into one of two study arms consisting of patients with resectable (Group 1) or non-resectable (Group 2) recurrent malignancies. The characteristics of the study subjects and dosage of adenoviral vector are shown in Tables 6 and 7, respectively. The women all had negative pregnancy tests and all patients used contraceptive methods. Informed consent was obtained from all patients prior to study entry.

**Gene-Transfer Vector.** The present study employed a replication-defective adenovirus serotype 5 vector with an enhancer (cytomegalovirus)-promoter designated Ad5CMVp53. Three lots of adenoviral vector with plaque-forming units (PFU) ranging from 10⁹ to 10¹¹ were produced by good manufacturing practices at Magenta, Inc. and Introgen Therapeutics, Inc. and shipped frozen (-70°C) to the University of Texas, M.D. Anderson Cancer Center. Each lot was efficacious for transduction utilizing western blotting as well as an *in vitro* tumor cell suppression growth assays. Vector was thawed and diluted in phosphate-buffered saline (vehicle) immediately prior to gene transfer and transported to patient rooms at 4°C.

**TABLE 6 Patient Characteristics**

| **Pt. No.** | **Age** | **Gender** | **Primary Cancer** | **Pathology** | **p53 genotype** | **Prior Failed Therapies** | **Site of Gene Transfer** | ***Study Arm** |
|---|---|---|---|---|---|---|---|---|
| 1 | 31 | F | Floor of Mouth | Moderately diff. SCC | MAA | surgery, xrt, chemo | Facial mass | 1 |
| 2 | 58 | M | Larynx | Moderately diff. SCC | WT | surgery, xrt | Submental mass, nasopharynx | 1 |
| 3 | 56 | M | Pyriform Sinus | Poorly differential | MAA | surgery, xrt | Left neck mass | 2 |
| 4 | 42 | F | Base of tongue | Poorly diff. SCC | WT | surgery, xrt | Right neck mass | 2 |
| 5 | 71 | M | Unknown | Poorly diff. SCC | WT | surgery, xrt, chemo | Left neck mass | 1 |
| 6 | 43 | M | Cervical, esophasgus | Poorly diff. SCC | MAA | surgery, xrt, chemo | Peristomal mass | 2 |
| 7 | 63 | M | tonsil | Moderately diff. SCC | WT | xrt, chemo | Left neck mass | 2 |
| 8 | 46 | M | Base of tongue | Moderately diff. SCC | MAA | surgery, xrt | Base of tongue | 1 |
| 9 | 57 | M | Larynx | Well diff. SCC | WTT | surgery, xrt | Peristomal Recurrence | 2 |
| 10 | 58 | M | Larynx | Moderately diff. SCC | MAA | surgery, xrt | Hypopharyng eal mass | 1 |
| 11 | 46 | M | Base of tongue | Poorly diff. SCC | MAA | xrt, chemo | Base of tongue | 1 |
| 12 | 57 | F | Floor of mouth | Moderately diff. SCC | WT | surgery, xrt | Left retromolar trigone | 2 |
| 13 | 66 | M | Base of tongue | Moderately diff. SCC | WT | surgery, xrt | Posterior ora tongue & tongue base | 2 |
| 14 | 48 | F | Floor of mouth | Poorly diff. SCC | WT | surgery, xrt | Floor of mouth | 2 |
| 15 | 63 | M | Mandible alveolar ridg | Moderately. diff. SCC | WT | surgery, xrt, chemo | Facial mass | 2 |
| 16 | 76 | F | Larynx | Poorly diff. SCC | MAA | xrt | Supraclavicul ar mass | 1 |
| 17 | 56 | M | Larynx | Moderately diff. SCC | WT | surgery, xrt, chemo | Base of tongue, tonsillar foss | 2 |
| 18 | 56 | M | L. Lateral pharyngeal wall | Moderately diff. SCC | WT | chemo | Facial neck mass | 2 |
| 19 | 53 | M | Unknown | Poorly diff. SCC | WT | surgery, xrt, chemo | Submental mass | 1 |
| 20 | 55 | M | Tongue | Poorly diff. SCC | WT | surgery, xrt, chemo | Neck mass | 2 |
| 21 | 66 | M | Base of tongue | Well diff. SCC | WT | chemo, xrt | Neck Mass | 2 |

**TABLE 7 Vector Dosing and Presence or Absence of Adenoviral Vector Nucleotides in Serum or Urine During Treatment**

| **Patient** | **PFU/ Transfer** | **Volume Injected** | **Gene Transfer Cycles** | **Site of Injections** |
|---|---|---|---|---|
| 1 | 10⁶ | 6cc | 2 | Facial mass |
| 2 | 10⁶ | 3cc | 2 | Submental mass, neopharynx |
| 3 | 10⁶ | 3cc, 4cc | 1 | Left neck mass |
| 4 | 10⁶ | 3cc, 5cc | 1 + 3 | Right neck mass |
| 5 | 10⁶ | 1.5cc | 1 | Left neck mass |
| 6 | 10⁶ | 3cc | 1 | Peristomal mass |
| 7 | 10⁷ | 2cc | 4 + 2 | Left neck mass |
| 8 | 10⁷ | 3cc | 1 | Base of tongue mass |
| 9 | 10⁷ | 1.5cc | 1 | Peristomal recurrence |
| 10 | 10⁸ | 1.5cc | 1 | Hypo-pharyngx mass |
| 11 | 10⁸ | 3cc | 1 | Base of tongue |
| 12 | 10⁸ | 1.5cc | 3 | Left retromolar trigone |
| 13 | 10⁹ | 1.5cc | 6 | R. Posterior tongue & tongue base |
| 14 | 10⁹ | 1.5cc | 1 + 1 | Floor of mouth |
| 15 | 10⁹ | 3cc | 1 | Left facial mass |
| 16 | 10⁹ | 1.5cc | 1 | L. Supraclavicular mass |
| 17 | 10⁹ | 1.5cc | 1 | Base of tongue, tonsillar fossa |
| 18 | 10⁹ | 3cc | 4 | Left facial mass |
| 19 | 10^{9.5} | 1.5cc | 1 | Submental mass |
| 20 | 10^{9.5} | 3cc | 3 | Left neck mass |
| 21 | 10^{9.5} | 1.5cc | 3 | Right neck mass |

**Dosage.** The adenoviral vector was administered to five cohorts of patients each, and logarithmically increasing doses. Dosage increases were established following two weeks of observation of the last patient treated at the prior dose. Following entry of the first six patients into the study, three patients were entered at each dosage level independent of the resectable or unresectable group the patient had been stratified to. The dose of biologic vector is described in terms of the total dose (in plaque-forming units). The estimated number of vectors administered per malignant epithelial cell was not approximated. The total volume of administration is shown in Table 7. The volume of adenoviral vector injected into the solid malignancies was determined by the clinical and radiographic estimated tumor volume. The vector was directly injected into the recurrent squamous carcinomas under direct vision and by manual palpation. Injections were spaced at 1 centimeter increments across masses. After gene transfer, the subject remained under close observation for at least 1-1/2 hours. Respiratory and body secretion isolation were maintained for 72 hours following the final gene transfer of vector.

**Detection of Vector.** The urine and serum samples were monitored for disseminated adenoviral vector utilizing viral culture of 293 cells as well as polymerase chain reactions (PCR) using primers that amplify the Elb region of the adenovirus and the 5' end of the wild-type p53 gene which were specific to the vector. PCR products were then southern transfered to improve viral detection, to 1-5 viral particles as well as verify PCR product specificity. Positive and negative controls were assayed in each reaction.

**Safety.** The symptoms, vital signs, blood counts were monitored, and the patients were physically examined and photographically documented daily. Chest radiography, blood chemistry testing, and performance status analysis were performed at the beginning of each treatment cycle. Serum titers of adenoviral antibody were measured before and following each cycle of gene transfer. Three days following the sixth gene transfer of the first cycle, tumor biopsies (or surgical resection) were obtained. Specimens were stored as snap-frozened, pathologically embedded specimens, as well as formalin-fixed specimens in every instance.

**Extraction of Nucleic Acids from Serum or Urine**. Ad5CMV-p53 adenoviral DNA was extracted from 0.5-ml aliquots of serum or urine by a method modified from Cunningham *et al.,* (1995). Briefly, distilled water was added to make 1 ml, and they were precipitated with 30% polyethyleneglycol (PEG). Since SDS alone was not sufficient to release the viral DNA from its particle, proteinase K was added to the SDS at 50°C for 2-16 hours following the PEG precipitation (Norder et *al.,* 1990). The samples were extracted with phenol, and viral DNA was precipitated with ethanol in the presence of glycogen *(Cunningham et al.,* 1995).. The precipitated DNA was recovered by centrifuging at 14000 g for 10 minutes at 4°C, resuspended in 0.3 ml of distilled water, and reprecipitated with ethanol. The DNA pellet was rinsed with 70% ethanol, vacuum-dried, and dissolved in 10 µl of distilled water. Samples were either analyzed immediately or stored at -20 until used. Extraction of nucleic acids was performed under biologic safety cabinet hoods to prevent possible cross-contamination of specimens.

**PCR Reactions on DNA Isolated from Serum Samples**. Primers were designed for specific amplification of the p53 gene from the adenoviral vector. The upper primer (5'-CACTGCCCAACAACACCA-3', SEQ ID NO:5) corresponds to the 3' end of the p53 gene and the lower primer (5'-GCCACGCCCACACATTT-3' SEQ ID NO:6) corresponds to the E1B region of adenovirus type 5 (nucleotides 3517 to 3533 of the wild-type sequence). Each PCR reaction tube contained 0.2 mM of each oligonucleotide, 0.4 mM dNTPs, 1X TaqPlus Long low salt buffer (from Stratagene), 0.6 µl TaqPlus Long (5U/ml) (from Stratagene), and 5 µl of test DNA. The samples were placed in an MJ Research Peltier Thermal Cycler (PTC-200) programmed for 93°C for 3 minutes, with the following three-step profile: 93°C for 30 seconds, 65°C for 45 seconds and 72°C for 45 seconds for a total of 30 or 35 cycles. 5 ul of 6X loading buffer (0.25% bromophenol blue, 0.25% xylene cyanol FF and 15% Ficoll (Type 400; Pharmacia) in water) was added to each tube at the end of the PCR run and loaded onto a 1% agarose, 1X TBE gel containing ethidium bromide (.6 µg/ml). The samples were electrophoresed at 100V for 1-1.5 hours and then photographed under UV light.

**Polymerase Chain Reaction (PCR).** Only 5 µl of prepared DNA could be used in a single polymerase chain reaction. For serum, the PCR was performed in a 20-µl volume containing 2 mM MgCl₂, 50 mM KCl, 0.1% Triton X-100, 200 µM each of deoxyribonucleoside triphosphates (dNTP), 10 mM Tris-HCl (pH 9.0), 5 µM each of the primers, and 1.7 units *of Taq* DNA polymerase (Promega). The reactions were carried out at 94°C for 30 sec, 58°C for 30 sec, and 72°C for 60 sec for 35 cycles, followed by a 10-min extension at 72°C. The PCR primers were selected from the sequence of the Ad5CMV-p53 with the sense primer located at the 3' end of the p53 cDNA (5'-GCCTGTCCTGGGAGAGACCG-3', SEQ ID NO:7), and the antisense primer was selected from the E1B region of adenovirus type 5 (5'-CCCTTAAGCCACGCCCACAC-3', SEQ ID NO:8). The PCR product (an 838-bp fragment) was separated on 1% agarose gel. The same PCR product was subcloned into pCR-Script vector (Stratagene), sequenced, and the gel purified insert was used as a probe to detect the PCR product. For urine, the PCR was performed in a 20-µl volume containing 2 mM MgSO₄, 10 mM (NH₄)₂SO₄, 10 mM KCI, 0.1% Triton X-100, 20 mM Tris-HCl (pH 8.8), 0.1 mg/ml bovine serum albumin, 200 µM each of deoxyribonucleoside triphosphates (dNTP), 5 µM each of the primers, and 2.5 units of *TaqPlus* long DNA polymerase (Stratagene). The reactions were carried out at 93°C for 60 sec, and then 93°C for 30 sec, 65°C for 45 sec, and 72°C for 45 sec for 35 cycles, followed by a 10-min extension at 72°C. The PCR primers were selected from the sequence of the Ad5CMV-p53 with the sense primer located at the 3' end of the p53 cDNA (5'-CACTGCCCAACAACACCA-3', SEQ ID NO:9), and the antisense primer was selected from the E1B region of adenovirus type 5 (5'-GCCACGCCCACACATTT-3', SEQ ID NO:10). The PCR product (an 724-bp fragment) was separated on 1% agarose gel.

**Southern Blot Analysis**. In the Southern blot used to verify the PCR product's specificity, the DNA in the gel was denatured and neutralized before blotting to nylon membrane (Hybond-N+, Amersham) by capillary absorption. The membrane was prehybridized for 15 min at 65°C in Rapid-hyb buffer (Amersham) and hybridized in the same buffer containing ³²P-labeled probe for 1-2 h. The membrane was washed in 0.1 x SSC and 0.1 % SDS at room temperature twice, and again twice at 65°C (15 min per wash). The washed membrane was exposed to x-ray film at -70°C for 1-16 h with intensifying screen.

**Controls and scoring of test samples.** The following controls were included with every batch of samples. At the DNA isolation step, two "negative" serum controls (pooled and aliquoted serum from Introgen employees), and two positive controls consisting of negative serum spiked with 10pfu or 100 pfu of AdCMV-p53 virus were employed. This was done to obtain a window of sensitivity where the 10 (and 100) pfu controls were positive, but the negative controls were negative. If the negative controls were positive, the PCR was repeated with only 30 cycles. If the 10 pfu control was negative, the DNA was further purified with an additional ethanol precipitation, and the PCR was repeated. The above two steps always placed the experimental parameters into the appropriate sensitivity window.

At the PCR stage, a positive control of 1 ng of AdCMV-p53 DNA (isolated from a clinical lot), and a negative (H₂0) control were employed. The PCR of the batch was repeated if any of the controls were false. There were no failed negative PCR controls.

For confirmation of putative positives, the DNA was re-isolated from serum (along with several temporally adjacent samples), and the PCR of this DNA repeated. Samples were scored as positive only if the results could be reproduced. Those samples which were positive in one of two sample analyses were considered negative for reporting purposes. Putative positives that could not be repeated (due to lack of further unprocessed specimen) were omitted from the database.

**Measuring Efficacy of Gene Transfer.** Surgically removed tissue specimens were placed in cryovials, immediately flash frozen, and then stored in liquid nitrogen storage until use. Frozen samples were decanted into the orifice of a stainless steel Bessman tissue pulverizer (Spectrum, Houston, TX) that had been pre-cooled by immersion in liquid nitrogen. Tissue was crushed to a fine powder by striking the Bessman pestle with a steel hammer five to ten times. Pulverized tissue was transferred to a glass tissue homogenizer (Fisher Scientific, Pittsburgh, PA) containing 1 ml TRI reagent (Molecular Research, Cincinnati, OH) per 50 mg tissue, and homogenized by five to ten up and down strokes with a Teflon pestle.

Following homogenization, RNA was isolated according to the instructions provided with the TRI reagent. Briefly, homogenates were transferred to polypropylene centrifuge tubes (Molecular Research) and stored for 5 min at room temperature prior to the addition of chloroform (0.2 ml per 1 ml of TRI reagent). Samples were then mixed vigorously, incubated an additional 15 min at room temperature, and centrifuged at 12,000 x g for 15 min at 4°C to separate the RNA containing aqueous layer from the phenol-chloroform phase. Isopropanol was added to the aqueous phase and RNA was precipitated by incubating at room temperature for 15 min. RNA pellets were recovered by centrifugation at 12,000 x g for 15 min at 4°C, washed once with 75% ethanol, air dried, dissolved in diethyl pyrocarbonate (DEPC)-treated water and quantitated by measuring the absorbance at 260 nm. Contaminating DNA was removed by incubating up to 50 µg RNA with 60 U DNase I (Pharmacia, Piscataway, NJ) for 25 min at 37°C in a total reaction volume of 260 µl. RNA was then extracted with phenol:chloroform, ethanol precipitated, washed once with 75% ethanol, pelleted by centrifugation at maximum speed in a microfuge for 15 min at 4°C, air dried, resuspended in DEPC-water, and stored at -80°C. The quality of RNA was assessed by running samples on an ordinary non-denaturing 0.8% agarose gel and visualizing the 28S and 18S ribosomal bands by ethidium bromide staining. To eliminate cross-contamination between samples and to minimize RNase activity, all re-usable instruments employed for RNA isolation were soaked in a 2% Liqui-Nox (Fisher Scientific) detergent solution for a minimum of 5 minutes, scrubbed free of debris, transferred to a 10% bleach solution for 3 minutes, rinsed thoroughly with deionized water, sprayed with 100% ethanol, dried, immersed in chloroform, and dried again before use.

Reverse transcription was performed using 1.5 µg total cellular RNA in a 23.5 µl reaction mix containing 111 ng random hexamers (Gibco BRL, Grand Island, NY), 40 units RNase inhibitor (Boehringer Mannheim, Indianapolis, IN), 0.4 mM of each dNTP (Perkin Elmer, Foster City, CA), and 300 units of Superscript II RNase H⁻ reverse transcriptase (Gibco BRL) in 1 x RT buffer (50 mM Tris pH 8.3, 75 mM potassium chloride, 3 mM magnesium chloride, and 20 mM dithiothreitol). RNA and random hexamers were heated to 70°C for 10 min and cooled on ice before the rest of the reaction mix was added. The reaction was incubated at 25°C for 5 min with 200 units reverse transcriptase, and then an additional 10 min at 25°C following addition of another 100 units reverse transcriptase, to facilitate primer annealing, before incubating at 42°C for 50 min. The RT-reactions were terminated by heat inactivating the reverse transcriptase for 15 min at 70°C. RNA complementary to cDNA was removed by digestion with 1 unit RNase H (Boehringer Mannheim) for 20 min at 37°C. RNA from the head and neck squamous cell carcinoma (HNSCC) line TU167 infected with recombinant adenovirus Ad5CMV-*p53* (multiplicity of infection of 100:1) was used as a positive control for detecting virally transcribed p53, and TU167 cells infected with the variant adenovirus vector d1312 (1) that does not contain the p53 transcriptional unit was used as a negative control.

To detect the Ad5CMV-*p53* transcript, PCR was performed in a reaction volume of 30 µl containing 0.2 mM of each dNTP, 1.5 mM magnesium chloride, 1 unit taq polymerase (Promega, Madison, WI), and 0.5 mM of each primer CMV2 (5' GGTGCATTGGAACGCGGATT, SEQ ID NO: 11) and P53EX3 (5' GGGGACAGAACGTTGTTTTC, SEQ ID NO:12) in 1 x PCR buffer (50 mM potassium chloride, 10 mM Tris pH 9.0, 0.1% Triton X-100). Primers CMV2 and P53EX3 amplify a 295-base fragment specific to the adenovirus derived p53 transcript. PCR conditions for detecting Ad5CMV-*p53* transcripts were as follows:
94°C for 1 min, followed by 94°C for 30 sec, 58°C for 40 sec, 70°C for 1 min for 35 cycles, and 70°C for 10 min extension time.

To ensure that the product amplified during PCR was detecting mRNA and not contaminating DNA in the RNA preparation, PCR was also performed using RT products from parallel reactions in which no reverse transcriptase was added.

RT-PCR specific for glyceraldehyde-3-phosphate dehydrogenase (GAPDH) was performed in order to check the integrity of the RT reactions. A 3 µl volume of the RT reaction was diluted in 30 µl of PCR mix containing 0.2 mM of each DNTP, 2 mM magnesium chloride, 1 unit taq polymerase, and 0.5 mM of each primer GAPDH1 (5' ACGGATTTGGTCGTATTGGG, SEQ ID NO:13) and GAPDH2 (5' TGATTITGGAGGGATCTCGC, SEQ ID NO:14) in 1 x PCR buffer. The GAPDH primers span 3 exons in the human GAPDH gene and amplify a 231-base product specific for mRNA. PCR conditions to detect GAPDH were as follows:
94°C for 1 min, followed by 94°C for 30 sec, 60°C for 12 sec, 72°C for 1 min for 35 cycles, and 72°C for 7 min extension time.
PCR was performed using a Perkin Elmer Gene Amp 9600 thermocylcer, and all primers were commercially synthesized (Genosys, Woodlands, TX).

**Immunohistochemical Determination of Intratumoral Gene.** Immunoperoxidase studies were performed on formalin-fixed, paraffin-embedded tissue sections using the avidin-biotin-peroxidase complex (ABC) method (1). The specimens were cut 3-4µm thick, deparaffinized in xylene, and rehydrated in descending grades (100-70%) of ethanol. Endogenous peroxidase activity was blocked with 3% hydrogen peroxide in methanol. After several washes in distilled water and phosphate buffered saline (PBS), section were incubated with a 1:10 dilution of normal horse serum to minimize background staining. This was followed by overnight incubation at 4°C with monoclonal antibodies to p53 (DO-1, Oncogene Science, Inc., Uniondale, NY; 1:80 dilution) and p21 (Oncogene Science, Inc., 1:100). Peroxidase staining procedure was done using ABC Elite kits (Vector Laboratories, Burlingame, CA). The immunostaining reaction was visualized using 0:05% 3.3'-diaminobenzidene in Tris-HC 1 buffer containing 0.01 % hydrogen peroxide, pH 7.6. Sections were counterstained with 0.01% toluidine blue and mounted in permount. Scoring was performed by counting positive nuclear staining in 200 cells of 10 consecutive high power fields by two independent observes.

**TUNEL Assay for DNA Fragmentation**. The TUNEL assay was performed using the Apoptag™ PLUS kit (Oncor, Gaithersburg, M.D.) according to instructions. provided by the manufacturer. Slides were counterstained with .4% methelene green. Corresponding hematoxilin and eosin stained slides were evaluated for the presence of inflammatory cell infiltrates and were graded on a scale of 1-4.

**Cytopathic Effect Assay Procedure**. Patient urine samples also were monitored for the presence of Ad5p53 by an assay in which any virus in the sample is allowed to infect a recipient cell monolayer. Those cells are monitored for the appearance of a cytopathic effect (CPE): the cells round up and detach from the surface. The patient urine samples assayed for CPE were from the first morning urines collected during the second week of the first treatment course, and on Day 0 or 1 (pretreatment). Samples of approximately 15 mls each were stored in sterile 15-ml conical tubes at -80°C until use. The IT293 cells, which form the recipient cell monolayer in this assay, are maintained in DMEM plus 10% FBS, in a 37°C humidified 10% CO₂ incubator. Two days prior to patient sample testing, the cells were plated at 2 x 10⁵ per well in 12-well plates.

At time of assay, the urine samples were thawed in an ice bath, and an aliquot was mixed 1:1 with DMEM and sterile-filtered using a 0.22 µm syringe filter. A 350 µl aliquot of this 1:1 mix was slowly added to each well after removing the growth medium. The plate was gently rocked 15 minutes later. After 30 minutes, 2.0 mis of DMEM plus 10% FBS was added to each well to dilute the sample. On day 3 (72 hours later) and day 6 of the assay, 0.5 ml aliquots of fresh medium were added to each well, to help maintain the cell monolayer for the maximum 6-7 days.

Patients samples were assayed in triplicate. The diluted pretreatment sample was assayed as is, and also spiked with 10⁵ pfu of Ad5p53 per well to detect any urine component that might interfere with the detection of virus by this procedure. Control wells were inoculated with DMEM alone spiked with 10⁵, 10⁴, 10³, 10², or 10¹ pfu per well, each in duplicate. The 10 pfu spike causes CPE under these conditions on day 2 of the assay; on each successive day, the next spiked control will show CPE. Therefore, the time at which CPE is detected in each patient sample indicates the level of Ad5p53 in that sample.

**Recombinant Competent Adenovirus.** The adenoviral p53 used in the clinical trial was tested for the presence of RCA using A549 cells by the Biotechnology Services Division of Microbiological Associates, Inc., (Rockville, Maryland).

**Statistical Analysis.** A single-arm study design was used. To prevent enrolling more patients than necessary in a trial of excessive toxicity was found, a Bayesian early stopping rule was implemented. The WILCOXON signed rank test and assigned test were used for comparisons between before and after treatment of the percentage of cells showing TUNEL and immunohistochemical staining, respectively. Statistical analysis was performed using the Survpac SPSS-Statistical package.

**Response and Toxicity.** Survival and response were assessed in this interim analysis, but were not considered aims of this analysis. The purpose of this interim analysis was to determine transduction potential of this gene transfer strategy. Patients were evaluable for response and toxicity following a 30-day observation after one cycle of gene transfer. Toxic effects of therapy were evaluated according to the National Cancer Institute's common toxicity criteria. (Xref.) Response to therapy was assessed by CT scan or ultrasound of the neck before each course of treatment. Patients were evaluable for response if they received at least one course of therapy followed by an appropriate documentation of response. Those patients undergoing surgical resection of their recurrent tumors could not be evaluated for response since surgery was performance prior to a 30 day observation period. All CT scans were evaluated by one radiologist and ultrasounds by another. Partial response was defined as a 50 percent or greater reduction in the sum of the products of the diameters of the measureable tumor; a minor response was defined as a 25 percent to less than 50 percent reduction in the sum of the products of the diameters of the measureable lesion. Progression of disease was defined as a 25 percent or greater increase in the sum of the products of the diameters.

Survival duration was measured from time of protocol entry until expiration. Each patient's response was reviewed by a data management committee consisting of a Head and Neck Surgical Oncologist, Radiologist, and Medical oncologist.

### Results

**Detection of Vector.** Adenoviral-vector DNA was detected by PCR in serum as well as urine samples from patients up to 48 hours following gene transfer. The detection limit for vector is 1-5 viral particles. Viral DNA was isolated in urine among patients undergoing gene transfer at each viral dosage, but not detected after 48 hours following gene transfer. Serum detection of viral DNA increased with increasing viral dosage above 10⁷ PFU, but was also not detectable 48 hours following gene transfer.

Urine samples were first analyzed for infectious virus, measured CPE on cell monolayers, prior to PCR nucleotide analysis The virus present in the urine was applied to 293 cells as described above, in order to monitor the CPE, which is observed as the cells round up and become from the petri dish. CPE was identified rarely in specimens. CPE was found late in overlay cultures (greater than six days) and these results were considered equivocable. In no instance was CPE confirmed by PCR and Southern blot transfer of the same urine sample for detected adenoviral nucleotides.

**Viral DNA Analysis of other Organ Systems.** PCR analysis demonstrated viral DNA greater than two months following 10⁹ Ad5CMVp53 gene transfer in skin, cardiac muscle, lung, and testicular tissues in snap frozen autopsy specimens carefully sampled to prevent cross-contamination between tissues. Renal parenchyma, adrenal glands and pancreatic tissues did not show viral DNA sequences specific for this vector. Immunohistochemical analysis of these autopsy specimens (did not reveal evidence of wild-type p53 protein product overexpression).

**Assessment of Gene Transfer.** All analyses were performed after at least 1 hour of exposure of tissues to the vector. The mRNA product was detected at 4 hours and 48 hours via RT-PCR. In contrast, biopsy specimens frozen after 1 hour of exposure or less, showed no *p53* mRNA. In addition, negative control samples obtained from an operative site that had not been subjected to gene transfer were also negative for the PCR product. Non-transduced and transduced biopsy specimens from four patients were analyzed by RT-PCR for the presence of AdSCMVp53 transcribed mRNA. Transduced specimens from two patients were positive on EtBr stained gels, while no Ad5CMVp53 product was detected in any of the non-transduced specimens despite the fact that GAPDH could be amplified from all specimens. The specificity of the 295 bp PCR product from the two positive patients was confirmed by Southern Blotting. Because PCR product was not observed when reverse transcriptase was ommitted from the RT reactions, the 295 bp product detected in the figure had to have been generated from mRNA, rather than contaminating DNA.

**Immunohistochemical Analysis.** All patients pre- and post-gene transfer specimens were simultaneously analyzed with positive and negative, controls in each experiment. Multiple sections of each specimen were analyzed and compared with hematoxylin and eosin-stained as well as TDT end-labeled specimens. Post-vector injected biopsy specimens confirmed gene transfer in three patients whose pre-transfer biopsy specimens did not show endogenously overexpressed p53 protein. In 5 of 21 patients (27%), p21 (CIP/WAF1) was not significantly endogenously expressed; this proved informative for gene transfer in the patient post-gene transfer biopsy specimens. p53 nuclear protein overexpression was also seen in tumor-associated lymphocytes as well as stromal tumor cells, thus indicating nontumor cell transduction as well.

**Serologic Antibody Response.** Adenovirus serotype 5 antibodies were induced in all patients following repeat injections of the vector. However, transduction efficiency was not found to be significiantly altered comparing the initial cycle of gene transfer and subsequent cycles. Mild tumor injection site erythema was recognized beginning at 3 x 10⁹ PFU., however these local reactions did not limit vector tolerance. No evidence of systemic hypersensitivity was found in any patients despite repeat viral dosing for as much as six consecutive months in a 10⁹ PFU-treated patient.

**Pathologic Observations.** Needle tracks have been identified in the majority of biopsy specimens, and gene product expression was demonstrated in deeper tissues beyond the injection sites. Similarly, the finding of end-labeled cells in transduced areas suggested the induction of apoptosis in tumor cells but the absence of apoptosis in stromal or inflammatory cells. Inflammatory cells were noted in patients and became prominent histologic findings among the patients who received doses of 10⁹ PFU or greater. Interestingly, patient number 7, whose sampled expressed endogenous wild-type p53, demonstrated hemorrhagic necrosis with no evidence of viable tumor in serially sectioned surgical specimens in his recurrent 2 cm neck mass after one-cycle of gene transfer. Pathologic findings of necrosis as well as apoptosis induction were frequent observations in specimens.

**Clinical Observations.** Patients have tolerated direct tumoral injections of vector with the most frequent adverse event being injection site discomfort. Evidence of local injection site erythema has been noted at 10⁹ PFU of vector and higher although systemic evidence of hypersensitivity has not been seen despite evidence of systemic antibody titer elevations. Patients number 5, 10, and 16 remain without evidence of disease with a median follow-up of seven months. Patients 7 and 13 exhibited stable disease for 3 and 5 months, respectively in their indicator lesion area. Patient number 20 exhibited a partial response as verified by CAT Scan and ultrasound, but developed spinal and pleural metastasis requiring removal from study due to disease progression.

In conclusion, patients with recurrent squamous carcinomas of the head and neck, adenovirally mediated gene transfer of wild-type *p53* is safe and can effectively transduce cancer and normal cells via direct tumoral injections or intraoperative instillation of vector. None of the patients exhibited toxicity that might limit vector administration or dose escalation. Transgene product expression was unaltered despite development of a systemic antibody response. The local inflammatory responses found pathologically within resected tumor specimens may, in fact, be beneficial.

### REFERENCES

The following references, to the extent that they provide exemplary procedural or other details supplementary to those set forth herein, are specifically incorporated herein by reference:
"Cancer facts and figures," American Cancer Society, Washington, DC: ACS, (Publication no. 90-425 M. no. 5008-LE).1990.
Cancer Facts and Figures, Publication No 93-400, M No. 5008-93. Washington, DC. American Cancer Society, 1993.
Anderson *et al.* US 5,399,346, 1995.
Baichwal and Sugden, "Vectors for gene transfer derived from animal DNA viruses: Transient and stable expression of transferred genes," In: Kucherlapati R, ed. Gene transfer. New York: Plenum Press, pp. 117-148, 1986.
Baker et al., "Suppression of human colorectal carcinoma cell growth by wild-type p53," Science, 249:912-915, 1990.
Barbacid, M. "ras genes," Ann. Rev. Biochem., 56:779-827, 1985.
Bartek et al., "Genetic and immunochemical analysis of mutant p53 in human breast cancer cell lines," Oncogene, 5:893-899, 1990.
Benvenisty and Neshif, "Direction introduction of genes into rats and expression of the genes," Proc. Nat'l Acad. Sci. USA, 83:9551-9555, 1986.
Berenson et al., "Frequent amplification of the bcl-1 locus in head and neck squamous cell carcinomas," Oncogene, 4:1111-1116, 1989.
Berges et al., "Cell proliferation, DNA repair, and p53 function are not required for programmed cell death of prostatic glandular cells induced by androgen ablation," Proc. Natl. Acad. Sci. USA, 90:8910-8914, 1993.
Berkner, "Development of adenovirus vectors for the expression of heterologous genes," BioTechniques, 6:616-629, 1988.
Bos, J., "ras oncogenes in human cancer: A review," Cancer Res., 49:2682, 1989.
Boshart et al., "A very strong enhancer is located upstream of an immediate early gene of human cytomegalovirus," Cell, 41:521-530, 1985.
Boussif et al., "A versatile vector for gene and oligonucleotide transfer into cells in culture and in vivo: Polyethylenimine," Proc. Natl. Acad Sci. USA, 92:7297-7301,1993.
Boyle et al., "The Incidence of p53 Mutation Increases with Progression of Head and Neck Cancer," Can Res., 53:4477-4480, 1993.
Brennan et al., "Molecular Assessment of Histopathological Staging in Squamous-Cell Carcinoma of the Head and Neck," NEJM, 332(7):429-425, 1995.
Brown et al., "Modulation of ras Expression by Anti-sense, Nonionic Deoxyoligonucleotide Analogs," Oncogene Res., 4:243-252, 1989.
Cai et al., "Stable expression of the wide-type p53 gene in human lung cancer cells after retrovirus-mediated gene transfer," Human Gene Therapy, 4:617-624, 1993.
Calhoun et al.; "Distant Metastases from Head and Neck Squamous Cell Carcinomas," Laryngoscope, 104:1199-1205, 1994.
Carnpbell, in Monoclonal Antibody Technology, Laboratory Techniques in Biochemistry and Molecular Biology Vol. 13, Burden and Von Knippenberg, Eds. pp. 75-83, Amsterdam, Elseview, 1984
Chen et al., "Genetic mechanisms of tumor suppression by the human p53 gene," Science, 250:1576-1580, 1990.
Chen and Okayama, "High-efficiency transfection of mammalian cells by plasmid DNA," Mol. Cell Biol., 7:2745-2752, 1987.
Chomczynski and Sacchi, "Single-step method of RNA isolation by guanidinium thiocyanate-phenol-chloroform extraction," Anal. Biochem., 162:156-159, 1987.
Chung et al., "Discordant p53 gene mutations in primary head and neck cancers and corresponding second primary cancers of the upper aerodigestive tract," 53:1676-1683, 1993.
Clarke et al., "Thymocyte apoptosis induced by p53-dependent and independent pathways," Nature, 362:849-852,1993.
Clayman et al., "Regulation of Urokinase-Type Plasminogen Activator Expression in Squamous-Cell Carcinoma of the Oral Cavity," Int J Cancer, 54:73-80, 1993.
Couch et al., "Immunization with types 4 and 7 adenovirus by selective infection of the intestinal tract," Am. Rev. Resp. Dis., 88:394-403, 1963.
Culver et al., "in vivo gene transfer with retroviral vector-producer cells for treatment of experimental brain tumors," Science, 256:1550-1552, 1992.
Debus et al., "Effects of Antisense Oligodeoxyribonucleotides Complementary mRNA of the Human c-Harvey-ras Oncogene on Cell Proliferation," J. Cancer Res. Clin. Oncol., 116 (Suppl. Part 1):S-162, 1990.
Der and Cooper, Cell, 32:201-8, 1983.
Diller et al., "p53 function as a cell cycle control protein in osteosarcomas," Mol. Cell. Biol., 10:5772-5781, 1990.
Donehower et al., "Mice deficient for p53 are developmentally normal but susceptible to spontaneous tumors," Nature, 356:215-221, 1992.
Dubensky et al., "Direct transfection of viral and plasmid DNA into the liver or spleen of mice," Proc. Nat'l Acad. Sci. USA, 81:7529-7533, 1984.
El-Deiry et al., "WAF1/CIP1 is induced in p53-mediated G1 arrest and apoptosis," Cancer Res., 54:1169-1174, 1994.
Fearon et al., Science, 247:49, 1990.
Fechheimer et al., "Transfection of mammalian cells with plasmid DNA by scrape loading and sonication loading," Proc. Nat'l Acad. Sci. USA, 84:8463-8467, 1987.
Ferkol et al., FASEB J., 7:1081-1091, 1993.
Field et al., "Elevated expression of the c-myc oncoprotein correlates with poor prognosis in head and neck squamous cell carcinoma," Oncogene, 4:1463-1468, 1989.
Field et al., "The Role of the p53 Tumor Suppressor Gene in Squamous Cell Carcinoma of the Head and Neck," Arch Otolaryngol Head Neck Surg, 119:1118-1122, 1993.
Fraley et al., "Entrapment of a bacterial plasmid in phospholipid vesicles: Potential for gene transfer," Proc. Nat'l Acad. Sci. USA, 76:3348-3352, 1979.
Fridman et al., "The minimal fragments of c-Raf-1 and NF1 that can suppress a v-Haras-induced phenotype," J. Biol. Chem., 269:30105-30108, 1994.
Friedmann, "Progress toward human gene therapy," Science, 244:1275-1281, 1989.
Fujiwara et al., "Therapeutic effect of a retroviral wild-type p53 expression vector in an orthotopic lung cancer model," JNCI, 86:1458-1462, 1994.
Fujiwara et al., "A retroviral wild-type p53 expression vector penetrates human lung cancer spheroids and inhibits growth by inducing apoptosis," Cancer Res., 53:4129-4133, 1993.
Gefter et al., Somatic Cell Genet. 3:231-236 (1977)
Georges et al., "Prevention of Orthotopic Human Lung Cancer Growth by Intratracheal Instillation of a Retroviral Antisense K-ras Construct," Cancer Res., 53:1743-1746, 1993.
Ghosh-Choudhury et al., "Protein IX, a minor component of the human adenovirus capsid, is essential for the packaging of full-length genomes," EMBO J., 6:1733-1739, 1987.
Ghosh and Bachhawat, "Targeting of liposomes to hepatocytes," In: Wu G. and C. Wu ed. Liver diseases, targeted diagnosis and therapy using specific receptors and ligands. New York: Marcel Dekker, pp. 87-104, 1991.
Goding, 1986, in Monoclonal Antibodies: Principles and Practice, 2d ed., Orlando, Fla., Academic Press, 1986, pp. 60-61, 65-66,71-74.
Gomez-Foix et al., "Adenovirus-mediated transfer of the muscle glycogen phosphorylase gene into hepatocytes confers altered regulation of glycogen," J. Biol. Chem., 267:25129-25134,1992.
Gopal, "Gene transfer method for transient gene expression, stable transfection, and cotransfection of suspension cell cultures," Mol. Cell Biol., 5:1188-1190, 1985.
Gorczyca et al., "Detection of DNA strand breaks in individual apoptotic cells by the in situ terminal deoxynucleotidyl transferase and nick translation assays," Cancer Res., 53:1945-1951,1993.
Graham and Prevec, "Adenovirus-based expression vectors and recombinant vaccines," Biotechnology, 20:363-390, 1992.
Graham and Prevec, "Manipulation of adenovirus vector," In: E.J. Murray (ed.), Methods in Molecular Biology: Gene Transfer and Expression Protocol, Clifton, NJ: Humana Press, 7:109-128, 1991.
Graham et al., "Characteristics of a human cell line transformed by DNA from human adenovirus type 5", J. Gen. Virol., 36:59-72, 1977.
Graham and van der Eb, "A new technique for the assay of infectivity of human adenovirus 5 DNA", Virology, 52:456-467, 1973.
Grunhaus and Horwitz, "Adenovirus as cloning vector," Seminar in Virology, 3:237-252, 1992.
Harland and Weintraub, "Translation of mammalian mRNA injected into Xenopus oocytes is specifically inhibited by antisense RNA," J. Cell Biol., 101:1094-1099, 1985.
Hartwell and Kastan, "Cell cycle Control and Cancer," Science, 266:1821-1828, 1994.
Hermonat and Muzycska, "Use of adenoassociated virus as a mammalian DNA cloning vector: Transduction of neomycin resistance into mammalian tissue culture cells," Proc. Nat'l Acad. Sci. USA, 81:6466-6470, 1984.
Herz and Gerard, "Adenovirus-mediated transfer of low density lipoprotein receptor gene acutely accelerates cholesterol clearance in normal mice," Proc. Nat'l Acad Sci. USA 90:2812-2816, 1993.
Hoollstein et al., "p53 mutations in human cancers," Science, 253:49-53, 1991.
Hopp et al., "A Short Polypeptide Marker Sequence Useful for Identification and Purification of Recombinant Proteins," BioTechnology, 7:1205-1210, 1988.
Hsu et al., "Use of Avidin-Biotin-Peroxidase Complex (ABC) in Immunoperoxidase Techniques: a Comparison Between ABC and Unlabeled Antibody (PAP) Procedures," J. Histochem. Cytochem., 29:577-580, 1981.
Jones and Shenk, "Isolation of deletion and substitution mutants of adenovirus type 5," Cell, 13:181-188, 1978.
Kaneda et al., "Increased expression of DNA cointroduced with nuclear protein in adult rat liver," Science, 243:375-378, 1989.
Karlsson et al., EMBO J., 5:2377-2385, 1986.
Kashani-Sabet et al., "Reversal of the malignant phenotype by an anti-ras ribozyme," Antisense Res. Dev., 2:3-15, 1992.
Kastan et al., "A mammalian cell cycle checkpoint pathway utilizing p53 and GADD45 is defective in ataxia-telangiectasia," Cell, 71:587-597, 1992.
Kato et al., "Expression of hepatitis B virus surface antigen in adult rat liver," J. Biol. Chem., 266:3361-3364, 1991.
Klein et al., "High-velocity microprojectiles for delivering nucleic acids into living . cells," Nature, 327:70-73, 1987.
Kohler and Milstein, Eur. J. Immunol., 6:511-519, 1976.
Kohler and Milstein, Nature, 256:495-497, 1975.
Kozarsky and Wilson, "Gene Therapy: Adenovirus Vectors," Curr. Opin. Genet. Dev., 3:499-503, 1993.
Kyte and Doolittle, "A simple method for displaying the hydropathic character of a protein," J. Mol. Biol., 157(1):105-132, 1982.
Lane and Crawford, "T antigen is bound to a host protein in SV40-transformed cells," Nature, 278:261-3, 1979.
Le Gal La Salle et al., "An adenovirus vector for gene transfer into neurons and glia in the brain," Science, 259:988-990, 1993.
Levrero et al., "Defective and nondefective adenovirus vectors for expressing foreign genes in vitro and in vivo," Gene, 101: 195-202, 1991.
Li et al., "Assessment of recombinant adenoviral vectors for hepatic gene therapy," Hum. Gene Ther., 4:403-409,1993.
Linzer and Levine, "Characterization of a 54K dalton cellular SV40 tumor antigen present in SV40-transformed cells and uninfected embryonal carcinoma cells," Cell, 17:43-52, 1979.
Lowe et al., "p53 is required for radiation-induced apoptosis in mouse thymocytes," Nature, 362:847-849, 1993.
Maestro et al., "High frequency of p53 gene alterations associated with protein overexpression in human squamous cell carcinoma of the larynx," Oncogene, 7:1159-1166, 1992.
Mann et al., "Construction of a retrovirus packaging mutant and its use to produce helper-free defective retrovirus," Cell, 33:153-159, 1983.
Markowitz et al., "A safe packaging line for gene transfer: Separating viral genes on two different plasmids," J. Virol., 62:1120-1124, 1988.
Martinez et al., "Cellular localization and cell cycle regulation by a temperature-sensitive p53 protein," Genes Dev., 5:151-159, 1991.
Marx, "Learning how to suppress cancer," Science, 261:1385-1387,1993.
Mashal et al., "Rearrangement and expression of p53 in the chronic phase and blast crisis of chronic myelogenous leukemia," Blood, 75:180-189, 1990.
Matlashewski et al., "Isolation and characterization of a human p53 cDNA clone: expression of the human p53 gene," The EMBO Journal, 3(13):3257-3262, 1984.
Mercer et al., "Negative growth regulation in a glioblastoma tumor cell line that conditionally expresses human wild-type p53," Proc. Natl. Acad Sci. USA, 87:6166-6170, 1990.
Merino et al., "An Analysis of Distant Metastasis From Squamous Cell Carcinoma of the Upper Respiratory and Digestive Tracts," Cancer, 40:147-156, 1977.
Mietz et al., "The transcriptional transactivation function of wild-type p53 is inhibited by SV40 large T-antigen and by HPV-16 E6 oncoprotein," EMBO J., 11:5013-5020, 1992.
Mitsudomi et al., "p53 gene mutations in non-small-cell lung cancer cell lines and their correlation with the presence of ras mutations and clinical features," Oncogene, 7:171-180,1992.
Mukhopadhyay et al., "Specific Inhibition of K-ras Expression and Tumorigenicity of Lung Cancer Cells by Antisense RNA," Cancer Res., 51:1744-1748,1991.
Mulcahy et al., "Requirements for ras protoonogene function during serum-stimulated growth ofNIH/3T3 cells," Nature, 313:241-243, 1985.
Mulligan, "The Basic Science of Gene Therapy," Science, 260:926-932, 1993.
Myers, EPO 0273085
Nicolau et al., "Liposomes as carriers for in vivo gene transfer and expression," Methods Enzymol., 149:157-176, 1987*.*
Nicolau and Sene, "Liposome-mediated DNA transfer in eukaryotic cells," Biochem. Biophys. Acta, 721:185-190, 1982.
Nylander et al., "p53 Expression and Cell Proliferation in Squamous Cell Carcinomas of the Head and Neck," Cancer, 75:87-93, 1995.
O'Malley, Jr. et al., "Adenovirus-mediated Gene Therapy for Human Head and Neck Squamous Cell Cancer in a Nude Mouse Model," Cancer Res, 55:1080-1085, 1995.
Ogiso et al., "Suppression of various human tumor cell lines by a dominant negative H-ras mutant," Gene Ther., 1:403-407, 1994.
Oren and Levine, "Molecular cloning of a cDNA specific for the murine p53 cellular tumor antigen," Proc. Natl. Acad Sci. USA, 80:56-59, January 1983.
Pavelic et al., "Overexpression of p53 Protein is Common in Premalignant Head and Neck Lesions," Anticancer Research, 14:2259-2266, 1994.
Perales et al., Proc. Natl. Acad Sci. USA, 91:4086-4090, 1994.
Potter et al., "Enhancer-dependent expression of human k immunoglobulin genes introduced into mouse pre-B lymphocytes by electroporation," Proc. Nat'l Acad. Sci. USA, 81:7161-7165, 1984.
Racher et al., Biotechnology Techniques, 9:169-174, 1995.
Ragot et al., "Efficient adenovirus-mediated transfer of a human minidystrophin gene to skeletal muscle of mdx mice," Nature, 361:647-650, 1993.
Ramqvist et al., "Wild-type p53 induces apoptosis in a Burkitt lymphoma (BL) line that carries mutant p53," Oncogene, 8:1495-1500, 1993.
Rao et al., "The adenovirus E1A proteins induce apoptosis which is inhibited by the E1B 19K and Bcl-2 proteins," Proc. Natl. Acad Sci. USA, 89:7742-7746, 1992.
Renan, "Cancer genes: current status, future prospects, and applicants in radiotherapy/oncology," Radiother. Oncol., 19:197-218, 1990.
Rich et al., "Development and analysis of recombinant adenoviruses for gene therapy of cystic fibrosis," Hum. Gene Ther., 4:461-476, 1993.
Ridgeway, "Mammalian expression vectors," In: Rodriguez RL, Denhardt DT, ed. Vectors: A survey of molecular cloning vectors and their uses. Stoneham: Butterworth, pp. 467-492, 1988.
Rippe et al., "DNA-mediated gene transfer into adult rat hepatocytes in primary culture," Mol. Cell Biol., 10:689-695, 1990.
Rodrigues et al., "p53 mutations in colorectal cancer," Proc. Natl. Acad. Sci. USA, 87:7555-7559, 1990.
Rosenfeld et al., "in vivo transfer of the human cystic fibrosis transmembrane conductance regulator gene to the airway epithelium," Cell, 68:143-155, 1992.
Rosenfeld et al., "Adenovirus-mediated transfer of a recombinant al-antitrypsin gene to the lung epithelium in vivo," Science, 252:431-434, 1991.
Roth et al., U.S. Serial No. 07/987,235
Sacks et al., "Establishment and Characterization of Two New Squamous Cell Carcinoma Cell Lines Derived from Tumors of the Head and Neck," Cancer Res., 48:2858-2866, 1988.
Shaulsky et al., "Nuclear accumulation of p53 protein is mediated by several nuclear localization signals and plays a role in tumorigenesis," Mol Cell Biol, 10(12):6565-6567, 1990.
Shaw et al., "Induction of apoptosis by wild-type p53 in a human colon tumor-derived cell line," Proc. Natl. Acad Sci. USA, 89:4495-4499, 1992.
Shirasawa et al., "Altered Growth of Human Colon Cancer Cell Lines Disrupted at Activated Ki-ras," Science, 260:85-88, 1993.
Somers et al., "Amplification of the int-2 gene in human head and neck squamous cell carcinomas," Oncogene, 5:915-920, 1990.
Southern and Berg, "Transformation of Mammalian Cells to Antibiotic Resistance with a Bacterial Gene Under Control of the SV40 Early Region Promoter," J. Mol. App. Gen., 1:327-341, 1982.
Stratford-Perricaudet and Perricaudet," Gene transfer into animals: the promise of adenovirus," p. 51-61, In: Human Gene Transfer, Eds, O. Cohen-Haguenauer and M. Boiron, Editions John Libbey Eurotext, France, 1991.
Stratford-Perricaudet et al., "Evaluation of the transfer and expression in mice of an enzyme-encoding gene using a human adenovirus vector," Hum. Gene Ther., 1:241-256, 1990.
Su et al., "Production of Recombinant Porcine Tumor Necrosis Factor Alpha in a Novel E.coli Expression System," Biotechniques, 13(5):756-761, 1992.
Tabin et al., Nature, 300:143-149, 1982.
Takahashi et al., "The p53 gene is very frequently mutated in small-cell lung cance. with a distinct nucleotide substitution pattern," Cancer Res., 52:734-736, 1992.
Thawley and Panje, eds, "Comprehensive Management of Head and Neck Tumors," Philadelphia: WB Saunders, 2:1158-1172, 1987.
Top et al., "Immunization with live types 7 and 4 adenovirus vaccines. II. Antibody response and protective effect against acute respiratory disease due to adenovirus type 7, "J. Infect. Dis., 124:155-160, 1971.
Tur-Kaspa et al., "Use of electroporation to introduce biologically active foreign genes into primary rat hepatocytes," Mol. Cell Biol., 6:716-718, 1986.
van der Riet et al., "Frequent loss of chromosome 9 p21-22 early in head and neck cancer progression," Cancer Res., 54:1156-1158, 1994.
Wagner et al., Science, 260:1510-1513, 1993.
Wang et al., "Apoptosis induced in human osteosarcoma cells is one of the mechanisms for the cytocidal effect of AdCMV-p53," Cancer Gene Therapy, 2(1):1-9,1995.
Watling et al., "Overexpression of p53 in Head and Neck Cancer," Head and Neck, 14:437-444, 1992.
Wijsman et al., "A new method to detect apoptosis in parriffin section: in situ end-labeling of fragmented DNA," J. Histochem. Cytochem., 41:7-12, 1993.
Wilcock and Lane, "Localization of p53, retinoblastoma, and host replication proteins at sites of viral replication in herpes-infected cells," Nature, 349:429-431, 1991.
Wong et al., "Appearance of β-lactamase activity in animal cells upon liposome mediated gene transfer," Gene, 10:87-94, 1980.
Wu and Wu, "Receptor-mediated in vitro gene transfections by a soluble DNA carrier system," J. Biol, Chem., 262:4429-4432, 1987.
Wu and Wu, Adv. Drug Delivery Rev., 12:159-167, 1993.
Wu and Wu, "Evidence for targeted gene delivery to HepG2 hepatoma cells in vitro," Biochemistry, 27:887-892, 1988.
Wu and Levine, "p53 and E2F-1 cooperate to mediate apoptosis," Proc. Natl. Acad. Sci. USA, 91:3602-3606, 1994.
Yamamoto et al., "High incidence of amplification of the epidermal growth factor receptor gene in human squamous carcinoma cell lines," Cancer Res., 46:414-416, 1986.
Yang et al., "in vivo and in vitro gene transfer to mammalian somatic cells by particle bombardment," Proc. Nat'l Acad. Sci. USA, 87:9568-9572, 1990.
Yonish-Rouach et al., "Wild-type p53 induces apoptosis of myeloid leukaemic cells that is inhibited by interleukin-6," Nature, 352:345-347, 1991.
Yuasa et al., Nature, 303:775-559, 1983.
Zelenin et al., "High-velocity mechanical DNA transfer of the chloramphenicol acetyltransferase gene into rodent liver, kidney and mammary gland cells in organ explants and in vivo," FEBS Lett., 280:94-96, 1991.
Zhang et al., U.S. Serial No. 08/423,573
Zhang and Roth, U.S. Serial No. 08/222,285
Zhang et al., "Generation and identification of recombinant adenovirus by liposome-mediated transfection and PCR analysis," Biotechniques, 15:868-872, 1993.
Zhang et al., "High-efficiency gene transfer and high-level expression of wild-type p53 in human lung cancer cells mediated by recombinant adenovirus," Cancer Gene Therapy, 1:1-10, 1994.

### Annex to the application documents - subsequently filed sequences listing

SEQUENCE LISTING

(1) GENERAL INFORMATION:

(i) APPLICANT:
(A) NAME: BOARD OF REGENTS, THE UNIVERSITY
OF TEXAS SYSTEM
(B) STREET: 201 WEST 7TH STREET
(C) CITY: AUSTIN
(D) STATE: TEXAS
(E) COUNTRY: USA
(F) POSTAL CODE (ZIP): 78701

(ii) TITLE OF INVENTION: METHODS AND COMPOSITIONS
FOR THE DIAGNOSIS OF CANCER

(iii) NUMBER OF SEQUENCES: 14

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version
#1.30 (EPO)

(v) CURRENT APPLICATION DATA:
(A) APPLICATION NUMBER: UNKNOWN
(B) FILING DATE: CONCURRENTLY HEREWITH
(C) CLASSIFICATION: UNKNOWN

(vi) PRIOR APPLICATION DATA:
(A) APPLICATION NUMBER: US 60/007,810
(B) FILING DATE: 30-NOV-1995

(2) INFORMATION FOR SEQ ID NO: 1:

(i) SEQUENCE CHARACTERISTICS:
(A) LENGTH: 2066 base pairs
(B) TYPE: nucleic acid
(C) STRANDEDNESS: single
(D) TOPOLOGY: linear

(xi) SEQUENCE DESCRIPTION: SEQ ID NO: 1:

CAAAACCTAC CAGGGCAGCT ACGGTTTCCG TCTGGGCTTC TTGCATTCTG GGACAGCCAA 60

GTCTGTGACT TGCACGTACT CCCCTGCCCT CAACAAGATG TTTTGCCAAC TGGCCAAGAC 120

CTGCCCTGTG CAGCTGTGGG TTGATTCCAC ACCCCCGCCC GGCACCCGCG TCCGCGCCAT 180

GGCCATCTAC AAGCAGTCAC AGCACATGAC GGAGGTTGTG AGGCGCTGCC CCCACCATGA 240

GCGCTGCTCA GATAGCGATG GTCTGGCCCC TCCTCAGCAT CTTATCCGAG TGGAAGGAAA 300

TTTGCGTGTG GAGTATTTGG ATGACAGAAA CACTTTTCGA CATAGTGTGG TGGTGCCCTA 360

TGAGCCGCCT GAGGTTGGCT CTGACTGTAC CACCATCCAC TACAACTACA TGTGTAACAG 420

TTCCTGCATG GGCGGCATGA ACCGGAGGCC CATCCTCACC ATCATCACAC TGGAAGACTC 480

CAGTGGTAAT CTACTGGGAC GGAACAGCTT TGAGGTGCGT GTTTGTGCCT GTCCTGGGAG 540

AGACCGGCGC ACAGAGGAAG AGAATCTCCG CAAGAAAGGG GAGCCTCACC ACGAGCTGCC 600

CCCAGGGAGC ACTAAGCGAG CACTGCCCAA CAACACCAGC TCCTCTCCCC AGCCAAAGAA 660

GAAACCACTG GATGGAGAAT ATTTCACCCT TCAGATCCGT GGGCGTGAGC GCTTCGAGAT 720

GTTCCGAGAG CTGAATGAGG CCTTGGAACT CAAGGATGCC CAGGCTGGGA AGGAGCCAGG 780

GGGGAGCAGG GCTCACTCCA GCCACCTGAA GTCCAAAAAG GGTCAGTCTA CCTCCCGCCA 840

TAAAAAACTC ATGTTCAAGA CAGAAGGGCC TGACTCAGAC TGACATTCTC CACTTCTTGT 900

TCCCCACTGA CAGCCTCCCA CCCCCATCTC TCCCTCCCCT GCGATTTTGG GTTTTGGGTC 960

TTTGAACCCT TGCTTGCAAT AGGTGTGCGT CAGAAGCACC CAGGACTTCC ATTTGCTTTG 1020

TCCCGGGGCT CCACTGAACA AGTTGGCCTG CACTGGTGTT TTGTTGTGGG GAGGAGGATG 1080

GGGAGTAGGA CATACCAGCT TAGATTTTAA GGTTTTTACT GTGAGGGATG TTTGGGAGAT 1140

GTAAGAAATG TTCTTGCAGT TAAGGGTTAG TTTACAATCA GCCACATTCT AGGTAGGGGC 1200

CCACTTCACC GTACTAACCA GGGAAGCTGT CCCTCACTGT TGAATTTTCT CTAACTTCAA 1260

GGCCCATATC TGTGAAATGC TGGCATTTGC ACCTACCTCA CAGAGTGCAT TGTGAGGGTT 1320

AATGAAATAA TGTACATCTG GCCTTGAAAC CACCTTTTAT TACATGGGGT CTAGAACTTG 1380

ACCCCCTTGA GGGTGCTTGT TCCCTCTCCC TGTTGGTCGG TGGGTTGGTA GTTTCTACAG 1440

TTGGGCAGCT GGTTAGGTAG AGGGAGTTGT CAAGTCTCTG CTGGCCCAGC CAAACCCTGT 1500

CTGACAACCT CTTGGTGAAC CTTAGATCCT AAAAGGAAAT GTCACCCCAT CCCACACCCT 1560

GGAGGATTTC ATCTCTTGTA TAGATGATCT GGATCCACCA AGACTTGTTT TAGCTCAGGG 1620

TCCAATTTCT TTTTTCTTTT TTTTTTTTTT TTTCTTTTTC TTTGAGACTG GGTCTCTTTG 1680

TTGCCCCAGG CTGGAGTGGA GTGGCGTGAT CTGGCTTACT GCAGCCTTTG CCTCCCCGGC 1740

TCGAGCAGTC CTGCCTCAGC CTCCGGAGTA GCTGGGACCA CAGGTTCATG CCACCATGGC 1800

CAGCCAACTT TTGCATGTTT TGTAGAGATG GGGTCTCACA GTGTTGCCCA GGCTGGTCTC 1860

AAACTCCTGG GCTCAGGCGA TCCACCTGTC TCAGCCTCCC AGAGTGCTGG GATTACAATT 1920

GTGAGCCACC ACGTCCAGCT GGAAGGGTCA ACATCTTTTA CATTCTGCAA GCACATCTGC 1980

ATTTTCACCC CACCCTTCCC CTCTTCTCCC TTTTTATATC CCATTTTTAT ATCGATCTCT 2040

TATTTTACAA TAAAACTTTG CTGCCA 2066

(2) INFORMATION FOR SEQ ID NO: 2:

(i) SEQUENCE CHARACTERISTICS:
(A) LENGTH: 293 amino acids
(B) TYPE: amino acid
(C) STRANDEDNESS: single
(D) TOPOLOGY: linear

(xi) SEQUENCE DESCRIPTION: SEQ ID NO: 2:

Lys Thr Tyr Gln Gly Ser Tyr Gly Phe Arg Leu Gly Phe Leu His Ser
1               5                   10                  15

Gly Thr Ala Lys Ser Val Thr Cys Thr Tyr Ser Pro Ala Leu Asn Lys
            20                  25                  30

Met Phe Cys Gln Leu Ala Lys Thr Cys Pro Val Gln Leu Trp Val Asp
        35                  40                  45

Ser Thr Pro Pro Pro Gly Thr Arg Val Arg Ala Met Ala Ile Tyr Lys
    50                  55                  60

Gln Ser Gln His Met Thr Glu Val Val Arg Arg Cys Pro His His Glu
65                  70                  75                  80

Arg Cys Ser Asp Ser Asp Gly Leu Ala Pro Pro Gln His Leu Ile Arg
                85                  90                  95

Val Glu Gly Asn Leu Arg Val Glu Tyr Leu Asp Asp Arg Asn Thr Phe
            100                 105                 110

Arg His Ser Val Val Val Pro Tyr Glu Pro Pro Glu Val Gly Ser Asp
        115                 120                 125

Cys Thr Thr Ile His Tyr Asn Tyr Met Cys Asn Ser Ser Cys Met Gly
    130                 135                 140

Gly Met Asn Arg Arg Pro Ile Leu Thr Ile Ile Thr Leu Glu Asp Ser
145                 150                 155                 160

Ser Gly Asn Leu Leu Gly Arg Asn Ser Phe Glu Val Arg Val Cys Ala
                165                 170                 175

Cys Pro Gly Arg Asp Arg Arg Thr Glu Glu Glu Asn Leu Arg Lys Lys
            180                 185                 190

Gly Glu Pro His His Glu Leu Pro Pro Gly Ser Thr Lys Arg Ala Leu
        195                 200                 205

Pro Asn Asn Thr Ser Ser Ser Pro Gln Pro Lys Lys Lys Pro Leu Asp
    210                 215                 220

Gly Glu Tyr Phe Thr Leu Gln Ile Arg Gly Arg Glu Arg Phe Glu Met
225                 230                 235                 240

Phe Arg Glu Leu Asn Glu Ala Leu Glu Leu Lys Asp Ala Gln Ala Gly
                245                 250                 255

Lys Glu Pro Gly Gly Ser Arg Ala His Ser Ser His Leu Lys Ser Lys
            260                 265                 270

Lys Gly Gln Ser Thr Ser Arg His Lys Lys Leu Met Phe Lys Thr Glu
        275                 280                 285

Gly Pro Asp Ser Asp
    290

(2) INFORMATION FOR SEQ ID NO: 3:

(i) SEQUENCE CHARACTERISTICS:
(A) LENGTH: 2066 base pairs
(B) TYPE: nucleic acid
(C) STRANDEDNESS: single
(D) TOPOLOGY: linear

(xi) SEQUENCE DESCRIPTION: SEQ ID NO: 3:

CAAAACTTAC CAGGGCAACT ATGGCTTCCA CCTGGGCTTC CTGCAGTCTG GGACAGCCAA 60

GTCTGTTATG TGCACGTACT CTCCTCCCCT CAATAAGCTA TTCTGCCAGC TGGCGAAGAC 120

GTGCCCTGTG CAGTTGTGGG TCAGCGCCAC ACCTCCAGCT GGGAGCCGTG TCCGCGCCAT 180

GGCCATCCAC AAGAAGTCAC AGCACTTGAC GGGGGTCGTG AGACGCTGCC CCCACCATGA 240

GCGCTGCTCC GATGGTGATG GCCTGGCTCC TCCCCAGCAT CTTATCCGGG TGGAAGGAAA 300

TTTGTATCCC GAGTATCTGG AAGACAGGCA GACTTTTCGC CACAGCGTGG TGGTACCTTA 360

TGAGCCACCC GAGGCCGGCT CTGAGTATAC CACCATCCAC TACAAGTACA TTTGTAATAG 420

CTCCTGCATG GGGGGCATGA ACCGCCGACC TATCCTTACC ATCATCACAC TGGAAGACTC 480

CAGTGGGAAC CTTCTGGGAC GGGACAGCTT TGAGGTTCGT GTTTGTGCCT GCCCTGGGAG 540

AGACCGCCGT ACAGAAGAAG AAAATTTCCG CAAAAAGGAA GTCCTTTGCC CTGAACTGCC 600

CCCAGGGAGC GCAAAGAGAG CGCTGCCCAC CTGCACAAGC GCCTCTCCCC CGCAAAAGAA 660

AAAACCACTT GATGGAGAGT ATTTCACCCT CAAGATCCGC GGGCGTAAAC GCTTCGAGAT 720

GTTCCGGGAG CTGAATGAGG CCTTAGAGTT AAAGGATGCC CATGCTACAG AGGAGTCTGG 780

AGACAGCAGG GCTCACTCCA GCTACCTGAA GACCAAGAAG GGCCAGTCTA CTTCCCGCCA 840

TAAAAAAACA ATGGTCAAGA AAGTGGGGCC TGACTCAGAC TGACATTCTC CACTTCTTGT 900

TCCCCACTGA CAGCCTCCCA CCCCCATCTC TCCCTCCCCT GCCTTTTGGG TTTTGGGTCT 960

TTGAACCCTT GCTTGCAATA GGTGTGCGTC AGAAGCACCC AGGACTTCCA TTTGCTTTGT 1020

CCCGGGGCTC CACTGAACAA GTTGGCCTGC ACTGGTGTTT TGTTGTGGGG AGGAGGATGG 1080

GGAGTAGGAC ATACCAGCTT AGATTTTAAG GTTTTTACTG TGAGGGATGT TTGGGAGATG 1140

TAAGAAATGT TCTTGCAGTT AAGGGTTAGT TTACAATCAG CCACATTCTA GGTAGGGGCC 1200

CACTTCACCG TACTAACCAG GGAAGCTGTC CCTCACTGTT GAATTTTCTC TAACTTCAAG 1260

GCCCATATCT GTGAAATGCT GGCATTTGCA CCTACCTCAC AGAGTGCATT GTGAGGGTTA 1320

ATGAAATAAT GTACATCTGG CCTTGAAACC ACCTTTTATT ACATGGGGTC TAGATGACCC 1380

CCTTGAGGTG CTTGTTCCCT CTCCCTGTTG GTCGGTGGGT TGGTAGTTTC TACAGTTGGG 1440

CAGCTGGTTA GGTTGAGGTA GTTGTCAGGT CTCTGCTGGC CCAGCGAAAT TCTATCCAGC 1500

CAGTTGTTGG ACCCTGGCAC CTCAAATGAA ATCTCACCCT ACCCCACACC CTGTAAGATT 1560

CTATCTCTTG TATAGATGAT CTGGATCCAC CAAGACTTGT TTTAGCTCAG GGTCCAATTT 1620

CTTTTTTCTT TTTTTTTTTT TTTTTCTTTT TCTTTGAGAC TGGGTCTCTT TGTTGCCCCA 1680

GGCTGGAGTG GAGTGGCGTG ATCTGGCTTA CTGCAGCCTT TGCCTCCCCG GCTCGAGCAG 1740

TCCTGCCTCA GCCTCCGGAG TAGCTGGGAC CACAGGTTCA TGCCACCATG GCCAGCCAAC 1800

TTTTGCATGT TTTGTAGAGA TGGGGTCTCA CAGTGTTGCC CAGGCTGGTC TCAAACTCCT 1860

GGGCTCAGGC GATCCACCTG TCTCAGCCTC CCAGAGTGCT GGGATTACAA TTGTGAGCCA 1920

CCACGTCCAG CTGGAAGGGC CTACTTTCCT TCCATTCTGC AAAGCCCTGC TGCATTTATC 1980

CACCCCACCC TCCACCTGTC TCCCTCTTTT TTTCTTACCC CTTTTTATAT ATCAATTTCT 2040

TATTTTACAA TAAAATTTTG TTATCA 2066

(2) INFORMATION FOR SEQ ID NO: 4:

(i) SEQUENCE CHARACTERISTICS:
(A) LENGTH: 293 amino acids
(B) TYPE: amino acid
(C) STRANDEDNESS: single
(D) TOPOLOGY: linear

(xi) SEQUENCE DESCRIPTION: SEQ ID NO: 4:

Lys Thr Tyr Gln Gly Asn Tyr Gly Phe His Leu Gly Phe Leu Gln Ser
1               5                   10                  15

Gly Thr Ala Lys Ser Val Met Cys Thr Tyr Ser Pro Pro Leu Asn Lys
            20                  25                  30

Leu Phe Cys Gln Leu Ala Lys Thr Cys Pro Val Gln Leu Trp Val Ser
        35                  40                  45

Ala Thr Pro Pro Ala Gly Ser Arg Val Arg Ala Met Ala Ile His Lys
    50                  55                  60

Lys Ser Gln His Met Thr Gly Val Val Arg Arg Cys Pro His His Glu
65                  70                  75                  80

Arg Cys Ser Asp Gly Asp Gly Leu Ala Pro Pro Gln His Leu Ile Arg
                85                  90                  95

Val Glu Gly Asn Leu Tyr Pro Glu Tyr Leu Glu Asp Arg Gln Thr Phe
            100                 105                 110

Arg His Ser Val Val Val Pro Tyr Glu Pro Pro Glu Ala Gly Ser Glu
        115                 120                 125

Tyr Thr Thr Ile His Tyr Lys Tyr Ile Cys Asn Ser Ser Cys Met Gly
    130                 135                 140

Gly Met Asn Arg Arg Pro Ile Leu Thr Ile Ile Thr Leu Glu Asp Ser
145                 150                 155                 160

Ser Gly Asn Leu Leu Gly Arg Asn Ser Phe Glu Val Arg Val Cys Ala
                165                 170                 175

Cys Pro Gly Arg Asp Arg Arg Thr Glu Glu Glu Asn Phe Arg Lys Lys
            180                 185                 190

Glu Val Leu Cys Pro Glu Leu Pro Pro Gly Ser Ala Lys Arg Ala Leu
        195                 200                 205

Pro Thr Cys Thr Ser Ala Ser Pro Pro Gln Lys Lys Lys Pro Leu Asp
    210                 215                 220

Gly Glu Tyr Phe Thr Leu Lys Ile Arg Gly Arg Leu Arg Phe Glu Met
225                 230                 235                 240

Phe Arg Glu Leu Asn Glu Ala Leu Glu Leu Lys Asp Ala His Ala Thr
                245                 250                 255

Glu Glu Ser Gly Asp Ser Arg Ala His Ser Ser Tyr Leu Lys Ser Lys
            260                 265                 270

Lys Gly Gln Ser Thr Ser Arg His Lys Lys Thr Met Val Lys Lys Val
        275                 280                 285

Gly Pro Asp Ser Asp
    290

(2) INFORMATION FOR SEQ ID NO: 5:

    (i) SEQUENCE CHARACTERISTICS:
        (A) LENGTH: 17 base pairs
        (B) TYPE: nucleic acid
        (C) STRANDEDNESS: single
        (D) TOPOLOGY: linear

    (xi) SEQUENCE DESCRIPTION: SEQ ID NO: 5:

ACTGCCCAAC AACACCA                                                    17

(2) INFORMATION FOR SEQ ID NO: 6:

(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO: 6:

GCCACGCCCA CACATTT 17

(2) INFORMATION FOR SEQ ID NO: 7:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 7:

GCCTGTCCTG GGAGAGACCG 20

(2) INFORMATION FOR SEQ ID NO: 8:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 8:

CCCTTAAGCC ACGCCCACAC 20

(2) INFORMATION FOR SEQ ID NO: 9:

(i) SEQUENCE CHARACTERISTICS:
(A) LENGTH: 18 base pairs
(B) TYPE: nucleic acid
(C) STRANDEDNESS: single
(D) TOPOLOGY: linear

(xi) SEQUENCE DESCRIPTION: SEQ ID NO: 9:

CACTGCCCAA CAACACCA 18

(2) INFORMATION FOR SEQ ID NO: 10:

(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO: 10:

GCCACGCCCA CACATTT 17

(2) INFORMATION FOR SEQ ID NO: 11:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 11:

GGTGCATTGG AACGCGGATT 20

(2) INFORMATION FOR SEQ ID NO: 12:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 12:

GGGGACAGAA CGTTGTTTTC 20

(2) INFORMATION FOR SEQ ID NO: 13:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 13:

ACGGATTTGG TCGTATTGGG 20

(2) INFORMATION FOR SEQ ID NO: 14:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 14:

TGATTTTGGA GGGATCTCGC 20

SEQUENCE LISTING

<110> THE BOARD OF REGENTS, THE UNIVERSITY OF TEXAS SYSTEM et al.

<120> METHODS AND COMPOSITIONS FOR THE DIAGNOSIS AND
      TREATMENT OF CANCER

<130> F62532PCEPT1

<140> 96942100.7
<141> 1996-11-27

<150> 60/007,810
<151> 1995-11-30

<160> 14

<170> PatentIn Ver. 2.1

<210> 1
<211> 2066
<212> DNA
<213> Artificial Sequence

<220>
<223> Description of Artificial Sequence:  Synthetic
      Primer

<400> 1
caaaacctac cagggcagct acggtttccg tctgggcttc ttgcattctg ggacagccaa 60
gtctgtgact tgcacgtact cccctgccct caacaagatg ttttgccaac tggccaagac 120
ctgccctgtg cagctgtggg ttgattccac acccccgccc ggcacccgcg tccgcgccat 180
ggccatctac aagcagtcac agcacatgac ggaggttgtg aggcgctgcc cccaccatga 240
gcgctgctca gatagcgatg gtctggcccc tcctcagcat cttatccgag tggaaggaaa 300
tttgcgtgtg gagtatttgg atgacagaaa cacttttcga catagtgtgg tggtgcccta 360
tgagccgcct gaggttggct ctgactgtac caccatccac tacaactaca tgtgtaacag 420
ttcctgcatg ggcggcatga accggaggcc catcctcacc atcatcacac tggaagactc 480
cagtggtaat ctactgggac ggaacagctt tgaggtgcgt gtttgtgcct gtcctgggag 540
agaccggcgc acagaggaag agaatctccg caagaaaggg gagcctcacc acgagctgcc 600
cccagggagc actaagcgag cactgcccaa caacaccagc tcctctcccc agccaaagaa 660
gaaaccactg gatggagaat atttcaccct tcagatccgt gggcgtgagc gcttcgagat 720
gttccgagag ctgaatgagg ccttggaact caaggatgcc caggctggga aggagccagg 780
ggggagcagg gctcactcca gccacctgaa gtccaaaaag ggtcagtcta cctcccgcca 840
taaaaaactc atgttcaaga cagaagggcc tgactcagac tgacattctc cacttcttgt 900
tccccactga cagcctccca cccccatctc tccctcccct gcgattttgg gttttgggtc 960
tttgaaccct tgcttgcaat aggtgtgcgt cagaagcacc caggacttcc atttgctttg 1020
tcccggggct ccactgaaca agttggcctg cactggtgtt ttgttgtggg gaggaggatg 1080
gggagtagga cataccagct tagattttaa ggtttttact gtgagggatg tttgggagat 1140
gtaagaaatg ttcttgcagt taagggttag tttacaatca gccacattct aggtaggggc 1200
ccacttcacc gtactaacca gggaagctgt ccctcactgt tgaattttct ctaacttcaa 1260
ggcccatatc tgtgaaatgc tggcatttgc acctacctca cagagtgcat tgtgagggtt 1320
aatgaaataa tgtacatctg gccttgaaac caccttttat tacatggggt ctagaacttg 1380
acccccttga gggtgcttgt tccctctccc tgttggtcgg tgggttggta gtttctacag 1440
ttgggcagct ggttaggtag agggagttgt caagtctctg ctggcccagc caaaccctgt 1500
ctgacaacct cttggtgaac cttagatcct aaaaggaaat gtcaccccat cccacaccct 1560
ggaggatttc atctcttgta tagatgatct ggatccacca agacttgttt tagctcaggg 1620
tccaatttct tttttctttt tttttttttt tttctttttc tttgagactg ggtctctttg 1680
ttgccccagg ctggagtgga gtggcgtgat ctggcttact gcagcctttg cctccccggc 1740
tcgagcagtc ctgcctcagc ctccggagta gctgggacca caggttcatg ccaccatggc 1800
cagccaactt ttgcatgttt tgtagagatg gggtctcaca gtgttgccca ggctggtctc 1860
aaactcctgg gctcaggcga tccacctgtc tcagcctccc agagtgctgg gattacaatt 1920
gtgagccacc acgtccagct ggaagggtca acatctttta cattctgcaa gcacatctgc 1980
attttcaccc cacccttccc ctcttctccc tttttatatc ccatttttat atcgatctct 2040
tattttacaa taaaactttg ctgcca                                      2066

<210> 2

<211> 293
<212> PRT
<213> Artificial Sequence

<220>
<223> Description of Artificial Sequence: Synthetic
      Peptide

<400> 2
Lys Thr Tyr Gln Gly Ser Tyr Gly Phe Arg Leu Gly Phe Leu His Ser
1               5                   10                  15

Gly Thr Ala Lys Ser Val Thr Cys Thr Tyr Ser Pro Ala Leu Asn Lys
            20                  25                  30

Met Phe Cys Gln Leu Ala Lys Thr Cys Pro Val Gln Leu Trp Val Asp
        35                  40                  45

Ser Thr Pro Pro Pro Gly Thr Arg Val Arg Ala Met Ala Ile Tyr Lys
    50                  55                  60

Gln Ser Gln His Met Thr Glu Val Val Arg Arg Cys Pro His His Glu
65                  70                  75                  80

Arg Cys Ser Asp Ser Asp Gly Leu Ala Pro Pro Gln His Leu Ile Arg
                85                  90                  95

Val Glu Gly Asn Leu Arg Val Glu Tyr Leu Asp Asp Arg Asn Thr Phe
            100                 105                 110

Arg His Ser Val Val Val Pro Tyr Glu Pro Pro Glu Val Gly Ser Asp
        115                 120                 125

Cys Thr Thr Ile His Tyr Asn Tyr Met Cys Asn Ser Ser Cys Met Gly
    130                 135                 140

Gly Met Asn Arg Arg Pro Ile Leu Thr Ile Ile Thr Leu Glu Asp Ser
145                 150                 155                 160

Ser Gly Asn Leu Leu Gly Arg Asn Ser Phe Glu Val Arg Val Cys Ala
                165                 170                 175

Cys Pro Gly Arg Asp Arg Arg Thr Glu Glu Glu Asn Leu Arg Lys Lys
            180                 185                 190

Gly Glu Pro His His Glu Leu Pro Pro Gly Ser Thr Lys Arg Ala Leu
        195                 200                 205

Pro Asn Asn Thr Ser Ser Ser Pro Gln Pro Lys Lys Lys Pro Leu Asp
    210                 215                 220

Gly Glu Tyr Phe Thr Leu Gln Ile Arg Gly Arg Glu Arg Phe Glu Met
225                 230                 235                 240

Phe Arg Glu Leu Asn Glu Ala Leu Glu Leu Lys Asp Ala Gln Ala Gly
                245                 250                 255

Lys Glu Pro Gly Gly Ser Arg Ala His Ser Ser His Leu Lys Ser Lys
            260                 265                 270

Lys Gly Gln Ser Thr Ser Arg His Lys Lys Leu Met Phe Lys Thr Glu
        275                 280                 285

Gly Pro Asp Ser Asp
    290


<210> 3
<211> 2066

<212> DNA
<213> Artificial Sequence

<220>
<223> Description of Artificial Sequence: Synthetic
      Primer

<400> 3
   caaaacttac cagggcaact atggcttcca cctgggcttc ctgcagtctg ggacagccaa 60
   gtctgttatg tgcacgtact ctcctcccct caataagcta ttctgccagc tggcgaagac 120
   gtgccctgtg cagttgtggg tcagcgccac acctccagct gggagccgtg tccgcgccat 180
   ggccatccac aagaagtcac agcacttgac gggggtcgtg agacgctgcc cccaccatga 240
   gcgctgctcc gatggtgatg gcctggctcc tccccagcat cttatccggg tggaaggaaa 300
   tttgtatccc gagtatctgg aagacaggca gacttttcgc cacagcgtgg tggtacctta 360
   tgagccaccc gaggccggct ctgagtatac caccatccac tacaagtaca tttgtaatag 420
   ctcctgcatg gggggcatga accgccgacc tatccttacc atcatcacac tggaagactc 480
   cagtgggaac cttctgggac gggacagctt tgaggttcgt gtttgtgcct gccctgggag 540
   agaccgccgt acagaagaag aaaatttccg caaaaaggaa gtcctttgcc ctgaactgcc 600
   cccagggagc gcaaagagag cgctgcccac ctgcacaagc gcctctcccc cgcaaaagaa 660
   aaaaccactt gatggagagt atttcaccct caagatccgc gggcgtaaac gcttcgagat 720
   gttccgggag ctgaatgagg ccttagagtt aaaggatgcc catgctacag aggagtctgg 780
   agacagcagg gctcactcca gctacctgaa gaccaagaag ggccagtcta cttcccgcca 840
   taaaaaaaca atggtcaaga aagtggggcc tgactcagac tgacattctc cacttcttgt 900
   tccccactga cagcctccca cccccatctc tccctcccct gccttttggg ttttgggtct 960
   ttgaaccctt gcttgcaata ggtgtgcgtc agaagcaccc aggacttcca tttgctttgt 1020
   cccggggctc cactgaacaa gttggcctgc actggtgttt tgttgtgggg aggaggatgg 1080
   ggagtaggac ataccagctt agattttaag gtttttactg tgagggatgt ttgggagatg 1140
   taagaaatgt tcttgcagtt aagggttagt ttacaatcag ccacattcta ggtaggggcc 1200
   cacttcaccg tactaaccag ggaagctgtc cctcactgtt gaattttctc taacttcaag 1260
   gcccatatct gtgaaatgct ggcatttgca cctacctcac agagtgcatt gtgagggtta 1320
   atgaaataat gtacatctgg ccttgaaacc accttttatt acatggggtc tagatgaccc 1380
   ccttgaggtg cttgttccct ctccctgttg gtcggtgggt tggtagtttc tacagttggg 1440
   cagctggtta ggttgaggta gttgtcaggt ctctgctggc ccagcgaaat tctatccagc 1500
   cagttgttgg accctggcac ctcaaatgaa atctcaccct accccacacc ctgtaagatt 1560
   ctatctcttg tatagatgat ctggatccac caagacttgt tttagctcag ggtccaattt 1620
   cttttttctt tttttttttt tttttctttt tctttgagac tgggtctctt tgttgcccca 1680
   ggctggagtg gagtggcgtg atctggctta ctgcagcctt tgcctccccg gctcgagcag 1740
   tcctgcctca gcctccggag tagctgggac cacaggttca tgccaccatg gccagccaac 1800
   ttttgcatgt tttgtagaga tggggtctca cagtgttgcc caggctggtc tcaaactcct 1860
   gggctcaggc gatccacctg tctcagcctc ccagagtgct gggattacaa ttgtgagcca 1920
   ccacgtccag ctggaagggc ctactttcct tccattctgc aaagccctgc tgcatttatc 1980
   caccccaccc tccacctgtc tccctctttt tttcttaccc ctttttatat atcaatttct 2040
   tattttacaa taaaattttg ttatca                                      2066


<210> 4
<211> 293
<212> PRT
<213> Artificial Sequence

<220>
<223> Description of Artificial Sequence: Synthetic
      Peptide

<400> 4
Lys Thr Tyr Gln Gly Asn Tyr Gly Phe His Leu Gly Phe Leu Gln Ser
  1               5                  10                  15

Gly Thr Ala Lys Ser Val Met Cys Thr Tyr Ser Pro Pro Leu Asn Lys
             20                  25                  30

Leu Phe Cys Gln Leu Ala Lys Thr Cys Pro Val Gln Leu Trp Val Ser
         35                  40                  45

Ala Thr Pro Pro Ala Gly Ser Arg Val Arg Ala Met Ala Ile His Lys
     50                  55                  60

Lys Ser Gln His Met Thr Gly Val Val Arg Arg Cys Pro His His Glu
 65                  70                  75                  80

Arg Cys Ser Asp Gly Asp Gly Leu Ala Pro Pro Gln His Leu Ile Arg
                85                  90                  95

Val Glu Gly Asn Leu Tyr Pro Glu Tyr Leu Glu Asp Arg Gln Thr Phe
            100                 105                 110

Arg His Ser Val Val Val Pro Tyr Glu Pro Pro Glu Ala Gly Ser Glu
        115                 120                 125

Tyr Thr Thr Ile His Tyr Lys Tyr Ile Cys Asn Ser Ser Cys Met Gly
    130                 135                 140

Gly Met Asn Arg Arg Pro Ile Leu Thr Ile Ile Thr Leu Glu Asp Ser
145                 150                 155                 160

Ser Gly Asn Leu Leu Gly Arg Asn Ser Phe Glu Val Arg Val Cys Ala
                165                 170                 175

Cys Pro Gly Arg Asp Arg Arg Thr Glu Glu Glu Asn Phe Arg Lys Lys
            180                 185                 190

Glu Val Leu Cys Pro Glu Leu Pro Pro Gly Ser Ala Lys Arg Ala Leu
        195                 200                 205

Pro Thr Cys Thr Ser Ala Ser Pro Pro Gln Lys Lys Lys Pro Leu Asp
    210                 215                 220

Gly Glu Tyr Phe Thr Leu Lys Ile Arg Gly Arg Leu Arg Phe Glu Met
225                 230                 235                 240

Phe Arg Glu Leu Asn Glu Ala Leu Glu Leu Lys Asp Ala His Ala Thr
                245                 250                 255

Glu Glu Ser Gly Asp Ser Arg Ala His Ser Ser Tyr Leu Lys Ser Lys
            260                 265                 270

Lys Gly Gln Ser Thr Ser Arg His Lys Lys Thr Met Val Lys Lys Val
        275                 280                 285

Gly Pro Asp Ser Asp
    290


<210> 5
<211> 17
<212> DNA
<213> Artificial Sequence

<220>
<223> Description of Artificial Sequence:  Synthetic
      Primer

<400> 5
actgcccaac aacacca                                                17


<210> 6
<211> 17
<212> DNA
<213> Artificial Sequence

<220>
<223> Description of Artificial Sequence:  Synthetic
      Primer

<400> 6
gccacgccca cacattt                                                17

<210> 7
<211> 20
<212> DNA
<213> Artificial Sequence

<220>
<223> Description of Artificial Sequence:  Synthetic
      Primer

<400> 7
gcctgtcctg ggagagaccg                                                   20


<210> 8
<211> 20
<212> DNA
<213> Artificial Sequence

<220>
<223> Description of Artificial Sequence:  Synthetic
      Primer

<400> 8
cccttaagcc acgcccacac                                                   20


<210> 9
<211> 18
<212> DNA
<213> Artificial Sequence

<220>
<223> Description of Artificial Sequence:  Synthetic
      Primer

<400> 9
cactgcccaa caacacca                                                     18


<210> 10
<211> 17
<212> DNA
<213> Artificial Sequence

<220>
<223> Description of Artificial Sequence:  Synthetic
      Primer

<400> 10
gccacgccca cacattt                                                      17


<210> 11
<211> 20
<212> DNA
<213> Artificial Sequence

<220>
<223> Description of Artificial Sequence:  Synthetic
      Primer

<400> 11
ggtgcattgg aacgcggatt                                                   20


<210> 12
<211> 20
<212> DNA

<213> Artificial Sequence

<220>
<223> Description of Artificial Sequence: Synthetic
      Primer

<400> 12
ggggacagaa cgttgttttc                                                    20


<210> 13
<211> 20
<212> DNA
<213> Artificial Sequence

<220>
<223> Description of Artificial Sequence: Synthetic
      Primer

<400> 13
acggatttgg tcgtattggg                                                    20


<210> 14
<211> 20
<212> DNA
<213> Artificial Sequence

<220>
<223> Description of Artificial Sequence: Synthetic
      Primer

<400> 14
tgattttgga gggatctcgc                                                    20

## Claims

1. Use of an expression construct, in particular a viral expression construct, comprising a promoter functional in eukaryotic cells and a polynucleotide encoding a functional p53 polypeptide, wherein said polynucleotide is positioned sense to and under the control of said promoter for the preparation of a medicament for the treatment of a human subject with a body cavity created by surgical excision of a tumor.

2. The use of Claim 1, wherein the medicament is to be administered to said body cavity in vivo, the administration resulting in expression of said functional p53 polypeptide in tumor cells remaining at the primary tumor site and inhibition of recurrent tumor cell growth.

3. The use of Claim 1, wherein said tumor is selected from the group consisting of a carcinoma, a glioma, a sarcoma, and a melanoma.

4. The use of Claim 1, wherein said tumor cell is malignant.

5. The use of Claim 1, wherein said tumor cell is benign.

6. The use of Claim 1, wherein said tumor is a tumor of the lung, skin, prostate, liver, testes, bone, brain, colon, pancreas, head and neck, stomach, ovary, breast or bladder.

7. The use of Claim 1, wherein said viral expression construct is selected from the group consisting of a retroviral vector, an adenoviral vector and an adeno-associated viral vector.

8. The use of Claim 7, wherein said viral vector is a replication-deficient adenoviral vector.

9. The use of Claim 8, wherein said replication-deficient adenoviral vector is lacking at least a portion of the E1-region.

10. The use of Claim 9, wherein said promoter is a CMV IE promoter.

11. The use of Claim 8, wherein the expression vector is administered to said body cavity by injection, via a sponge, swab or other device.

12. The use of Claim 1, wherein said tumor comprises cells that express a functional p53 polypeptide.

13. The use of Claim 1, wherein said expression construct is to be administered in a volume of about 3 ml to about 10 ml.

14. The use of Claim 11, wherein the amount of adenovirus to be administered in each contacting is between about 10⁷ and 10¹² pfu.

15. The use of Claim 1, wherein the body cavity is continuously perfused with said medicament via a catheter inserted into the cavity prior to closure of the surgical entry site.

16. The use of Claim 1, wherein the p53-encoding polynucleotide is tagged so that expression of p53 from said expression construct can be detected.

17. The use of Claim 16, wherein the tag is a continuous epitope.

18. The use of Claim 1, wherein administration of the medicament further comprises contacting said tumor with a DNA damaging agent.

19. The use of Claim 18, wherein said DNA damaging agent is a radiotherapeutic agent.

20. The use of Claim 19, wherein said radiotherapeutic agent is selected from the group consisting of γ-irradiation, x-irradiation, uv-irradiation and microwaves.

21. The use of Claim 19, wherein said DNA damaging agent is a chemotherapeutic agent.

22. The use of Claim 21, wherein said chemotherapeutic agent is selected from the group consisting of adriamycin, 5-fluorouracil, etoposide, camptothecin, actinomycin-D, mitomycin C, verapamil, doxorubicin, podophyllotoxin and cisplatin.

23. The use of Claim 1, wherein administration of the medicament further comprises contacting said tumor with a cytokine.

24. The use of Claim 1, wherein administration of the medicament further comprises contacting said body cavity with a second therapeutic gene other than a gene encoding a p53 polypeptide.

25. The use of Claim 24, wherein said second therapeutic gene is selected from the group consisting of a Dp gene, p21, p16, p27, E₂F, Rb, APC, DC, NF-1, NF-2, WT-1, MEN-I, MEN-II, BRCA1, VHL, FCC, MCC, ras, myc, neu, raf, erb, src, fms, jun, trk, ret, gsp, hst, bcl, abl, Bax, Bcl-Xₛ and EIA.

26. The use of Claim 1, wherein microscopic residual tumor cell growth in a human subject is inhibited.

27. The use of Claim 1, wherein the resected tumor is a squamous cell carcinoma.

28. The use of Claim 1, wherein the endogenous p53 of said resected tumor is wild-type.

29. The use of Claim 28, wherein said tumor is a tumor of the lung, skin, prostate, liver, testes, bone, brain, colon, pancreas, head and neck, stomach, ovary, breast or bladder:

30. The use of Claim 1, wherein the resulting tumor bed is to be contacted with said expression construct at least twice.

31. The use of Claim 1, wherein said expression construct is to be contacted with said body cavity prior to closing of the incision.

32. The use of Claim 1, wherein said body cavity is to be contacted with from about 10⁶ to about 10⁹ infectious adenoviral particles.

33. The use of Claim 1, wherein the administration of the medicament further comprises contacting said tumor with said expression construct prior to resecting said tumor.

34. The use of Claim 33, wherein said tumor is to be injected with said expression construct.

35. The use of Claim 34, wherein said tumor is to be injected with about 10⁶ to about 10⁹ infectious adenoviral particles.

36. The use of Claim 34, wherein said tumor is to be injected with a total of about 1 ml to about 10 ml.

37. The use of Claim 34, wherein said tumor is to be injected at least twice.

38. The use of Claim 37, wherein each of said injections comprise about 0.1 ml to about 0.5 ml volumes spaced about 1 cm apart.

39. The use of Claim 18, wherein said DNA damaging agent is to be contacted before resection.

40. The use of Claim 18, wherein said DNA damaging agent is to be contacted after resection.

41. The use of Claim 18, wherein said DNA damaging agent is to be contacted before and after resection.

42. The use of Claim 23, wherein said cytokine is selected from the group consisting of IL-1α, IL-1β, IL-2, IL-3, IL-4, IL-5, IL-6, IL-7, IL-8, IL-9, IL-10, IL-11, IL-12, IL-13, TGF-β, GM-CSF, M-CSF, TNFα, TNFβ, LAF, TCGF, BCGF, TRF, BAF, BDG, MP, LIF, OSM, TMF, PDGF, IFN-α, IFN-β and IFN-γ.
